(19) Europäisches Patentamt
European Patent Office
Office européen des brevets

(11) EP 4 678 181 A1

(12) EUROPEAN PATENT APPLICATION
published in accordance with Art. 153(4) EPC

(43) Date of publication:
**14.01.2026 Bulletin 2026/03**

(21) Application number: **24767431.0**

(22) Date of filing: **07.03.2024**

(51) International Patent Classification (IPC):
***A61K 38/20** (2006.01)* ***A61K 47/68** (2017.01)*
***A61K 45/06** (2006.01)* ***A61P 35/00** (2006.01)*

(52) Cooperative Patent Classification (CPC):
**A61K 38/20; A61K 45/06; A61K 47/68; A61P 35/00**

(86) International application number:
**PCT/KR2024/002946**

(87) International publication number:
**WO 2024/186143 (12.09.2024 Gazette 2024/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.03.2023 KR 20230029834**

(71) Applicant: **Hanmi Pharm. Co., Ltd.**
**Hwaseong-si, Gyeonggi-do 18536 (KR)**

(72) Inventors:
- **KIM, Jin Young**
  **Hwaseong-si, Gyeonggi-do 18469 (KR)**
- **PARK, Jun Sub**
  **Hwaseong-si, Gyeonggi-do 18469 (KR)**
- **CHOI, Jae Hyuk**
  **Hwaseong-si, Gyeonggi-do 18469 (KR)**
- **JEONG, Seong Ju**
  **Hwaseong-si, Gyeonggi-do 18469 (KR)**
- **HEO, Yong Ho**
  **Hwaseong-si, Gyeonggi-do 18469 (KR)**
- **PARK, Da Hyeon**
  **Hwaseong-si, Gyeonggi-do 18469 (KR)**
- **LEE, Jong Soo**
  **Hwaseong-si, Gyeonggi-do 18469 (KR)**
- **LEE, A Ram**
  **Hwaseong-si, Gyeonggi-do 18469 (KR)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

# (54) COMBINATION THERAPY OF INTERLEUKIN-2 ANALOG OR CONJUGATE THEREOF AND IMMUNE CHECKPOINT INHIBITOR FOR PREVENTION OR TREATMENT OF CANCER

(57) The present invention relates to a combined use of an interleukin-2 analog or conjugate thereof and an immune checkpoint inhibitor for preventing or treating cancer and a pharmaceutical composition comprising the same.

[FIG. 3]

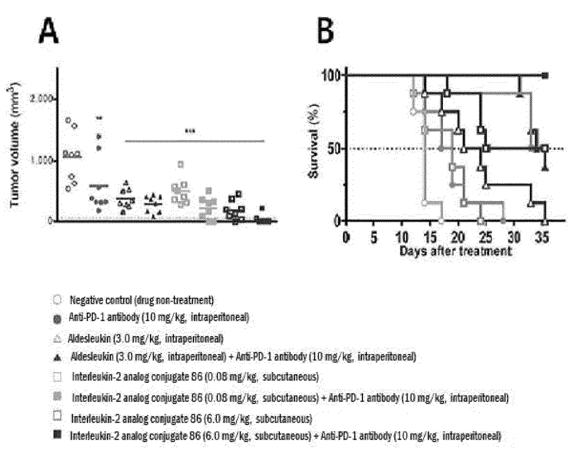




Processed by Luminess, 75001 PARIS (FR)

## Description

### [Technical Field]

The present invention relates to a combination therapy of an interleukin-2 analog or conjugate thereof and an immune checkpoint inhibitor for preventing or treating cancer.

### [Background Art]

Interleukin-2, which is an important immunostimulator with a molecular weight of about 15 kDa and is composed of a total of 133 amino acid residues, activates various cells of the immune system, including T cells and B cells. The high efficacy of interleukin-2 as an immunostimulator can be used to treat various immune-related disease symptoms, including cancer and AIDS (Korean Patent Publication No. 10-2017-0070091). Interleukin-2 (brand name: Proleukin) is currently an FDA-approved drug for the treatment of metastatic renal cell carcinoma and metastatic melanoma. However, the severe toxicity associated with high-dose interleukin-2 therapy limits the number of patients who can receive this therapy, and thus in fact, only a few suitable patients have undergone this therapy. The toxicity associated with interleukin-2 includes severe fever, nausea, vomiting, vascular leak, severe hypotension, pulmonary edema, and vascular leak syndrome, which may cause liver damage.

The interleukin-2 receptor has three kinds of subunit receptors. The subunit consists of an alpha chain (IL-2R$\alpha$, CD25), a beta chain (IL-2R$\beta$ or CD122), and a gamma chain (IL-2R$\gamma$ or CD132). Interleukin-2 can exhibit various functions by binding to receptor subunits in various combinations. The single interleukin-2 alpha receptor is called a low-affinity interleukin-2 receptor, and it is not involved in signaling. Complexes of interleukin-2 beta and gamma receptors bind to interleukin-2 with intermediate affinity. Complexes of interleukin-2 alpha, beta, and gamma receptors bind to interleukin-2 with high affinity. The complexes of interleukin-2 beta and gamma receptors are required for effective signal conversion through kinase activation on multiple signaling pathways. In particular, interleukin-2 beta- and gamma-binding receptors are prominent in $CD8^+$ cells and natural killer (NK) cells. In addition, the complexes of interleukin-2 alpha, beta, and gamma receptors with high affinity are usually found in $CD4^+$ T regulatory cells (Treg) and, recently, found in activated T cells. The interleukin-2 beta receptor is distributed in $CD8^+$ T cells or natural killer cells (NK cells) to be involved in the immune response in the body, and therefore, research has been conducted to develop medicines by enhancing the activity of the beta receptor for immune activation.

In general, immune cells have proteins called immune checkpoints in their cell membranes and thus can inhibit unnecessary autoimmune responses and eliminate cancer cells by detecting tumor-specific antigens expressed due to changes, such as mutations, in cancer cells. However, for the evasion of the above immune actions, cancer cells alter the functions of the immune checkpoints to suppress T cell functions and prevent the occurrence of proper immune responses.

Immune checkpoint inhibitors are medicines that activate the patient's autoimmune system to induce the immune cells in the body to suppress the growth of cancer cells. Unlike existing immunotherapies, the immune checkpoint inhibitors bind to the interaction sites between cancer cells and T cells to block signals for immune evasion, thereby allowing T cells to destroy cancer cells without being hindered by immune evasion. Therefore, research continues on treating cancer by using immune checkpoint inhibitors in immunotherapy for cancer patients.

As described above, research has been actively conducted on treatment methods where the body's immune system is activated to act on cancer cells, achieving anti-cancer effects. However, the use of drugs stimulating the immune system in the anticancer treatment may cause side effects due to immune cell overactivity, and thus there is a need to discover safe and effective anticancer medicines and therapies.

### [Disclosure]

### [Technical Problem]

There is a need to develop safe therapeutics that can activate the immune system and exert anti-cancer effects

### [Technical Solution]

An aspect of the present invention is to provide a pharmaceutical composition comprising an interleukin-2 analog or long-acting conjugate thereof for preventing or treating cancer, wherein the composition is administered in combination with an immune checkpoint inhibitor.

Another aspect of the present invention is to provide a therapy for preventing or treating cancer, wherein an interleukin-2 analog or long-acting conjugate thereof is used in combination with an immune checkpoint inhibitor.

Still another aspect of the present invention is to provide a combination comprising: an interleukin-2 analog or

long-acting conjugate thereof; and an immune checkpoint inhibitor.

Still another aspect of the present invention is to provide a pharmaceutical combination comprising the composition for preventing or treating cancer.

Still another aspect of the present invention is to provide a pharmaceutical kit for preventing, alleviating, or treating cancer, the kit comprising: an interleukin-2 analog or long-acting conjugate thereof; and an immune checkpoint inhibitor.

Still another aspect of the present invention is to provide a method for preventing or treating cancer, the method comprising administering to and/or using for a subject in need thereof the combination, pharmaceutical composition, or pharmaceutical kit.

Still another aspect of the present invention is to provide a method for preventing, alleviating, or treating cancer, the method comprising administering to and/or using for a subject in need thereof a composition comprising a pharmaceutically effective amount of an interleukin-2 analog or long-acting conjugate thereof in combination with a composition comprising a pharmaceutically effective amount of an immune checkpoint inhibitor.

Still another aspect of the present invention is to provide use of the combination, pharmaceutical composition, or pharmaceutical kit, for preventing, alleviating, or treating cancer and/or in the preparation of a medicine for prevention, alleviation, or treatment of cancer.

**[Advantageous Effects]**

The pharmaceutical composition containing an interleukin-2 analog or long-acting conjugate including the same for preventing or treating cancer according to the present invention exhibits a tumor growth inhibitory effect and reduces side effects by administration in combination with an immune checkpoint inhibitor and thus can be used as an excellent anticancer medicine.

**[Brief Description of Drawings]**

FIG. 1 shows the SDS-PAGE results of interleukin-2 analog long-acting conjugates (of analogs 21, 41, and 52).

FIGS. 2A to 2C show the purity analysis results of interleukin-2 analog long-acting conjugates (of analogs 21, 41, and 52).

FIG. 3 shows the evaluation results of anti-tumor efficacy by combination therapy of an interleukin-2 analog long-acting conjugate and an immune checkpoint inhibitor (anti-PD-1 antibody) in malignant melanoma animal models. The results verified the tumor size (FIG. 3A) and the survival rate of mouse models (FIG. 3B) in the mouse models administered aldesleukin or interleukin-2 analog conjugate 86 alone or administered aldesleukin and interleukin 2 analog conjugate 86 in combination with an immune checkpoint inhibitor. In FIG. 3, the dose of the long-acting conjugate was based on only the weight of the interleukin 2 analog site in the entire conjugate.

FIG. 4 shows the evaluation results of anti-tumor efficacy by combination therapy of an interleukin-2 analog long-acting conjugate and an immune checkpoint inhibitor (anti-PD-1 antibody) in lung cancer animal models. The results verified the tumor size in the mouse models administered aldesleukin or interleukin-2 analog conjugate 86 alone or administered aldesleukin and interleukin 2 analog conjugate 86 in combination with an immune checkpoint inhibitor. In FIG. 4, the dose of the long-acting conjugate was based on only the weight of the interleukin 2 analog site in the entire conjugate.

FIG. 5 shows the analysis results of anti-tumor efficacy by combination therapy of an interleukin-2 analog long-acting conjugate and an immune checkpoint inhibitor (anti-PD-1 antibody) in lung cancer animal models by using Bliss independence model. In FIG. 5, the dose of the long-acting conjugate was based on only the weight of the interleukin 2 analog site in the entire conjugate.

FIG. 6 shows the analysis results of anticancer efficacy and recurrence preventing efficacy through the memory response by combination therapy of an interleukin-2 analog long-acting conjugate and an immune checkpoint inhibitor (anti-PD-1 antibody) in pancreatic cancer mouse models. In FIG. 6, the dose of the long-acting conjugate was based on only the weight of the interleukin 2 analog site in the entire conjugate.

**[Best Mode]**

In accordance with an aspect of the present invention, there is provided a composition comprising a novel interleukin-2 analog (or IL-2 analog) or long-acting conjugate thereof, wherein the composition is administered in combination with an immune checkpoint inhibitor. The interleukin-2 (IL-2) analog has an increased binding affinity for interleukin-2 beta receptor compared with native interleukin-2 or aldesleukin, which is an interleukin-2 analog, and may

include a sequence with a modification of at least one amino acid in the native interleukin-2.

In one specific embodiment, the interleukin-2 analog may include an amino acid sequence selected from the group consisting of SEQ ID NOS: 3 to 106.

In another embodiment, the long-acting conjugate may be represented by Chemical Formula 1 below:

[Chemical Formula 1] X-L-F

where: X is an interleukin-2 analog including any one sequence selected from amino acid sequences of SEQ ID NOS: 3 to 106;
L is a polyethylene glycol linker;
F is a dimeric immunoglobulin Fc region, and
the - symbols represent covalent linkages between X and L and between L and F, respectively,
wherein in the long-acting conjugate, one end of L is covalently linked to only one polypeptide chain of the dimeric Fc region and X is covalently linked to the other end of L.

In the composition according to any one of the previous embodiments, the long-acting conjugate may include as a part thereof an interleukin-2 analog having an altered binding affinity for the interleukin-2 alpha receptor, and an increased binding affinity for the interleukin-2 beta receptor compared with the native interleukin-2 or aldesleukin.

In the composition according to any one of the previous embodiments, the interleukin-2 analog may have an increased binding affinity for the interleukin-2 beta receptor compared with aldesleukin.

In the composition according to any one of the previous embodiments, the interleukin-2 analog may include any one sequence selected from the group consisting of amino acid sequences of SEQ ID NOS: 10, 13-15, 17, 20-22, 32, 35, 36, 42, 53, 54, 56, 58-60, 62, 71, 72, 74-78, 85, 87, 89, 91-94, 97-106, 10, 13, 14, 16, 17, 20, 21, 22, 32, 35, 36, 42, 53, 54, 87, 89, 91, 92, 93, 94, 98, 99, 100, 101, 103, 104, and 105.

In the composition according to any one of the previous embodiments, the interleukin-2 analog may include any one sequence selected from the group consisting of amino acid sequences of SEQ ID NOS: 17, 22, 42, 53, 56, 58-60, 62, 71, 72, 74-77, 87, 89, 91-93, 98-101, and 103-106.

In the composition according to any one of the previous embodiments, the interleukin-2 analog may include any one sequence selected from the group consisting of amino acid sequences of SEQ ID NOS: 22, 42, 53, 87, 105, and 106.

In the composition according to any one of the previous embodiments, the interleukin-2 analog may further include at least one amino acid at the C-terminus.

In the composition according to any one of the previous embodiments, the immune checkpoint inhibitor is a PD-1 antagonist.

In the composition according to any one of the previous embodiments, the immune checkpoint inhibitor may be at least one selected from an anti-PD-1 antibody or an antigen-binding fragment thereof.

In the composition according to any one of the previous embodiments, the anti-PD-1 antibody may be selected from the group consisting of nivolumab, pembrolizumab, cemiplimab, spartalizumab, camrelizumab, sintilimab, tislelizumab, toripalimab, dostarlimab, INCMGA00012, AMP-224, and AMP-514.

In the composition according to any one of the previous embodiments, the long-acting conjugate may include an IgG4 Fc region.

In the composition according to any one of the previous embodiments, the long-acting conjugate may include an aglycosylated immunoglobulin Fc region.

In the composition according to any one of the previous embodiments, the immunoglobulin Fc region may be derived from an aglycosylated Fc region of human IgG4.

In the composition according to any one of the previous embodiments, the immunoglobulin Fc region has a structure in which two polypeptide chains are linked through a disulfide bond, wherein two polypeptide chains are linked through only a nitrogen atom of one of the two chains.

In the composition according to any one of the previous embodiments, the immunoglobulin Fc region may include a monomer having the amino acid sequence of SEQ ID NO: 438.

In the composition according to any one of the previous embodiments, the immunoglobulin Fc region is a homodimer of monomers of the amino acid sequence of SEQ ID NO: 438.

In the composition according to any one of the previous embodiments, the immunoglobulin Fc region is linked through a nitrogen atom of an N-terminal proline.

In the composition according to any one of the previous embodiments, in the long-acting conjugate, X may be covalently linked to one Fc region of the dimeric immunoglobulin Fc region via the polyethylene glycol linker.

In the composition according to any one of the previous embodiments, one end of the linker may be linked to only one Fc region chain of two Fc region chains of the dimeric immunoglobulin Fc region.

In the composition according to any one of the previous embodiments, the polyethylene glycol linker may have a

molecular weight of 1 kDa to 100 kDa.

In the composition according to any one of the previous embodiments, the composition may further contain an excipient.

In the composition according to any one of the previous embodiments, the cancer may be any one selected from the group consisting of renal cell cancer, melanoma, colorectal cancer, liver cancer, uterine cancer, ovarian cancer, pancreatic cancer, gallbladder cancer, lung cancer, small cell lung cancer, non-small cell lung cancer, skin cancer, breast cancer, bladder cancer, stomach cancer, head or neck cancer, esophageal cancer, laryngeal cancer, bone cancer, rectal cancer, perianal cancer, colon cancer, fallopian tube carcinoma, endometrial carcinoma, cervical carcinoma, vaginal carcinoma, vulvar carcinoma, Hodgkin's disease, small bowel cancer, endocrine gland cancer, thyroid cancer, parathyroid cancer, adrenal cancer, soft tissue sarcoma, urethral cancer, penile cancer, prostate cancer, chronic or acute leukemia, lymphocyte lymphoma, renal pelvis carcinoma, CNS tumor, primary CNS lymphoma, spinal tumor, brain tumor, glioma (astrocytoma, glioblastoma, oligodendroglioma, ependymoma), blastocytoma, meningioma, brainstem glioma, pituitary adenoma, neuroblastoma, congenital tumor, craniopharyngioma, and brain tumor.

In the composition according to any one of the previous embodiments, the cancer may have a low responsiveness to a PD-1 antagonist.

In the composition according to any one of the previous embodiments, the cancer having a low responsiveness to a PD-1 antagonist may be an immune-infiltrating tumor (cold tumor).

In the composition according to any one of the previous embodiments, the composition may be administered via an intraperitoneal, intravenous, intramuscular, subcutaneous, intradermal, oral, topical, intranasal, intrapulmonary, or intrarectal route.

In the composition according to any one of the previous embodiments, the composition may be administered at time intervals ranging from 1 week to 1 month.

In the composition according to any one of the previous embodiments, the composition may be administered in combination with a composition containing an immune checkpoint inhibitor simultaneously, separately, sequentially, or in reverse order.

In accordance with another aspect of the present invention, there is provided a method for preventing or treating cancer, the method including administering to a subject in need thereof the interleukin-2 analog or long-acting conjugate thereof or a composition containing the same, and an immune checkpoint inhibitor or a composition containing the same.

In accordance with another aspect of the present invention, there is provided the combined use of the interleukin-2 analog or long-acting conjugate thereof or a composition containing the same, and an immune checkpoint inhibitor or a composition containing the same, for preventing or treating cancer.

In accordance with another aspect of the present invention, there is provided the use of the interleukin-2 analog or long-acting conjugate thereof or a composition containing the same, which is administered in combination with an immune checkpoint inhibitor, in the preparation of a medicine for the prevention or treatment of cancer.

In accordance with another aspect of the present invention, there is provided the interleukin-2 analog or long-acting conjugate thereof or a kit including the same, wherein the interleukin-2 analog or long-acting conjugate thereof is administered in combination with an immune checkpoint inhibitor.

**[Mode for Invention]**

Hereinafter, detailed contents for implementing the present invention will be described below. Each description and embodiment disclosed in the present application may also be applied to other descriptions and embodiments. That is, all combinations of various elements disclosed in this disclosure fall within the scope of the present invention. Furthermore, the scope of the present invention is not limited by the specific description below. Furthermore, throughout the overall specification, many papers and patent documents are referenced and their citations are provided. The disclosures of cited papers and patent documents are entirely incorporated by reference herein, and the level of the technical field within which the present invention falls and details of the present invention are explained more clearly.

Through the entire specification, conventional 1-letter and 3-letter codes for amino acids are used. The amino acids mentioned in abbreviations herein are described according to the IUPAC-IUB rules.

| | |
|---|---|
| alanine A, | arginine R |
| asparagine N, | aspartic acid D |
| cysteine C, | glutamic acid E |
| glutamine Q, | glycine G |
| histidine H, | isoleucine I |
| leucine L, | lysine K |
| Methionine M, | phenylalanine F |

(continued)

| | |
|---|---|
| proline P, | serine S |
| threonine T, | tryptophan W |
| tyrosine Y, | valine V |

In an aspect of the present invention, there is provided a composition for prevention or treatment of cancer, the composition containing an interleukin-2 analog or long-acting conjugate thereof, wherein the composition is administered in combination with an immune checkpoint inhibitor.

The interleukin-2 analog of the present invention may have an altered binding affinity for an interleukin-2 receptor, particularly, an increased binding affinity for the interleukin-2 beta receptor. Specifically, the interleukin-2 analog of the present invention may have an increased binding affinity for the interleukin-2 beta receptor compared with native interleukin-2 or known aldesleukin, and more specifically, may also have an altered (increased or reduced) binding affinity for the interleukin-2 alpha receptor and may include a sequence with a modification of at least one amino acid in the native interleukin-2.

A specific aspect of the composition of the present invention may include an interleukin-2 analog including, consisting of, or consisting essentially of any one sequence selected from the amino acid sequences of SEQ ID NOS: 3 to 106, but is not limited thereto.

Specifically, the composition of the present invention may be a pharmaceutical composition containing an interleukin-2 analog or long-acting conjugate thereof for preventing or treating cancer, and more specifically, a pharmaceutical composition administered in combination with an immune checkpoint inhibitor while containing an interleukin-2 analog or long-acting conjugate thereof, and a pharmaceutically acceptable excipient, but is not limited thereto.

The pharmaceutical composition of the present invention may be administered:

a) as one mixture where (i) an interleukin-2 analog or long-acting conjugate thereof; and (ii) an immune checkpoint inhibitor are mixed; or

b) in a form where (i) an interleukin-2 analog or long-acting conjugate thereof; and (ii) an immune checkpoint inhibitor are separated from each other, but are not limited thereto.

For example, an interleukin-2 analog or long-acting conjugate thereof and an immune checkpoint inhibitor may be formulated as one preparation or may be individually formulated. When the interleukin-2 analog or long-acting conjugate thereof and the immune checkpoint inhibitor are separated from each other, the interleukin-2 analog or long-acting conjugate thereof and the immune checkpoint inhibitor may be formulated as individual preparations and administered simultaneously, individually, sequentially, or in reverse order.

In the present invention, combined administration does not only refer to simultaneous administration, but may also be understood as a dosing form wherein the interleukin-2 analog or long-acting conjugate thereof and the immune checkpoint inhibitor act together on a subject so that each of the substances can perform functions thereof at a level equivalent to or higher than natural functions thereof. Therefore, the term "combined" or "in combination" used herein is to be understood as encompassing the administration of an interleukin-2 analog or long-acting conjugate thereof and an immune checkpoint inhibitor in any manner such as simultaneously, individually, sequentially, or in reverse order. When administration is performed sequentially, in reverse order, or individually, the order of administration is not particularly limited, but the interval of administration of the secondary ingredient should be determined so as not to lose beneficial effects of the combination.

The interleukin-2 analog or long-acting conjugate thereof and the immune checkpoint inhibitor, or the composition containing the same of the present invention may be provided in a kit form, but is not limited thereto. The term "kit" as used herein may include the composition according to the present invention for combined administration of an interleukin-2 analog or long-acting conjugate thereof and an immune checkpoint inhibitor. Specifically, the kit according to the present invention may include a single formulation of an interleukin-2 analog or long-acting conjugate thereof and an immune checkpoint inhibitor, or individual formulations of an interleukin-2 analog or long-acting conjugate thereof and an immune checkpoint inhibitor, and further include a substance required for combined administration of the two ingredients, but is not limited thereto.

The term "interleukin-2 (IL-2)" as used herein refers to a type of cytokine that signals in the immune system of the body and means an immunomodulator. The interleukin-2 is generally known as an important immunostimulator of about 15 kDa.

The term "interleukin-2 analog" as used herein refers to an analog having a modification of at least one amino acid in the sequence of the native form and, especially, may be an interleukin-2 analog having a modification of an amino acid in the interleukin-2, whereby the binding affinity for the receptor is increased or reduced compared with the native interleukin-2. Specifically, the interleukin-2 analog of the present invention may be a non-naturally occurring analog.

The native interleukin-2 may be human interleukin-2, the sequence of which may be available from known databases. Specifically, the sequence may be the amino acid sequence of SEQ ID NO: 1, but is not limited thereto.

In the present invention, the meaning that the native interleukin-2 may include the amino acid sequence of SEQ ID NO: 1 is that the sequence of the native interleukin-2 is not only the same as SEQ ID NO: 1, but a sequence having at least 80%, at least 85%, at least 90%, at least 91%, at least 92%, at least 93%, at least 94%, at least 95%, at least 96%, at least 97%, at least 98%, or at least 99% homology with SEQ ID NO: 1 also falls within the range of the native interleukin-2 of the present invention, wherein the position of an amino acid modification means that a modification occurs at the corresponding position in the amino acid sequence of SEQ ID NO: 1 when a sequence having a homology of at least 80%, at least 85%, at least 90%, at least 91%, at least 92%, at least 93%, at least 94%, at least 95%, at least 96%, at least 97%, at least 98%, or at least 99% is aligned with respect to SEQ ID NO: 1.

The term "aldesleukin" as used herein may refer to an interleukin-2 analog that is commercially available, and the aldesleukin may be aldesleukin (brand name: Proleukin®), and specifically, may have the amino acid sequence of SEQ ID NO: 2. Herein, the aldesleukin is used exchangeably with "interleukin-2 analog 1". The interleukin-2 analog according to the present invention may have an altered binding affinity for the interleukin-2 alpha receptor and/or an increased binding affinity for the interleukin-2 beta receptor compared with the interleukin-2 analog 1.

The interleukin-2 alpha receptor is known to be not involved in the signaling system of interleukin-2, but increases the binding affinity between interleukin-2 and the other interleukin-2 receptors (beta or gamma receptors) by 10 to 100 times and is expressed in $CD4^+$ regulatory T cells and the like.

Since the interleukin-2 beta receptor is mainly distributed in $CD8^+$ T cells or natural killer cells (NK cells) to perform an important role in activating the immune response and macrophage action, the activation of the interleukin-2 beta receptor can be predicted to achieve tumor cell death and body's immune response activation.

Therefore, the interleukin-2 analog of the present invention having an increased binding affinity for the interleukin-2 beta receptor can increase treatment effects, such as tumor suppression and death, and reduce side effects.

As one example, the interleukin-2 analog may include, consist essentially of, or consist of an amino acid sequence selected from the group consisting of SEQ ID NOS: 3 to 106, but is not limited thereto.

In one specific embodiment, the interleukin-2 analog may include, consist essentially of, or consist of any one sequence selected from the group consisting of amino acid sequences of SEQ ID NOS: 10, 13-15, 17, 20-22, 32, 35, 36, 42, 53, 54, 56, 58-60, 62, 71, 72, 74-78, 85, 87, 89, 91-94, 97-106, 10, 13, 14, 16, 17, 20, 21, 22, 32, 35, 36, 42, 53, 54, 87, 89, 91, 92, 93, 94, 98, 99, 100, 101, 103, 104, and 105, but is not limited thereto.

In another specific embodiment, the interleukin-2 analog may include, consist essentially of, or consist of any one sequence selected from the group consisting of amino acid sequences of SEQ ID NOS: 17, 22, 42, 53, 56, 58-60, 62, 71, 72, 74-77, 87, 89, 91-93, 98-101, and 103-106, but is not limited thereto. In another specific embodiment, the interleukin-2 analog may include, consist essentially of, or consist of any one sequence selected from the group consisting of amino acid sequences of SEQ ID NOS: 10, 13, 14, 15, 16, 17, 20, 21, 22, 32, 35, 36, 42, 53, 54, 56, 58, 59, 60, 62, 71, 72, 74, 75, 76, 77, 78, 85, 87, 89, 91, 92, 93, 94, 95, 98, 99, 100, 101, 103, 104, 105, and 106, but is not limited thereto.

In another specific embodiment, the interleukin-2 analog may include, consist essentially of, or consist of any one sequence selected from the group consisting of amino acid sequences of SEQ ID NOS: 10, 13, 14, 16, 17, 20, 21, 22, 32, 35, 36, 42, 53, 54, 87, 89, 91, 92, 93, 94, 98, 99, 100, 101, 103, 104, and 105, but is not limited thereto.

In another specific embodiment, the interleukin-2 analog may include, consist essentially of, or consist of any one sequence selected from the group consisting of amino acid sequences of SEQ ID NOS: 22, 42, 53, 87, 105, and 106, but is not limited thereto.

In addition, the interleukin-2 analog may further include at least one amino acid at the C-terminus, but is not limited thereto.

Although stated as "interleukin-2 analog consisting of a specific sequence number" herein, this terminology does not exclude the addition of nonsense sequences upstream or downstream of the amino acid sequence of the corresponding sequence number or naturally-occurring mutations, or silent mutations thereof, as long as the analog has the equivalent or corresponding activity to the interleukin-2 analog consisting of amino acid sequence of the corresponding sequence number, and it is apparent that an analog having such sequence addition or mutation falls within the scope of the present invention.

The interleukin-2 analog of the present invention may include amino acid sequences having at least 40%, at least 45%, at least 50%, at least 55%, at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% homology or identity with the amino acid sequences of SEQ ID NOS: 3 to 106, but is not limited thereto.

The term "homology" or "identity" as used herein refers to a degree of relevance between two given amino acid sequences or nucleotide sequences, and the term may be expressed as a percentage.

The sequence homology or identity of conserved polynucleotides or polypeptides may be determined by standard alignment algorithms, and default gap penalties established by a program to be used may be used together.

Substantially, homologous or identical sequences may hybridize under moderately or highly stringent conditions along their entire sequence or a part thereof. It is apparent that hybridization also includes hybridization of a polynucleotide with a polynucleotide, which includes a general codon or a codon where codon degeneracy is considered.

The terms homology and identity may be often used interchangeably with each other.

Whether any two nucleotide or peptide sequences have homology, similarity, or identity may be determined using any computer algorithm known in the art, such as the "FASTA" program, using default parameters disclosed as in Pearson et al (1988) [Proc. Natl. Acad. Sci. USA 85]: 2444. Alternatively, this may be determined using the Needleman-Wunsch algorithm (Needleman and Wunsch, 1970, J. Mol. Biol. 48: 443-453), which is performed in the Needleman program of the European Molecular Biology Open Software Suite (EMBOSS) package (Rice et al., 2000, Trends Genet. 16: 276-277) (version 5.0.0 or versions thereafter) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.] (1988) SIAM J Applied Math 48: 1073). For example, the homology, similarity, or identity may be determined using BLAST of the National Center for Biotechnology Information database, or ClustalW.

The homology, similarity, or identity between polynucleotides or polypeptides may be determined by comparing sequence information using the GAP computer program, for example, Needleman et al., (1970), J Mol Biol. 48:443, as known in Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. Default parameters for the GAP program may include (1) a unary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358, 1979 (or the EDNAFULL (EMBOSS version of NCBI NUC4.4) substitution matrix); (2) a penalty of 3.0 for each gap and an additional 0.10 penalty for each symbol in each gap (or a gap opening penalty of 10 and a gap extension penalty of 0.5); and (3) no penalty for end gaps. Therefore, the term "homology" or "identity" as used herein represents the relevance between sequences

The interleukin-2 analog of the present invention can be used as a novel interleukin-2 substitute that has an altered in vitro activity by weakening or increasing of the binding affinity for the interleukin-2 alpha and/or beta receptors. In particular, the interleukin-2 analog of the present invention can be used as an effective medicine due to the activities for the two types of receptors since it has an increased binding affinity for the beta receptor as well as an altered (i.e., increased or reduced) binding affinity for the alpha receptor.

In the present invention, such a modification for preparing an interleukin-2 analog encompasses all of a modification using an L-type or D-type amino acid and/or a non-native amino acid; and/or a modification of a native sequence, for example, a modification of a side-chain functional group, an intramolecular covalent bonding (e.g., a ring formation between side chains), methylation, acylation, ubiquitination, phosphorylation, aminohexanation, biotinylation, and the like.

Additionally, the modification encompasses all of those where one or more amino acids are added to the amino and/or carboxy terminus of the native interleukin-2.

Regarding the substitution or addition of amino acids, not only the 20 amino acids commonly observed in human proteins, but also atypical or non-naturally occurring amino acids may be used. Commercial sources of atypical amino acids may include Sigma-Aldrich, ChemPep, and Genzyme Pharmaceuticals. The peptides including these amino acids and typical peptide sequences may be synthesized and purchased from companies specialized in the synthesis of commercial peptides, for example, American Peptide Company and Bachem in USA, or Anygen in Korea.

Amino acid derivatives may also be accessible in a similar manner, and for example, 4-imidazoacetic acid or the like may be used.

For the protection from in vivo proteolytic enzymes and the increase in stability, the interleukin-2 analog according to the present invention may be in a modified form in which the N-terminus and/or C-terminus thereof is chemically modified or protected by organic groups, or amino acids may be added to the peptide terminus.

In particular, a chemically synthesized peptide has electrically charged N- and C-termini, and thus for elimination of these charges, the N-terminus may be acetylated and/or the C-terminus may be amidated, but is not particularly limited thereto.

The interleukin-2 analog of the present invention may be synthesized through a solid-phase synthesis method, produced by a recombinant method, or may be manufactured upon commercial request, but is not limited thereto

In addition, the interleukin-2 analog of the present invention may be synthesized depending on the length thereof by a method well-known in the art, for example, by an automatic peptide synthesizer, and may also be produced by genetic engineering technology.

Specifically, the interleukin-2 analog of the present invention may be prepared by a standard synthesis method, a recombinant expression system, or any other method known in the art. Accordingly, the interleukin-2 analog according to the present invention may be synthesized by a number of methods including, for example, the following:

(a) a method where a peptide is synthesized by a solid-phase or liquid-phase process stepwise or by fragment assembly, and a final peptide product is separated and purified;

(b) a method where a nucleic acid construct encoding a peptide is expressed in a host cell and an expression product is collected from a host cell culture;

(c) a method where the in vitro cell-free expression of a nucleic acid construct encoding a peptide is performed and an expression product is collected therefrom; or

a method where peptide fragments are obtained by any combination of (a), (b), and (c), the fragments are linked to obtain a peptide, and then the corresponding peptide is collected.

In the present invention, the binding affinity of any interleukin-2 analog (or long-acting conjugate including the same) for native interleukin-2 receptors may be measured using surface plasmon resonance (SPR), which is a method for measuring the affinity for the receptors.

Specifically, upon the SPR analysis, there may be: a method, using the protein-ligand binding principle, in which an interleukin-2 receptor is immobilized to a sensor chip and an interleukin-2 analog diluted in an experimental buffer by serial dilution is flowed to induce the binding to the immobilized receptor, and then, only an experimental buffer is flowed at the same flow rate to induce the dissociation between the interleukin-2 analog and the receptor, thereby measuring the binding affinity; or a method in which an antibody to a human immunoglobulin Fc region is immobilized to a sensor chip, and then an interleukin-2 receptor to which an Fc region is bound is immobilized, and an interleukin-2 analog is flowed to measure binding affinity, but the binding affinity measurement method is not limited thereto.

More specifically, a biotin-labeled human interleukin-2 receptor is immobilized to a streptavidin biosensor chip, and a long-acting conjugate of an interleukin-2 analog diluted to HBS-EP+ buffer by two-fold serial dilution is flowed at a flow rate of 20 μL/min for 3 minutes, and then only HBS-EP+ buffer is flowed at the same flow rate to induce the dissociation between the interleukin-2 receptor and the long-acting conjugate of the interleukin-2 analog, and thereafter, the obtained association constant and dissociation constant are used to measure the binding affinity according to the 1:1 binding fitting model using the Biaevaluation program, but is not limited thereto.

More specifically, the interleukin-2 analog of the present invention may have a reduced or increased binding affinity for the interleukin-2 alpha receptor compared with native interleukin-2 or aldesleukin (or interleukin-2 analog 1).

Specifically, the interleukin-2 analog of the present invention may have a binding affinity for the interleukin-2 alpha receptor that is about 0.001-fold or greater, about 0.005-fold or greater, about 0.01-fold or greater, about 0.05-fold or greater, about 0.1-fold or greater, about 0.3-fold or greater, about 0.5-fold or greater, about 0.7-fold or greater, about 0.9-fold or greater, about 1.1-fold or greater, about 1.3-fold or greater, about 1.5-fold or greater, or about 1.7-fold or greater, compared with the binding affinity of native interleukin-2 or aldesleukin for the interleukin-2 alpha receptor, but the numerical value of the binding affinity is not limited, and the numerical value falls within the scope of the present invention as long as the binding affinity thereof is altered compared with that of native interleukin-2 or aldesleukin.

Alternatively, relative to the binding affinity (100%) of aldesleukin for the interleukin-2 alpha receptor, the interleukin-2 analog of the present invention may have no binding affinity for the interleukin-2 alpha receptor or have a binding affinity for the interleukin-2 alpha receptor of about 1% or more, about 5% or more, about 7% or more, about 10% or more, about 15% or more, about 20% or more, about 30% or more, about 50% or more, about 70% or more, about 90% or more, about 100% or more, about 150% or more, or about 200% or more, but the numerical value of the binding affinity is not limited, and the numerical value falls within the scope of the present invention as long as the binding affinity thereof is altered compared with that of native interleukin-2 or aldesleukin.

In addition, specifically, the interleukin-2 analog of the present invention may have a binding affinity for the interleukin-2 beta receptor of about 0.1-fold or greater, about 0.3-fold or greater, about 0.5-fold or greater, about 0.7-fold or greater, about 1.0-fold or greater, about 10-fold or greater, about 20-fold or greater, about 30-fold or greater, about 40-fold or greater, about 50-fold or greater, about 60-fold or greater, about 70-fold or greater, about 80-fold or greater, about 90-fold or greater, or about 100-fold or greater, compared with the binding affinity of native interleukin-2 or aldesleukin for the interleukin-2 beta receptor, but the numerical value of the binding affinity is not limited, and the numerical value falls within the scope of the present invention as long as the binding affinity thereof is altered or increased compared with that of native interleukin-2 or aldesleukin.

Alternatively, relative to the binding affinity (100%) of aldesleukin for the interleukin-2 beta receptor, the interleukin-2 analog of the present invention may have a binding affinity for the interleukin-2 beta receptors of about 5% or more, about 9% or more, about 10% or more, about 20% or more, about 30% or more, about 50% or more, about 100% or more, about 200% or more, about 500% or more, about 700% or more, about 1,000% or more, about 1,500% or more, about 3,000% or more, about 5,000% or more, about 7,000% or more, about 10,000% or more, about 12,000% or more, about 15,000% or more, about 20,000% or more, or about 25,000% or more, but the numerical value of the binding affinity is not limited, and the numerical value falls within the scope of the present invention as long as the binding affinity thereof is increased compared with that of native interleukin-2 or aldesleukin.

The term "about" as used herein refers to a range encompassing ±0.5, ±0.4, ±0.3, ±0.2, ±0.1, and the like, and

includes all of the values in the range equivalent or similar to those stated after this term, but is not limited thereto.

The interleukin-2 analog of the present invention has an altered binding affinity for the interleukin-2 alpha receptor and an increased binding affinity for the interleukin-2 beta receptor, compared with native interleukin-2 or aldesleukin.

In a specific embodiment of the present invention, for the preparation of the interleukin-2 analog of the present invention, an interleukin-2 analog was produced by introducing a mutation(s) into native interleukin-2 (SEQ ID NO: 1). The interleukin-2 analog prepared in the present invention may include an amino acid sequence of any one of SEQ ID NOS: 3 to 106 or may be encoded by a nucleotide sequence of any one of SEQ ID NOS: 108 to 211.

A nucleic acid encoding the interleukin-2 analog of the present invention may be modified so that a modification (deletion, substitution, and/or addition of an amino acid) can be introduced into an amino acid(s) at a specific position(s) in a nucleotide sequence encoding the native interleukin-2 of SEQ ID NO: 1 and, specifically, may include a nucleotide sequence encoding an amino acid sequence of any one of SEQ ID NOS: 3 to 106. As one example, the nucleic acid of the present invention may have or include a nucleotide sequence of any one of SEQ ID NOS: 108 to 211.

In the polynucleotide of the present invention, various modifications may be made in the coding region within a range that does not alter the amino acid sequence of the interleukin-2 analog of the present invention, considering codon degeneracy or codons preferred in an organism where the nucleic acid of the present invention is to be expressed. Specifically, the nucleic acid of the present invention may have or include a nucleotide sequence having at least 70%, at least 75%, at least 80%, at least 85%, at least 90%, at least 95%, at least 96%, at least 97%, or at least 98%, and less than 100% homology or identity to a sequence of any one of SEQ ID NOS: 108 to 211, or may consist of or consist essentially of a nucleotide sequence having at least 70%, at least 75%, at least 80%, at least 85%, at least 90%, at least 95%, at least 96%, at least 97%, or at least 98%, and less than 100% homology or identity to a sequence of any one of SEQ ID NOS: 108 to 211, but is not limited thereto.

Additionally, the nucleic acid of the present invention may include, without limitation, a probe that can be prepared from a known gene sequence, for example, a sequence that can hybridize with a sequence complementary to all or part of the nucleic acid sequence of the present invention under stringent conditions. The term "stringent conditions" refers to conditions that enable specific hybridization between polynucleotides. Such conditions are described in detail in the literature (see J. Sambrook et al., Molecular Cloning, A Laboratory Manual, 2nd Edition, Cold Spring Harbor Laboratory press, Cold Spring Harbor, New York, 1989; and F. M. Ausubel et al., Current Protocols in Molecular Biology, John Wiley & Sons, Inc., New York, 9.50-9.51, 11.7-11.8).

Hybridization requires that two nucleic acids have complementary sequences although mismatches between bases are possible depending on the stringency of the hybridization. The term "complementary" is used to describe the relationship between nucleotide bases that can hybridize with each another. For example, with respect to DNA, adenine is complementary to thymine and cytosine is complementary to guanine. Therefore, the nucleic acid of the present invention may include isolated nucleic acid fragments complementary to the entire sequence as well as substantially similar nucleic acid sequences.

The appropriate stringency for hybridizing polynucleotides depends on the length of the polynucleotides and the degree of complementarity, and variables are well known in the art (e.g., Sambrook et al., supra).

Both homology and identity are as described above.

Furthermore, the interleukin-2 analog of the present invention may have an increased in vivo half-life, compared with native interleukin-2 or aldesleukin, but is not particularly limited thereto. For example, the interleukin-2 analog of the present invention may include a form of a long-acting conjugate having an increased half-life by linking a biocompatible substance (e.g., an immunoglobulin Fc region) for increasing the half-life to the interleukin-2 analog directly or via a linker, but is not limited thereto.

The long-acting conjugate according to the present invention not only includes an interleukin-2 analog with an increased binding affinity for the interleukin-2 beta receptor but also includes an immunoglobulin Fc region as a representative carrier for increasing the half-life thereof, thereby increasing the half-life of the interleukin-2 analog, increasing the blood exposure, and increasing the in vivo immune response, and thus, effectively achieving the growth inhibition and reduction of cancer cells.

The above description can be applied to other embodiments or aspects of the present invention, but is not limited thereto.

Another aspect to implement the composition of the present invention may include the long-acting conjugate of the interleukin-2 analog, but is not limited thereto.

In the present invention, the long-acting conjugate of the interleukin-2 analog may be in the form in which a biocompatible substance capable of increasing the in vivo half-life thereof is linked to the interleukin-2 analog. Herein, the biocompatible substance may be used interchangeably with a carrier.

In the present invention, the long-acting conjugate may exhibit an increased duration of efficacy compared with an interleukin-2 analog, to which a carrier is not conjugated, and in the present invention, such a conjugate is referred to as a "long-acting conjugate" or "conjugate".

Such a conjugate may be a non-naturally occurring conjugate.

In an embodiment of the present invention, the long-acting conjugate is a long-acting conjugate represented by Chemical Formula 1 below:

[Chemical Formula 1] X-L-F

where: X is the interleukin-2 analog;
L is a polyethylene glycol linker;
F is a dimeric immunoglobulin Fc region, and
the - symbols represent covalent linkages between X and L and between L and F, respectively,
wherein in the long-acting conjugate, one end of L is covalently linked to only one polypeptide chain of the dimeric Fc region and X is covalently linked to the other end of L.

More specifically, in the long-acting conjugate, one molecule of X may be linked to one polypeptide chain of the Fc region via L. In the long-acting conjugate of the present invention, X and L and L and F may be linked to each other through covalent linkages, respectively, wherein the conjugate may be a conjugate in which X, L, and F are linked to each other through covalent linkages in the form of Chemical Formula 1.

The interleukin-2 analog in the long-acting conjugate of the present invention has an altered binding affinity for an interleukin-2 receptor, especially, an increased binding affinity for the interleukin-2 beta receptor, when present alone, without being a part of the conjugate. Specifically, the interleukin-2 analog of the present invention may have an increased binding affinity for the interleukin-2 beta receptor, and more specifically, may also have an altered (increased or reduced) binding affinity for the interleukin-2 alpha receptor, compared with native interleukin-2 or known aldesleukin, when present alone, without being a part of the conjugate.

In the present invention, the interleukin-2 analog may correspond to one moiety constituting the conjugate. Specifically, the interleukin-2 analog corresponds to X in Chemical Formula 1, and the interleukin-2 analog is as described above.

In the conjugate, F is a substance capable of increasing the half-life of X, i.e., the interleukin-2 analog, and corresponds to one moiety constituting the conjugate of the present invention.

F and X may be linked to each other by a covalent chemical linkage, and F and X may be linked to each other via L by a covalent chemical linkage.

Specifically, F is an immunoglobulin Fc region, and the immunoglobulin Fc region may be an IgG Fc region or an aglycosylated IgG4 Fc region, but is not particularly limited thereto.

As a specific example of the present invention, F (immunoglobulin Fc region) is a dimer consisting of two polypeptide chains, and may have a structure in which one end of L is linked only to one of the two polypeptide chains, but not limited thereto.

At least one amino acid side chain within the peptide of the present invention may be conjugated to such a biocompatible substance to increase solubility and/or half-life in vivo, and/or increase bio-availability thereof. These modifications may also reduce the clearance of therapeutic proteins and peptides.

The above-described biocompatible substance may be soluble (amphipathic or hydrophilic) and/or non-toxic and/or pharmaceutically acceptable.

In a specific embodiment, the long-acting conjugate of the present invention may have the interleukin-2 analog and the immunoglobulin Fc region linked to each other, but is not limited thereto.

In the present invention, the term "immunoglobulin Fc region" refers to a region that includes heavy chain constant region 2 (CH2) and/or heavy chain constant region 3 (CH3) domains, excluding heavy chain and light chain variable regions of an immunoglobulin. The immunoglobulin Fc region may be one moiety constituting the conjugate of the present invention. Specifically, the immunoglobulin Fc region corresponds to F in Chemical Formula 1.

Herein, the Fc region includes not only a native sequence obtained by papain digestion of an immunoglobulin, but also derivatives thereof, for example, variants, in which one or more amino acid residues in the native sequence are converted by deletion, insertion, non-conservative or conservative substitution, or a combination thereof, and thus become different from the native sequence or the like. The derivatives, substituents, and variants are assumed to retain the ability to bind to FcRn. In the present invention, F may be a human immunoglobulin region, but is not limited thereto. Herein, the "biocompatible substance" or "carrier" may refer to the Fc region.

F (immunoglobulin Fc region) may have a structure in which two polypeptide chains are linked to each other via a disulfide linkage, only through a nitrogen atom of one of the two chains, but is not limited thereto. The linking via the nitrogen atom may be made through the reductive amination of an ε-amino group or an N-terminal amino group of lysine.

The reductive amination reaction refers to a reaction in which an amine group or an amino group of a reactant reacts with an aldehyde (i.e., a functional group capable of reductive amination) of another reactant to produce an amine and then forms an amine bond by a reduction reaction, and this is an organic synthesis reaction well known in the art.

In one embodiment with respect to the long-acting conjugate of the present invention, the immunoglobulin Fc

region is linked to the linker via a nitrogen atom at the N-terminus thereof.

This immunoglobulin Fc region may include a hinge region in the heavy chain constant region, but is not limited thereto.

In the present invention, the immunoglobulin Fc region may include a specific hinge sequence at the N-terminus.

As used herein, the term "hinge sequence" refers to a site which is located on a heavy chain and forms a dimer of the immunoglobulin Fc region through an inter-disulfide bond.

In the present invention, the hinge sequence may be modified to have only one cysteine residue by deletion of a part in a hinge sequence having the following amino acid sequence, but is not limited thereto:
Glu-Ser-Lys-Tyr-Gly-Pro-Pro-Cys-Pro-Ser-Cys-Pro (SEQ ID NO: 418).

The hinge sequence may include only one cysteine residue by deletion of the cysteine residue at position 8 or 11 in the hinge sequence of SEQ ID NO: 418. The hinge sequence of the present invention may consist of 3 to 12 amino acids including only one cysteine residue, but is not limited thereto. More specifically, the hinge sequence of the present invention may have the following sequence: Glu-Ser-Lys-Tyr-Gly-Pro-Pro-Pro-Ser-Cys-Pro (SEQ ID NO: 419), Glu-Ser-Lys-Tyr-Gly-Pro-Pro-Cys-Pro-Ser-Pro (SEQ ID NO: 420), Glu-Ser-Lys-Tyr-Gly-Pro-Pro-Cys-Pro-Ser (SEQ ID NO: 421), Glu-Ser-Lys-Tyr-Gly-Pro-Pro-Cys-Pro-Pro (SEQ ID NO: 422), Lys-Tyr-Gly-Pro-Pro-Cys-Pro-Ser (SEQ ID NO: 423), Glu-Ser-Lys-Tyr-Gly-Pro-Pro-Cys (SEQ ID NO: 424), Glu-Lys-Tyr-Gly-Pro-Pro-Cys (SEQ ID NO: 425), Glu-Ser-Pro-Ser-Cys-Pro (SEQ ID NO: 426), Glu-Pro-Ser-Cys-Pro (SEQ ID NO: 427), Pro-Ser-Cys-Pro (SEQ ID NO: 428), Glu-Ser-Lys-Tyr-Gly-Pro-Pro-Ser-Cys-Pro (SEQ ID NO: 429), Lys-Tyr-Gly-Pro-Pro-Pro-Ser-Cys-Pro (SEQ ID NO: 430), Glu-Ser-Lys-Tyr-Gly-Pro-Ser-Cys-Pro (SEQ ID NO: 431), Glu-Ser-Lys-Tyr-Gly-Pro-Pro-Cys (SEQ ID NO: 432), Lys-Tyr-Gly-Pro-Pro-Cys-Pro (SEQ ID NO: 433), Glu-Ser-Lys-Pro-Ser-Cys-Pro (SEQ ID NO: 434), Glu-Ser-Pro-Ser-Cys-Pro (SEQ ID NO: 435), Glu-Pro-Ser-Cys (SEQ ID NO: 436), and Ser-Cys-Pro (SEQ ID NO: 437).

More specifically, the hinge sequence may include an amino acid sequence of SEQ ID NO: 428 (Pro-Ser-Cys-Pro) or SEQ ID NO: 437 (Ser-Cys-Pro), but is not limited thereto.

In a more specific embodiment of the long-acting conjugate of the present invention, in the conjugate, the N-terminus of the immunoglobulin Fc region is proline, and in the conjugate, the Fc region is linked to the linker via a nitrogen atom of the proline.

In an embodiment of the long-acting conjugate of the present invention, the immunoglobulin Fc region may be a dimeric form in which two chains of the immunoglobulin Fc region form a homodimer or a heterodimer due to the presence of the hinge sequence. The conjugate of Chemical Formula 1 of the present invention may be in the form in which one end of the linker is linked to one chain of the immunoglobulin Fc region of the dimer, but is not limited thereto.

As used herein, the term "N-terminus" refers to an amino terminus of a protein or polypeptide, and may include the outermost end of the amino terminus, or 1, 2, 3, 4, 5, 6, 7, 8, 9, or 10 or more amino acids from the outermost end. In the immunoglobulin Fc region of the present invention, a hinge sequence may be included in the N-terminus, but is not limited thereto.

Furthermore, the immunoglobulin Fc region of the present invention may be an extended Fc region including a part of or the entire heavy chain constant region 1 (CH1) and/or light chain constant region 1 (CL1), excluding the heavy chain and light chain variable regions of an immunoglobulin, as long as it has an effect that is substantially equivalent or improved as compared to its native form. Alternatively, the immunoglobulin Fc region may be a region in which a part of a significantly long amino acid sequence corresponding to CH2 and/or CH3 is removed.

For example, the immunoglobulin Fc region of the present invention may be 1) CH1 domain, CH2 domain, CH3 domain, and CH4 domain, 2) CH1 domain and CH2 domain, 3) CH1 domain and CH3 domain, 4) CH2 domain and CH3 domain, 5) a combination of one or more domains among CH1 domain, CH2 domain, CH3 domain, and CH4 domain, and an immunoglobulin hinge region (or a part of the hinge region), and 6) a dimer of each domain of the heavy chain constant region and the light chain constant region, but is not limited thereto.

In the present invention, the immunoglobulin Fc region may be in the form of a dimer or multi-mer consisting of single-chain immunoglobulins consisting of domains of the same origin, but is not limited thereto.

In an embodiment of the long-acting conjugate of the present invention, the immunoglobulin Fc region, F, is a dimer consisting of two polypeptide chains, wherein the dimeric Fc region, F, and X may be covalently linked to each other via one linker L containing ethylene glycol repeating units. In a specific embodiment, X is covalently linked only to one of the two polypeptide chains of the dimeric Fc region, F, via the linker L. In a more specific embodiment, only one molecule of X may be covalently linked via L to one polypeptide chain, to which X is linked, of the two polypeptide chains of the dimeric Fc region, F. In a most specific embodiment, F is a homodimer.

In another embodiment, the immunoglobulin Fc region, F, may be a dimer consisting of two polypeptide chains, wherein one end of L is linked only to one polypeptide chain of the two polypeptide chains, but is not limited thereto.

In another embodiment with respect to the long-acting conjugate of the present invention, two X molecules may be symmetrically linked to one Fc region in a dimeric form. Specifically, the immunoglobulin Fc region and X may be linked to each other via L. However, such linking is not limited to the above-described embodiments.

The immunoglobulin Fc region of the present invention includes not only the native amino acid sequence as well

as a derivative thereof. The amino acid sequence derivative means that one or more amino acid residues in the native amino acid sequence have a different sequence due to deletion, insertion, non-conservative or conservative substitution, or a combination thereof.

For example, the amino acid residues at positions 214 to 238, 297 to 299, 318 to 322, or 327 to 331, which are known to be important for linkage in IgG Fc, may be used as appropriate sites for variation.

In addition, various types of derivatives are possible by, for example, removing a site capable of forming a disulfide bond, deleting some amino acid residues at the N-terminus of native Fc, or adding a methionine residue at the N-terminus of native Fc. In addition, for removal of effector functions, complement-binding sites, for example, a C1q-binding site, may be removed, and an antibody dependent cell mediated cytotoxicity (ADCC) site may be removed. The techniques for preparing the sequence derivatives of the immunoglobulin Fc region are disclosed in WO 97/34631 and WO 96/32478, and the like.

Amino acid exchanges in proteins and peptides, which do not alter the entire activity of molecules, are well known in the art (H. Neurath, R. L. Hill, The Proteins, Academic Press, New York, 1979). The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, amino acids may be modified by phosphorylation, sulfation, acrylation, glycosylation, methylation, farnesylation, acetylation, amidation, or the like.

The above-described Fc derivatives exhibit a biological activity equivalent to that of the Fc region of the present invention, and may have an increased structural stability against heat, pH, and the like compared with the Fc region.

Such an Fc region may be obtained from native forms isolated from living bodies of humans or animals, such as cows, goats, pigs, mice, rabbits, hamsters, rats, and guinea pigs, or may be recombinant forms or derivatives thereof obtained from transformed animal cells or microorganisms. In particular, the Fc region may be obtained from a native immunoglobulin by isolating a whole immunoglobulin from a living human or animal body and then treating the isolated immunoglobulin with protease. The isolated immunoglobulin is digested into Fab and Fc when treated with papain, and digested into pF'c and $F(ab)_2$ when treated with pepsin. These fragments may be subjected to size exclusion chromatography or the like to separate Fc or pF'c therefrom. In a more specific embodiment, the immunoglobulin Fc region is a recombinant immunoglobulin Fc region where a human-derived Fc region is obtained from a microorganism.

In addition, the immunoglobulin Fc region may be in the form of having native glycans, increased glycans compared with a native form or decreased glycans compared with the native form, or may be in a deglycosylated form. The increase, decrease, or removal of the immunoglobulin Fc glycans may be achieved by using conventional methods, such as a chemical method, an enzymatic method, and a genetic engineering method using a microorganism. Particularly, the immunoglobulin Fc region obtained by removal of glycans from Fc exhibits a shape deterioration in binding affinity for the complement c1q and a reduction or loss in antibody-dependent cytotoxicity or complement-dependent cytotoxicity, and thus cause no unnecessary immune response in vivo. In this regard, an immunoglobulin Fc region in a deglycosylated or aglycosylated form may be more suitable to the objective of the present invention as a drug carrier.

As used herein, the term "deglycosylation" indicates an Fc region from which a glycan is removed by an enzyme, and the term "aglycosylation" indicates an Fc region that is produced in prokaryote, more specifically *E. coli,* and is not glycosylated.

Meanwhile, the immunoglobulin Fc region may be derived from humans or other animals including cattle, goats, pigs, mice, rabbits, hamsters, rats, and guinea pigs and, more specifically from humans.

In addition, the immunoglobulin Fc region may be an Fc region derived from IgG, IgA, IgD, IgE and IgM, or that is made by combinations thereof or hybrids thereof. In a still more specific embodiment, the immunoglobulin Fc region is derived from IgG or IgM, which is most abundant in the human blood, and in a still more specific embodiment, the immunoglobulin Fc region is derived from IgG, which is known to increase the half-lives of ligand-binding proteins. In a still more specific embodiment, the immunoglobulin Fc region is an IgG4 Fc region, and in a most specific embodiment, the immunoglobulin Fc region is an aglycosylated Fc region derived from human IgG4, but is not limited thereto.

In a specific embodiment, the immunoglobulin Fc region may be a human IgG4 Fc fragment in the form of a homodimer in which two monomers are linked to each other via a disulfide bond (inter-chain form) formed between cysteines that are amino acids located at position 3 in each monomer. In particular, each monomer of the homodimer has/may have a disulfide bond formed between cysteine at position 35 and cysteine at position 95 and a disulfide bond formed between cysteine at position 141 and cysteine at position 199, that is, two disulfide bonds (intra-chain form).

With respect to the number of amino acids, each monomer may consist of 221 amino acids and the number of amino acids constituting the homodimer may be 442 in total, without being limited thereto. Specifically, the immunoglobulin Fc fragment may be in the form of a homodimer in which two monomers each having an amino acid sequence of SEQ ID NO: 438 (consisting of 221 amino acids) are linked to each other via a disulfide bond between cysteines, each of which is an amino acid at position 3 of each monomer, wherein the monomers of the homodimer have each independently an intra-chain disulfide bond formed between cysteines at positions 35 and 95 and an intra-chain disulfide bond formed between cysteines at positions 141 and 199, but is not limited thereto.

In Chemical Formula 1, F may include a monomer of the amino acid sequence of SEQ ID NO: 438, and F may be a homodimer of the monomer of the amino acid sequence of SEQ ID NO: 438, but is not limited thereto.

As one example, the immunoglobulin Fc region may be a homodimer including the amino acid sequence of SEQ ID NO: 439 (consisting of 442 amino acids), but is not limited thereto.

In an embodiment, the immunoglobulin Fc region and X may be aglycosylated, but are not limited thereto.

The term "combination" as used herein means that polypeptides encoding single-chain immunoglobulin Fc regions of the same origin are linked to a single-chain polypeptide of a different origin to form a dimer or multimer. That is, a dimer or multimer may be prepared from two or more fragments selected from the group consisting of IgG Fc, IgA Fc, IgM Fc, IgD Fc, and IgE Fc fragments.

The term "hybrid" as used herein means that sequences encoding two or more immunoglobulin Fc fragments of different origin are present in a single-chain immunoglobulin constant region. In the present invention, several types of hybrid are possible. That is, the hybrid domain may be composed of one to four domains selected from the group consisting of CH1, CH2, CH3, and CH4 of IgG Fc, IgM Fc, IgA Fc, IgE Fc, and IgD Fc that can be hybridized, and may include a hinge region.

Meanwhile, IgG may also be divided into IgG1, IgG2, IgG3, and IgG4 subclasses, and the present invention may also include a combination thereof or a hybridization thereof. Specifically, the IgG2 and IgG4 subclasses are preferred, and most specifically, the Fc fragment of IgG4, which rarely has effector functions such as complement-dependent cytotoxicity (CDC) is preferred.

Additionally, the above-described conjugate may have an increased duration of efficacy compared with the native interleukin-2 or aldesleukin, or compared with X not modified with F, and such a conjugate may be not only in the above-described form but also in the form of being encapsulated in biodegradable nanoparticles, but is not limited thereto.

In the present invention, the "polyethylene glycol linker" includes a biocompatible polymer having two or more repeating units linked to each other. The repeating units are linked to each other by any covalent linkage but not a peptide linkage. The polyethylene glycol linker may be one moiety constituting the conjugate of the present invention. Herein, the polyethylene glycol linker may be used interchangeably with "non-peptidyl linker" or "non-peptidyl polymer".

In a specific embodiment, the conjugate may be one in which F and X are covalently linked to each other via a non-peptidyl linker including reactive groups capable of binding to F (specifically an immunoglobulin Fc region) and X (specifically an interleukin-2 analog) at both ends thereof.

Specifically, in the present invention, the non-peptidyl linker may include the reactive groups at the ends thereof and thus may form a conjugate by reaction with the other components constituting the conjugate. When the non-peptidyl linker having reactive functional groups at both ends thereof binds to X and F in Chemical Formula 1 through the respective reactive groups to form a conjugate, the non-peptidyl linker or non-peptidyl polymer may be named a non-peptidyl polymer linker moiety or a non-peptidyl linker moiety.

In a specific embodiment, L (polyethylene glycol linker) may be a linker containing ethylene glycol repeating units, for example, polyethylene glycol, but is not limited thereto. Herein, the polyethylene glycol is a term encompassing all of the forms of homopolymers of ethylene glycol, PEG copolymers, and monomethyl-substituted PEG polymers (mPEG), but is not particularly limited thereto. In addition, derivatives thereof that are already known in the art and derivatives that can be easily prepared within the skill in the art are also included in the scope of the present invention.

The polyethylene glycol linker may include, at the ends thereof, functional groups used in the preparation of a conjugate before being formed into the conjugate, while including the ethylene glycol repeating units. The long-acting conjugate according to the present invention may be in the form in which X and F are linked through the functional groups, but is not limited thereto. In the present invention, the non-peptidyl linker may include two, or three or more functional groups, and the respective functional groups may be identical to or different from each other, but is not limited thereto.

Specifically, the linker may include repeating units each represented by Chemical Formula 2 below. An example thereof may be polyethylene glycol (PEG), but is not limited thereto:

[Chemical Formula 2]

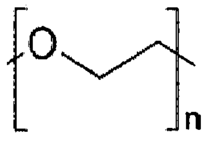

where n is 10 to 2,400, n is 10 to 480, or n is 50 to 250, but is not limited thereto.

In the long-acting conjugate, the PEG moiety may include a structure of - $(CH_2CH_2O)_n$- and an oxygen atom interposed between the linking element and - $(CH_2CH_2O)_n$-, but is not limited thereto.

In an embodiment, the ethylene glycol repeating units may be represented by, for example, $[OCH_2CH_2]n$, where

the n value is a natural number and may be determined such that the average molecular weight, for example, the number average molecular weight of the $[OCH_2CH_2]n$ moiety in the interleukin-2 analog conjugate is more than 0 kDa to about 100 kDa, but is not limited thereto. As one example, the n value is a natural number, and the average molecular weight, for example, the number average molecular weight of the $[OCH_2CH_2]n$ site in the interleukin-2 analog conjugate may be about 1 to about 100 kDa, about 1 to about 80 kDa, about 1 to about 50 kDa, about 1 to about 30 kDa, about 1 to about 25 kDa, about 1 to about 20 kDa, about 1 to about 15 kDa, about 1 to about 13 kDa, about 1 to about 11 kDa, about 1 to about 10 kDa, about 1 to about 8 kDa, about 1 to about 5 kDa, about 1 to about 3.4 kDa, about 2 to about 30 kDa, about 3 to about 30 kDa, about 3 to about 27 kDa, about 3 to about 25 kDa, about 3 to about 22 kDa, about 3 to about 20 kDa, about 3 to about 18 kDa, about 3 to about 16 kDa, about 3 to about 15 kDa, about 3 to about 13 kDa, about 3 to about 11 kDa, about 3 to about 10 kDa, about 3 to about 8 kDa, about 3 to about 5 kDa, about 3 to about 3.4 kDa, about 8 to about 30 kDa, about 8 to about 27 kDa, about 8 to about 25 kDa, about 8 to about 22 kDa, about 8 to about 20 kDa, about 8 to about 18 kDa, about 8 to about 16 kDa, about 8 to about 15 kDa, about 8 to about 13 kDa, about 8 to about 11 kDa, about 8 to about 10 kDa, about 9 to about 15 kDa, about 9 to about 14 kDa, about 9 to about 13 kDa, about 9 to about 12 kDa, about 9 to about 11 kDa, about 9.5 to about 10.5 kDa, or about 10 kDa, but is not limited thereto.

In a specific embodiment, the conjugate may have a structure in which an interleukin-2 analog and an immunoglobulin Fc region (F) are covalently linked through a linker (L) containing ethylene glycol repeating units, but is not limited thereto.

In another specific embodiment, in the long-acting conjugate, L may be a linker containing ethylene glycol repeating units, and F may be an immunoglobulin Fc region in a dimeric form. More specifically, one molecule of X may be covalently linked to one Fc region in a dimeric form of Fc region through the linker containing ethylene glycol repeating units, but is not limited thereto. In another specific embodiment, one end of the linker containing ethylene glycol repeating units may be linked to only one Fc region chain of two Fc region chains of the immunoglobulin Fc region in a dimeric form, but the immunoglobulin Fc region is not limited thereto.

The molecular weight of the polyethylene glycol linker usable in the present invention may be in the range of more than 0 to 200 kDa, specifically, about 1 to 100 kDa, about 1 to 50 kDa, about 1 to 30 kDa, about 2 to 30 kDa, about 1 to 20 kDa, more specifically about 3.4 kDa to 10 kDa, and still more specifically, about 3.4 kDa, but is not limited thereto. In addition, the non-peptidyl linker of the present invention, which is linked to the polypeptide corresponding to F may employ not only a single type of polymer but also a combination of different types of polymers.

The term "about" as used herein refers to a range encompassing ±0.5, ±0.4, ±0.3, ±0.2, ±0.1, and the like, and includes all of the values in the range equivalent or similar to those stated after this term, but is not limited thereto.

Specifically, the non-peptidyl linker may have reactive groups at both ends thereof while not binding to F and X, and F and X may be linked through the reactive groups.

In a specific embodiment, both ends of the linker may bind to a thiol group, an amino group, or a hydroxyl group of the immunoglobulin Fc region and to a thiol group, an amino group, an azide group, or a hydroxyl group of the interleukin-2 analog (X), but are not limited thereto.

Specifically, the linker may include, at both ends thereof, reactive groups capable of binding to the immunoglobulin Fc region and the interleukin-2 analog (X), respectively. Specifically, the linker may include a reactive group capable of binding to a thiol group of cysteine; an amino group located at the N-terminus, lysine, arginine, glutamine, and/or histidine; and/or a hydroxyl group at the C-terminus of the immunoglobulin Fc region; and to a thiol group of cysteine; an amino group of lysine, arginine, glutamine, and/or histidine; an azide group of azido-lysine; and/or a hydroxyl group of the interleukin-2 analog (X), but is not limited thereto.

More specifically, the reactive group of the linker may be one or more selected from the group consisting of an aldehyde group, a maleimide group, and a succinimide derivative, but is not limited thereto.

In the above description, examples of the aldehyde group may include a propionaldehyde group or a butyraldehyde group, but are not limited thereto.

In the above description, examples of the succinimide derivative may include succinimidyl valerate, succinimidyl methylbutanoate, succinimidyl methylpropionate, succinimidyl butanoate, succinimidyl propionate, N-hydroxysuccinimide, hydroxy succinimidyl, succinimidyl carboxymethyl, and succinimidyl carbonate, but are not limited thereto.

The linker may be linked to the immunoglobulin Fc region, F, and the interleukin-2 analog X via the reactive groups to thereby be converted into a linker moiety.

Furthermore, a final product produced by reductive alkylation through aldehyde bonds is more stable than a linkage formed by amide bonds. The aldehyde reactive group selectively reacts with the N-terminus at low pH while forming a covalent bond with a lysine residue at high pH, e.g., at a pH of 9.0.

In addition, the reactive groups of both ends of the linker may be the same as or different from each other and, for example, aldehyde groups may be provided at both ends, or a maleimide group may be provided at one end and an aldehyde group, a propionaldehyde group, or a butyraldehyde group may be provided at the other end. However, the reactive groups are not particularly limited as long as F, specifically the immunoglobulin Fc region, and X may be linked to the respective ends of the linker.

For example, the linker may include, as reactive groups, a maleimide group at one end and an aldehyde group, a propionaldehyde group, or a butyraldehyde group at the other end.

When polyethylene glycol having hydroxyl reactive groups at both ends is used as a linker, the interleukin-2 analog long-acting conjugate of the present invention may be produced by activating the hydroxyl groups to various reactive groups through known chemical reactions or using commercially available polyethylene glycol having modified reactive groups.

In a specific embodiment, the linker may be linked to a cysteine residue of X, more specifically a -SH group of cysteine, but is not limited thereto.

Specifically, the reactive group of the linker may be linked to the -SH group of the cysteine residue, and all of those described above will be applied to the reactive group. When maleimide-PEG-aldehyde is used, the maleimide group may be linked to the -SH group of X via a thioether bond, and the aldehyde group may be linked to the $-NH_2$ group of F, specifically the immunoglobulin Fc, through reductive amination, but is not limited thereto.

In another specific embodiment, the linker may be linked to a lysine residue of X, more specifically the amino group of the lysine, but is not limited thereto.

In the conjugate, the reactive group of the linker may be linked to $-NH_2$ located at the N-terminus of the immunoglobulin Fc region, but is not limited thereto.

Additionally, in the conjugate, the interleukin-2 analog according to the present invention may be linked to the linker having reactive groups through the C-terminus thereof, but this is merely an example.

The term "C-terminus" as used herein refers to the carboxyl terminus of a peptide, and for the purpose of the present invention, the term refers to a site that can bind to the linker. For example, the C-terminus may include not only the amino acid residue at the extreme end of the C-terminus but also amino acid residues near the C-terminus and, specifically, include the amino acid residues at positions 1 to 20 from the extreme end, but is not limited thereto.

Furthermore, the above-described conjugate may have an increased duration of efficacy compared with X not modified with F, and such a conjugate may be not only in the above-described forms but also in a form of being encapsulated in a biodegradable nanoparticle.

Specifically, the interleukin-2 analog long-acting conjugate of the present invention may have no binding affinity or may have binding affinity for the interleukin-2 alpha receptor of about 0.001-fold or greater, about 0.005-fold or greater, about 0.01-fold or greater, about 0.05-fold or greater, about 0.1-fold or greater, about 0.3-fold or greater, about 0.5-fold or greater, about 0.6-fold or greater, about 0.7-fold or greater, about 0.8-fold or greater, about 0.9-fold or greater, about 1.1-fold or greater, about 1.3-fold or greater, about 1.5-fold or greater, or about 1.7-fold or greater compared with the binding affinity of native interleukin-2, aldesleukin, or a long-acting conjugate including the same for the interleukin-2 alpha receptor, but the numerical value of the binding affinity is not limited, and the numerical value falls within the scope of the present invention as long as the binding affinity thereof is altered compared with that of native interleukin-2 or aldesleukin.

In addition, specifically, the interleukin-2 analog long-acting conjugate of the present invention may have a binding affinity for the interleukin-2 beta receptor of about 0.1-fold or greater, about 0.3-fold or greater, about 0.5-fold or greater, about 0.7-fold or greater, about 1.0-fold or greater, about 10-fold or greater, about 20-fold or greater, about 30-fold or greater, about 40-fold or greater, about 50-fold or greater, about 60-fold or greater, about 70-fold or greater, about 80-fold or greater, about 90-fold or greater, about 100-fold or greater, about 130-fold or greater, about 150-fold or greater, or about 200-fold or greater, compared with the binding affinity of native interleukin-2, aldesleukin, or a long-acting conjugate including the same for the interleukin-2 beta receptor, but the numerical value of the binding affinity is not limited, and the numerical value falls within the scope of the present invention as long as the binding affinity thereof is altered or increased compared with that of native interleukin-2 or aldesleukin.

Unless specified otherwise herein, the description or claims with respect to "interleukin-2 analog" according to the present invention or a "conjugate" in which an interleukin-2 analog is covalently linked to a biocompatible substance, may be applied to the forms, which include not only the corresponding interleukin-2 analog or conjugate but also salts (e.g., pharmaceutically acceptable salts of the interleukin-2 analog) of the corresponding interleukin-2 analog or conjugate, or solvates thereof. Therefore, even though "interleukin-2 analog" or "conjugate" is merely described herein, the corresponding description may also be equally applied to a particular salt thereof, a particular solvate thereof, and a particular solvate of the particular salt thereof. These salts may be in the form in which, for example, any pharmaceutically acceptable salt is used. The type of the salt is not particularly limited. However, the salt is preferably in the form that is safe and effective to a subject, e.g., a mammal, but is not particularly limited thereto.

The type of the salt is not particularly limited. However, the salt is preferably in the form that is safe and effective to a subject, e.g., a mammal, but is not particularly limited thereto.

The term "pharmaceutically acceptable" refers to a substance that can be effectively used for a desired purpose without causing excessive toxicity, irritation, allergic reactions, and the like within the scope of the medical and pharmaceutical decision.

The term "pharmaceutically acceptable salt" as used herein refers to a salt derived from pharmaceutically acceptable inorganic acids, organic acids, or bases. Examples of suitable acids may include hydrochloric acid, bromic

acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, and the like. Salts derived from suitable bases may include alkali metals such as sodium and potassium, alkali earth metals such as magnesium, ammonium, and the like.

The term "solvate" as used herein refers to a complex formed of the interleukin-2 analog according to the present invention or a salt thereof and a solvent molecule.

The composition according to the present invention may contain the interleukin-2 analog or long-acting conjugate thereof and, more specifically, the composition may be a pharmaceutical composition used for cancer prevention or treatment while administered in combination with an immune checkpoint inhibitor or a composition containing the same. The interleukin-2 analog and long-acting conjugate thereof are as described above. More specifically, the composition according to the present invention may contain a pharmacologically effective amount of the interleukin-2 analog or long-acting conjugate thereof and further contain a pharmaceutically acceptable carrier while administered in combination with a pharmacologically effective amount of an immune checkpoint inhibitor or a composition containing the same.

Alternatively, the composition of the present invention may further contain an immune checkpoint inhibitor, but is not limited thereto.

In a specific embodiment, the composition according to the present invention may contain an interleukin-2 analog including an amino acid sequence of any one selected from the group consisting of SEQ ID NOS: 3 to 106, or a long-acting conjugate including the same and, more specifically, the composition according to the present invention may contain an interleukin-2 analog including an amino acid sequence of any one selected from the group consisting of SEQ ID NOS: 22, 42, 53, 87, 105, and 106 or a long-acting conjugate thereof, and may be administered in combination with an immune checkpoint inhibitor or a composition containing the same.

The composition containing an interleukin-2 analog or interleukin-2 analog long-acting conjugate of the present invention may be administered in combination with a composition containing an immune checkpoint inhibitor simultaneously, sequentially, or in reverse order, but is not limited thereto.

The term "pharmaceutically effective amount" as used herein refers to a safe dose at which the interleukin-2 analog or long-acting conjugate thereof and an immune checkpoint inhibitor exhibit cancer prevention or treatment effects without showing toxicity or side effects in patients. Specifically, the term may refer to a dose that can exhibit a significant activity on interleukin-2 receptors (e.g., beta and/or alpha receptors), or a dose that can activate T cells, but is not limited thereto.

The composition according to the present invention may exhibit one or more of the following properties, but is not limited as long as the composition exhibits an increased immune response, an anticancer effect, and the like.

(i) high blood exposure compared with the administration of aldesleukin alone or combined administration of aldesleukin and an immune checkpoint inhibitor;
(ii) excellent tumor growth inhibition compared with the administration of aldesleukin alone or combined administration of aldesleukin and an immune checkpoint inhibitor;
(iii) excellent memory T cell production response compared with the administration of aldesleukin alone or combined administration of aldesleukin and an immune checkpoint inhibitor;
(iv) excellent immune checkpoint protein activation inhibition compared with the administration of aldesleukin alone or combined administration of aldesleukin and an immune checkpoint inhibitor; and
(v) excellent T cell activation compared with the administration of aldesleukin alone or combined administration of aldesleukin and an immune checkpoint inhibitor.

Interleukin-2, known as a T cell growth factor, is a protein involved in immunomodulation and has the activity of proliferating T cells, stimulating B cells, and secreting γ-interferon by acting on T cells. Cancer prevention or treatment effects can be obtained by removing cancer cells using the immune system in the body on the basis of the immunomodulatory activity of interleukin-2.

In particular, the interleukin-2 analog of the present invention has an increased binding affinity for the interleukin-2 beta receptor, which play a major role in signaling, leading to a more effective anticancer effect in the immune system of a subject. In addition, the long-acting conjugate including the interleukin-2 analog has an increased binding affinity for the interleukin-2 beta receptor, an increased duration of efficacy, and a high blood exposure, leading to excellent bioavailability, thus consequently exhibiting excellent tumor growth inhibitory ability, consequently exhibiting effective cancer prevention or treatment effects. Furthermore, the long-acting conjugate has an excellent memory T cell production ability and thus can exhibit a cancer recurrence inhibitory effect through the immune memory response of a subject, leading to a safe and effective cancer medicine.

When an immune checkpoint inhibitor is used together with the interleukin-2 analog according to the present invention, the immune checkpoint inhibitor activates T cells, thereby allowing the T cells to attack cancer cells evading the

immune system through the immune checkpoint, thus achieving an excellent effect resulting from combined administration.

The term "immune checkpoint inhibitor" as used herein refers to a medicine that activates T cells to attack cancer cells by binding to an immune checkpoint protein involved in the suppression of T cells.

The immune checkpoint inhibitor means a medicine that prevents the binding of an immune checkpoint protein (e.g., PD-1) and a cancer cell ligand (e.g., PD-L1), thereby maintaining the original function of immune cells (e.g., T cells).

The immune checkpoint inhibitor may include a peptide, an antibody or an antigen-binding fragment thereof, a nucleic acid molecule, and a small molecule, but is not limited thereto as long as it can have an antagonistic effect.

Examples of the immune checkpoint inhibitor of the present invention may include an anti-PD-1 antibody or an antigen-binding fragment thereof.

Programmed cell death-1 (PD-1), one of the most well-known immune checkpoint proteins, is located outside the immune cells and can specifically bind to programmed death-ligand 1 (PD-L1) protein of various cells in the human body. The binding of PD-L1 and PD-1 inactivates the immune cell function to suppress the immune response, thereby preventing the occurrence of unnecessary autoimmune responses. However, a large amount of the PD-L1 protein, expressed on the surface of cancer cells, binds to PD-1 of the immune cells, thereby inhibiting T cell functions and preventing the immune response in the body. Particularly, the cancer cells can survive by evading the immune response.

The immune checkpoint inhibitor of the present invention performs an antagonistic action on an immune checkpoint protein, exploited by cancer cells exploit for immune evasion, thereby suitably ensuring an immune response, which may be disabled by cancer cells, leading to an anti-cancer action using the immune response in the body.

In the present invention, the anti-programmed cell death-1 (PD-1) antibody may be selected from the group consisting of nivolumab, pembrolizumab, cemiplimab, spartalizumab, camrelizumab, sintilimab, tislelizumab, toripalimab, dostarlimab, INCMGA00012, AMP-224, and AMP-514, but is not limited thereto.

The pharmaceutical composition containing an interleukin 2 analog or long-acting conjugate thereof for preventing or treating cancer of the present invention is administered in combination with an immune checkpoint inhibitor or a composition containing the same and thus can exhibit excellent anticancer efficacy and reduce side effects through a synergistic action of the interleukin 2 analog or long-acting conjugate thereof and the immune checkpoint inhibitor.

Immune checkpoint inhibitors are known to exhibit different levels of reactivity depending on the phenotype of the tumor microenvironment (TME), and these immune checkpoint inhibitors have limitations as cancer medicines since the reactivity of immune checkpoint inhibitors is very low in immune-infiltrating tumors (cold tumors).

The interleukin 2 analog or long-acting conjugate thereof of the present invention can change the tumor microenvironment with low responsiveness to an immune checkpoint inhibitor to induce a transition from an immune-infiltrating tumor to an infiltrating tumor (hot tumor). Therefore, the administration of an immune checkpoint inhibitor in combination with an interleukin-2 analog or long-acting conjugate thereof can obtain an excellent treatment effect.

Programmed cell death-1 (PD-1), one of the immune checkpoint proteins, is located outside the immune cells and can specifically bind to programmed death-ligand 1 (PD-L1) protein of various cells in the human body.

The pharmaceutical composition containing an interleukin-2 analog or long-acting conjugate thereof for preventing or treating cancer of the present invention can more effectively treat cancer with low responsiveness to a PD-1 antagonist, but is not limited thereto.

In the present invention, examples of the cancer may include renal cell cancer, melanoma, colorectal cancer, liver cancer, uterine cancer, ovarian cancer, pancreatic cancer, gallbladder cancer, lung cancer, small cell lung cancer, non-small cell lung cancer, skin cancer, breast cancer, bladder cancer, stomach cancer, head or neck cancer, esophageal cancer, laryngeal cancer, bone cancer, rectal cancer, perianal cancer, colon cancer, fallopian tube carcinoma, endometrial carcinoma, cervical carcinoma, vaginal carcinoma, vulvar carcinoma, Hodgkin's disease, small bowel cancer, endocrine gland cancer, thyroid cancer, parathyroid cancer, adrenal cancer, soft tissue sarcoma, urethral cancer, penile cancer, prostate cancer, chronic or acute leukemia, lymphocyte lymphoma, renal pelvis carcinoma, CNS tumor, primary CNS lymphoma, spinal tumor, brain tumor, glioma (astrocytoma, glioblastoma, oligodendroglioma, ependymoma), blastocytoma, meningioma, brainstem glioma, pituitary adenoma, neuroblastoma, congenital tumor, craniopharyngioma, and brain tumor, but are not limited thereto.

Specifically, the cancer may be any one selected from the group consisting of large bowel cancer, liver cancer, ovarian cancer, pancreatic cancer, gallbladder cancer, kidney cancer, lung cancer, skin cancer, melanoma, breast cancer, bladder cancer, and gastric cancer, but is not limited thereto. Alternatively, the cancer according to the present invention may be primary cancer, recurrent cancer, or metastatic cancer, but is not limited thereto. Furthermore, the cancer may include metastatic renal cell carcinoma or metastatic melanoma. More specifically, the cancer may be a carcinoma with low responsiveness to a PD-1 antagonist.

Although not particularly limited, the pharmaceutical composition of the present invention may contain an interleukin-2 analog or long-acting conjugate thereof in 0.01 to 99% w/v.

Specifically, the composition of the present invention may contain an interleukin-2 analog or long-acting conjugate thereof, and an immune checkpoint inhibitor, each of which is administered at an amount of 0.00001 mg/mL

to 100 mg/mL, but is not limited thereto.

More specifically, in the composition of the present invention, the long-acting conjugate of the interleukin-2 analog may be contained at 0.0001 to 2120 mg/kg, and an immune checkpoint inhibitor may be administered at 0.0001 to 1000 mg/kg in combination with the long-acting conjugate of the interleukin-2 analog, but is not limited thereto. Alternatively, in the composition of the present invention, an interleukin-2 analog may be contained at 0.00005 to 500 mg/kg, and an immune checkpoint inhibitor may be administered at 0.0001 to 1000 mg/kg in combination with the interleukin-2 analog, but is not limited thereto.

However, the dose of the immune checkpoint inhibitor may be changed so as to exhibit an excellent effect of combined administration of the interleukin-2 analog or long-acting conjugate thereof of the present invention.

For example, the amount of the immune checkpoint inhibitor contained in the composition of the present invention may be expressed on the basis of a PD-1 inhibitor, and when containing another immune checkpoint inhibitor, the composition may contain another immune checkpoint inhibitor at an amount corresponding to the dose of the PD-1 inhibitor, but is not limited thereto.

In addition, the interleukin 2 analog or long-acting conjugate thereof and the immune checkpoint inhibitor, which are contained in the composition of the present invention, may be administered in appropriate divided doses, and the numbers of administration may be the same as or different from each other. For example, when the interleukin-2 analog or long-acting conjugate thereof of the composition is administered once a week, the immune checkpoint inhibitor may be administered once, twice, or more times a week, but is not limited thereto.

The term "prevention" as used herein refers to any action that inhibits or delays cancer or a tumor by the administration of: the interleukin-2 analog (e.g., the interleukin-2 analog itself or a long-acting conjugate form in which a biocompatible substance is bound to the interleukin-2 analog) or a composition containing the same; and an immune checkpoint inhibitor or a composition containing the same.

The term "treatment" as used herein refers to any action that alleviates or advantageously change the symptoms of cancer by the administration of: the interleukin-2 analog (e.g., the interleukin-2 analog itself or a long-acting conjugate form in which a biocompatible substance is bound to the interleukin-2) or a composition containing the same; and an immune checkpoint inhibitor or a composition containing the same.

The use of the interleukin-2 analog or long-acting conjugate thereof of the present invention and an immune checkpoint inhibitor can significantly increase the blood exposure, blood half-life, and in vivo duration of efficacy, thereby decreasing the number of times of administration, leading to an improvement in patient's quality of life.

The pharmaceutical composition of the present invention may further contain a pharmaceutically acceptable carrier or a diluent. The pharmaceutically acceptable carrier or diluent may be non-naturally occurring.

The term "pharmaceutically acceptable" as used herein means the amount enough to exhibit a treatment effect, without causing side effects, and such an amount can be easily determined by a person skilled in the art depending the factors well-known in a medical field, such as the type of disease, patient's age, body weight, health, and sex, patient's sensitivity to drugs, route of administration, method of administration, number of times of administration, duration of treatment, drugs to be used in combination or concurrently.

The pharmaceutical composition of the present invention containing an interleukin-2 analog or long-acting conjugate thereof or an immune checkpoint inhibitor may contain a pharmaceutically acceptable excipient. With respect to the excipient, although not particularly limited, a binder, a lubricant, a disintegrant, a solubilizer, a dispersant, a stabilizer, a suspending agent, a coloring agent, a flavoring agent, and the like may be used for oral administration, a buffer, a preservative, an analgesic, a solubilizer, an isotonic agent, a stabilizer, and the like may be mixed for an injection; and a base, an excipient, a lubricant, a preservative, and the like may be used for topical administration.

The formulation of the composition of the present invention may be prepared in various manners by mixing with the above-described pharmaceutically acceptable excipient. For example, for oral administration, the composition of the present invention may be formulated in the form of a tablet, a troche, a capsule, an elixir, a suspension, a syrup, a wafer, or the like, and for an injection, the composition may be formulated as a single-dose ampoule or a multi-dose form. The composition may be formulated into a solution, a suspension, a tablet, a pill, a capsule, a sustained-release preparation, or the like.

Meanwhile, examples of the carrier, excipient, and diluent suitable for formulation may include lactose, dextrose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oils. In addition, the composition may further contain a filler, an anticoagulant, a lubricant, a humectant, a flavoring agent, a preservative, and the like.

In addition, the pharmaceutical composition of the present invention may have any one formulation selected from the group consisting of a tablet, a pill, a powder, granules, a capsule, a suspension, a liquid medicine for internal use, an emulsion, a syrup, a sterile aqueous solution, a non-aqueous solvent, a lyophilized preparation, and a suppository.

In addition, the composition may be formulated into a single dose form suitable for the patient's body, and specifically, may be formulated into a preparation useful for the administration of a protein drug, and may be administered

by an administration method commonly used in the art through an oral, or parenteral route including a skin, intravenous, intramuscular, intra-arterial, intramedullary, intrathecal, intraventricular, pulmonary, transdermal, subcutaneous, intraperitoneal, intranasal, intragastric, topical, sublingual, vaginal, or rectal route, but is not limited thereto.

In addition, the conjugate may be used by mixing with various carriers approved as medicines, such as physiological saline or organic solvents, and for increasing stability or absorptivity, carbohydrates such as glucose, sucrose, or dextran, antioxidants such as ascorbic acid or glutathione, chelating agents, low-molecular weight proteins, or other stabilizers may be used as medicines.

In accordance with another aspect of the present invention, there is provided a method for preventing or treating cancer, the method including administering to a subject in need thereof a pharmaceutical composition, containing an interleukin-2 analog or long-acting conjugate containing the same, in combination with an immune checkpoint inhibitor or a composition containing the same. In the method, the pharmaceutical composition, containing an interleukin-2 analog or long-acting conjugate containing the same, and the immune checkpoint inhibitor or the composition containing the same may be administered simultaneously, sequentially, or in reverse order, but is not limited thereto.

The interleukin-2 analog and/or long-acting conjugate of the interleukin-2 analog, the immune checkpoint inhibitor, the composition containing the same, cancer, prevention, and treatment are as described above.

In accordance with another aspect of the present invention, there is provided a method for preventing or treating cancer, the method including administering to a subject an interleukin-2 analog or long-acting conjugate including the same in combination with an immune checkpoint inhibitor.

The method may include administering to a subject a composition containing an interleukin-2 analog or long-acting conjugate including the same and an immune checkpoint inhibitor or administering an interleukin-2 analog or long-acting conjugate including the same and an immune checkpoint inhibitor as individual preparations, but is not limited thereto.

In the method of the present invention, (i) an interleukin-2 analog or long-acting conjugate including the same and (ii) an immune checkpoint inhibitor may be administered as one preparation or may be administered as individual preparations simultaneously, sequentially, or in reverse order, but the present invention is not limited thereto.

The interleukin-2 analog or long-acting conjugate including the same, the immune checkpoint inhibitor, the composition, cancer, prevention, and treatment are as described above.

In the present invention, the subject refers to a subject having cancer or suspected of having cancer, meaning a mammal including humans, rats, livestock, and the like, but any subject that can be treated with the interleukin-2 analog and/or conjugate, the immune checkpoint inhibitor, or the composition containing the same of the present invention may be included without limitation.

The term "administration" as used herein refers to an introduction of a predetermined substance (e.g., an interleukin-2 analog, a long-acting conjugate thereof, or an immune checkpoint inhibitor) into a patient by any appropriate method. The route of administration of the substance may include, but is not particularly limited to, any typical route through which the substance can arrive at a target in vivo, and for example, the route of administration may be intraperitoneal administration, intravenous administration, intramuscular administration, subcutaneous administration, intradermal administration, oral administration, topical administration, intranasal administration, intrapulmonary administration, intrarectal administration, or the like.

The method of the present invention may include administering a pharmaceutically effective amount of the pharmaceutical composition containing an interleukin-2 analog or long-acting conjugate thereof and administering a pharmaceutically effective amount of an immune checkpoint inhibitor or a pharmaceutical composition containing the same. The appropriate total daily dose may be determined by a practitioner within appropriate medical judgment, and may be administered once or several times in divided doses. For the purpose of the present invention, the specific therapeutically effective dose for a specific patient may be preferably applied differently depending on various factors well known in the medical art, including the type and degree of the response to be achieved, a specific composition including whether other agents are occasionally used therewith or not, the patient's age, body weight, general health condition, sex and a diet, the time of administration, the route of administration, the secretion rate of the composition, the duration of treatment, other drugs used in combination or concurrently with the composition of the present invention, and like factors well-known in the medical art.

In the method of the present invention, the dose and the number of times of administration during administration are determined according to the type of drug, which is an active ingredient, along with several related factors, such as the disease to be treated, the route of administration, patient's age, gender and weight, and disease severity. Specifically, the composition of the present invention may contain the interleukin-2 analog or the long-acting conjugate including the same and the immune checkpoint inhibitor at pharmaceutically effective amounts, but is not limited thereto.

The containing of the interleukin-2 analog or long-acting conjugate thereof and the immune checkpoint inhibitor at pharmaceutically effective amounts means the degrees at which desired pharmaceutical activity (e.g., prevention, alleviation, or treatment of cancer) can be obtained by the interleukin-2 analog or long-acting conjugate thereof and the immune checkpoint inhibitor, and may also mean the levels at which toxicities or side effects are absent or slightly present,

up to a pharmaceutically acceptable level, in a subject to be administered, but the level is not limited thereto. Such a pharmaceutically effective amount may be determined by comprehensively considering the number of times of administration, patient, formulation, and the like.

Although not particularly limited, the pharmaceutical composition of the present invention may contain the ingredient (active ingredient) in an amount of 0.01 to 99% (w/v).

The total effective amount of the composition of the present invention may be administered to a patient in a single dose, or may be administered in multiple doses by a fractionated treatment protocol for a long period of time. The pharmaceutical composition of the present invention may contain an active ingredient, of which the content may vary depending on the severity of a disease. Specifically, the preferred total daily dose of the interleukin-2 analog or long-acting conjugate thereof and the immune checkpoint inhibitor of the present invention, which are administered in combination, may be about 0.0001 mg to 500 mg for 1 kg of body weight of a patient. However, with respect to the doses of the interleukin-2 analog or conjugate thereof and the immune checkpoint inhibitor, the effective doses thereof for a patient are determined considering various factors including patient's age, body weight, health condition, and sex, the severity of a disease, a diet, and a rate of excretion, in addition to the route of administration of the pharmaceutical composition and the number of times of treatment using the same, and thus, considering these, a person skilled in the art can determine an appropriate effective dose according to the particular use of the composition of the present invention. The pharmaceutical composition according to the present invention is not particularly limited to the formulation, route of administration, and method of administration, as long as the pharmaceutical composition exhibits the effects of the present invention.

The pharmaceutical composition of the present invention may have excellent in vivo duration and titer, thereby significantly reducing the number and frequency of administration of the pharmaceutical preparation of the present invention. The pharmaceutical composition may be administered via intraperitoneal, intravenous, intramuscular, subcutaneous, intradermal, oral, topical, intranasal, intrapulmonary, or intrarectal route, but the administration is not limited to a specific route of administration as long as the desired pharmacological effect can be obtained.

As an example, the pharmaceutical composition of the present invention may be administered once a week, once every two weeks, once every three weeks, once every four weeks, or once a month, or may be administered once or multiple times at time intervals ranging from one week to one month, but the frequency is not limited thereto.

Specifically, the interleukin-2 analog or long-acting conjugate thereof and the immune checkpoint inhibitor may be administered at an amount of about 0.00001 mg or more or 0.0000001 mg or more for 1 kg of the patient's body weight per week, respectively, and when the two substances are used in combination, the total amount is about 0.00001 mg or more for 1 kg of the patient's body weight per week, but is not limited thereto.

Specifically, in the method of the present invention, 0.00001 to 175 mg of the interleukin 2 analog long-acting conjugate and 0.0000001 to 100 mg of the immune checkpoint inhibitor may be administered for 1 kg of patient's body weight per week, but is not limited thereto. Alternatively, in the method of the present invention, 0.000001 to 50 mg of the interleukin-2 analog and 0.0000001 to 100 mg of the immune checkpoint inhibitor may be administered for 1 kg of patient's body weight per week, but is not limited thereto.

Alternatively, in the method of the present invention, the interleukin 2 analog or long-acting conjugate thereof may be administered at 0.001 to 33000 nmol/kg, and the immune checkpoint inhibitor may be administered at 0.0001 to 7000 nmol/kg, but is not limited thereto.

Furthermore, the interleukin-2 analog or long-acting conjugate thereof and the immune checkpoint inhibitor of the present invention may be administered in appropriate divided doses, and the numbers of administration may be the same as or different from each other. For example, when the interleukin-2 analog or long-acting conjugate thereof is administered once a week, the immune checkpoint inhibitor may be administered once, twice, or more times a week, but is not limited thereto.

However, the dose of the immune checkpoint inhibitor may be changed so as to exhibit an excellent effect of combined administration of the interleukin-2 analog or long-acting conjugate thereof of the present invention.

For example, the dose of the immune checkpoint inhibitor may be expressed on the basis of a PD-1 inhibitor, and the composition may contain another immune checkpoint inhibitor at an amount that corresponds to the dose of the PD-1 inhibitor, but is not limited thereto.

In another aspect of the present invention, there is provided use of the interleukin-2 analog or long-acting conjugate thereof or a composition containing the same, which is administered in combination with an immune checkpoint inhibitor, in the preparation of a medicine for prevention or treatment of cancer.

In still another aspect of the present invention, there is provided the combined use of an interleukin-2 analog or long-acting conjugate thereof and an immune checkpoint inhibitor for the prevention or treatment of cancer.

The interleukin-2 analog and/or long-acting conjugate thereof, immune checkpoint inhibitor, composition containing the same, cancer, prevention, treatment, route of administration, and number of times of administration are as described above.

In another aspect of the present invention, there is provided the use of an interleukin-2 analog or long-acting conjugate thereof or a composition containing the same, which is administered in combination with an immune checkpoint

inhibitor, for preventing or treating cancer.

The interleukin-2 analog and/or long-acting conjugate thereof, immune checkpoint inhibitor, composition containing the same, cancer, prevention, treatment, route of administration, and number of times of administration are as described above.

Unless the context clearly requires otherwise, the expressions of "comprise", "include", "contain", and the like should be understood to mean that they include the stated integer(s) or group of integers but are not excluding other integers or sets of integers.

Hereinafter, the present invention will be described in more detail with reference to exemplary embodiments. These exemplary embodiments are given for specifically illustrating the present invention, and the scope of the present invention is not limited to these exemplary embodiments.

## Example 1: Construction of expression vectors for native interleukin-2 and interleukin-2 analogs

For the preparation of expression vectors for native interleukin-2, encoding 133 amino acids, an interleukin-2 that was synthesized based on the reported interleukin-2 sequence (NM_000586.3; SEQ ID NO: 1) was cloned into the pET-22b vector (Novagen). In addition, novel interleukin-2 analogs were prepared in which amino acids of the interleukin-2 used as a template were modified.

PCR conditions for the amplification of the interleukin-2 analogs were 16 cycles of a process consisting of 95°C for 30 seconds, 55°C for 60 seconds, and 65°C for 6.5 minutes. The mutagenesis products obtained under the conditions above were sequenced, and it was verified that the modifications shown in Table 1 below were observed at the desired modification positions for each interleukin-2 analog, based on the native form. The expression vectors thus obtained were named pET22b-interleukin-2 analog 1 to 105.

The altered sequence of amino acids and analog name for each are shown in Table 1 below. To prepare these interleukin-2 analogs, PCR was performed using forward (F) and reverse (R) primers to amplify each analog gene.

In Table 1 below, analog 1 is aldesleukin, and primer #1 to #204 correspond to SEQ ID NOS: 214 to 417, respectively.

TABLE 1

| Types, modification positions, and altered sequences of interleukin-2 analogs | | |
|---|---|---|
| **Analog** | **Modification position and altered sequence** | **Primer#** |
| 1 | desA1, C125S | 197, 198, 201, 202 |
| 2 | desA1, C125S, S87C | 197, 198, 201, 202, 149, 150 |
| 3 | desA1, C125S, K32C | 197, 198, 201, 202, 19, 20 |
| 4 | desA1, C125S, K35C | 197, 198, 201, 202, 21, 22 |
| 5 | desA1, C125S, K43C | 197, 198, 201, 202, 47, 48 |
| 6 | desA1, C125S, K48C | 197, 198, 201, 202, 53, 54 |
| 7 | desA1, C125S, K49C | 197, 198, 201, 202, 55, 56 |
| 8 | desA1, C125S, K76C | 197, 198, 201, 202, 73, 74 |
| 9 | desA1, C125S, R38A | 197, 198, 201, 202, 25, 26 |
| 10 | desA1, C125S, F42K | 197, 198, 201, 202, 35, 36 |
| 11 | desA1, C125S, F42A | 197, 198, 201, 202, 33, 34 |
| 12 | desA1, C125S, R38A, F42K | 197, 198, 201, 202, 25, 26, 35, 36 |
| 13 | desA1, C125S, R38A, F42A | 197, 198, 201, 202, 25, 26, 33, 34 |
| 14 | desA1, C125S, L19Y, R38A, F42K | 197, 198, 201, 202, 13, 14, 25, 26, 35, 36 |
| 15 | desA1, C125S, R38A, F42K, D84E | 197, 198, 201, 202, 25, 26, 35, 36, 109, 110 |
| 16 | desA1, C125S, R38A, F42K, N88Q | 197, 198, 201, 202, 25, 26, 35, 36, 153, 154 |
| 17 | desA1, C125S, R38A, F42K, V91T | 197, 198, 201, 202, 25, 26, 35, 36, 165, 166 |
| 18 | desA1, C125S, R38A, F42K, E61Q | 197, 198, 201, 202, 25, 26, 35, 36, 59, 60 |
| 19 | desA1, C125S, R38A, F42K, R81D, I92F | 197, 198, 201, 202, 25, 26, 35, 36, 97, 98, 167, 168 |

(continued)

| Types, modification positions, and altered sequences of interleukin-2 analogs | | |
|---|---|---|
| **Analog** | **Modification position and altered sequence** | **Primer#** |
| 20 | desA1, C125S, R38A, F42K, L85V, I86V, I92F | 197, 198, 201, 202, 25, 26, 35, 36, 139, 140, 167, 168 |
| 21 | desA1, C125S, R38A, F42K, L80F, R81D, L85V, I86V, I92F | 197, 198, 201, 202, 25, 26, 35, 36, 79, 80, 167, 168 |
| 22 | desA1, C125S, L12V, R38A, F42K | 197, 198, 201, 202, 3, 4, 25, 26, 35, 36 |
| 23 | desA1, C125S, L12F, R38A, F42K | 197, 198, 201, 202, 1, 2, 25, 26, 35, 36 |
| 24 | desA1, C125S, L19V, R38A, F42K | 197, 198, 201, 202, 11, 12, 25, 26, 35, 36 |
| 25 | desA1, C125S, L19F, R38A, F42K | 197, 198, 201, 202, 9, 10, 25, 26, 35, 36 |
| 26 | DesA1, C125S, R38A, F42K, I89F | 197, 198, 201, 202, 25, 26, 35, 36, 157, 158 |
| 27 | desA1, C125S, R38A, F42K, V91F | 197, 198, 201, 202, 25, 26, 35, 36, 163, 164 |
| 28 | desA1, C125S, R38A, F42K, L94V | 197, 198, 201, 202, 25, 26, 35, 36 |
| 29 | desA1, C125S, R38A, F42K, Q126T | 197, 198, 201, 202, 25, 26, 35, 36, 199, 200 |
| 30 | desA1, C125S, R38A, R81D, I92F | 197, 198, 201, 202, 27, 28, 97, 98, 169, 170 |
| 31 | desA1, C125S, R38A, D84E | 197, 198, 201, 202, 27, 28, 109, 110 |
| 32 | desA1, C125S, R38A, R81D, D84E, I92F | 197, 198, 201, 202, 27, 28, 95, 96, 169, 170 |
| 33 | desA1, C125S, R38A, L80F | 197, 198, 201, 202, 27, 28, 77, 78 |
| 34 | desA1, C125S, R38A, L80F, D84E | 197, 198, 201, 202, 27, 28, 77, 78, 109, 110 |
| 35 | desA1, C125S, R38A, L94F, L96F | 197, 198, 201, 202, 27, 28, 189, 190 |
| 36 | desA1, C125S, R38A, L94F, L96V | 197, 198, 201, 202, 27, 28, 193, 194 |
| 37 | desA1, C125S, R38A, L94F, L96I | 197, 198, 201, 202, 27, 28, 191, 192 |
| 38 | desA1, C125S, R38A, F42K, R81D, I92F, L94F, L96F | 197, 198, 201, 202, 25, 26, 35, 36, 97, 98, 175, 176 |
| 39 | desA1, C125S, R38A, F42K, R81D, I92F, L94F, L96V | 197, 198, 201, 202, 25, 26, 35, 36, 97, 98, 179, 180 |
| 40 | desA1, C125S, R38A, F42K, R81D, I92F, L94F, L96I | 197, 198, 201, 202, 25, 26, 35, 36, 97, 98, 177, 178 |
| 41 | desA1, C125S, L80F, R81D, L85V, I86V, I92F | 197, 198, 201, 202, 25, 26, 29, 30, 35, 36, 41, 42, 79, 80, 167, 168 |
| 42 | desA1, C125S, R38A, F42K, R81E, I92F | 197, 198, 201, 202, 25, 26, 35, 36, 103, 104, 169, 170 |
| 43 | desA1, C125S, R38A, F42K, R81D, I92L | 197, 198, 201, 202, 25, 26, 35, 36, 99, 100, 183, 184 |
| 44 | desA1, C125S, R38A, F42K, R81D, D84V, I92F | 197, 198, 201, 202, 25, 26, 35, 36, 99, 100, 113, 114, 169, 170 |
| 45 | desA1, C125S, R38A, F42K, R81D, D84F, I92F | 197, 198, 201, 202, 25, 26, 35, 36, 99, 100, 111, 112, 169, 170 |
| 46 | desA1, C125S, D20V, R38A, F42K, R81D, I92F | 197, 198, 201, 202, 17, 18, 25, 26, 35, 36, 99, 100, 169, 170 |
| 47 | desA1, C125S, D20F, R38A, F42K, R81D, I92F | 197, 198, 201, 202, 15, 16, 25, 26, 35, 36, 99, 100, 169, 170 |
| 48 | desA1, C125S, R38A, F42K, R81D, N88V, I92F | 197, 198, 201, 202, 25, 26, 35, 36, 99, 100, 155, 156, 169, 170 |

(continued)

| Types, modification positions, and altered sequences of interleukin-2 analogs | | |
|---|---|---|
| **Analog** | **Modification position and altered sequence** | **Primer#** |
| 49 | desA1, C125S, R38A, F42K, R81D, N88F, I92F | 197, 198, 201, 202, 25, 26, 35, 36, 99, 100, 151, 152, 169, 170 |
| 50 | desA1, C125S, F42K, L80F, R81D, L85V, I86V, I92F | 197, 198, 201, 202, 25, 26, 29, 30, 35, 36, 79, 80, 139, 140, 169, 170 |
| 51 | desA1, C125S, E61Q, R81D, I92F | 197, 198, 201, 202, 59, 60, 99, 100, 169, 170 |
| 52 | desA1, C125S, R38A, F42K, L80F, R81D, I92F | 197, 198, 201, 202, 25, 26, 35, 36, 79, 80, 169, 170 |
| 53 | desA1, C125S, R38A, F42K, L80F, R81D, D84E, I92F | 197, 198, 201, 202, 25, 26, 35, 36, 79, 80, 95, 96, 169, 170 |
| 54 | desA1, C125S, R38A, F42K, Q74H, R81D, I92F | 197, 198, 201, 202, 25, 26, 35, 36, 71, 72, 99, 100, 169, 170 |
| 55 | desA1, C125S, R38A, F42K, Q74H, L80F, R81D, I92F | 197, 198, 201, 202, 25, 26, 35, 36, 71, 72, 79, 80, 169, 170 |
| 56 | desA1, C125S, R38A, F42K, Y45A, Q74H, R81D, I92F | 197, 198, 201, 202, 25, 26, 35, 36, 49, 50, 71, 72, 99, 100, 169, 170 |
| 57 | desA1, C125S, R38A, F42K, Y45A, Q74H, L80F, R81D, I92F | 197, 198, 201, 202, 25, 26, 35, 36, 49, 50, 71, 72, 79, 80, 169, 170 |
| 58 | desA1, C125S, R38A, F42K, L80F, R81D, L85A, I86A, I92F | 197, 198, 201, 202, 25, 26, 35, 36, 79, 80, 115, 116, 167, 168 |
| 59 | desA1, C125S, R38A, F42K, L80F, R81D, L85A, I86A, I92Y | 197, 198, 201, 202, 25, 26, 35, 36, 79, 80, 115, 116, 187, 188 |
| 60 | desA1, C125S, R38A, F42K, Y45A, L80Y, L85A, I86A, I92Y | 197, 198, 201, 202, 27, 28, 35, 36, 41, 42, 51, 52, 93, 94, 115, 116, 187, 188 |
| 61 | desA1, C125S, R38A, F42K, L80Y, R81D, L85G, I86V, I92Y | 197, 198, 201, 202, 25, 26, 35, 36, 87, 88, 125, 126, 187, 188 |
| 62 | desA1, C125S, R38A, F42K, L80W, R81E, L85G, I86A, I92F | 197, 198, 201, 202, 27, 28, 35, 36, 41, 42, 85, 86, 123, 124, 171, 172 |
| 63 | desA1, C125S, R38A, F42K, L80D, R81E, L85T, I86G, I92F | 197, 198, 201, 202, 25, 26, 35, 36, 75, 76, 133, 134, 167, 168 |
| 64 | desA1, C125S, R38A, F42K, L80Y, R81N, L85V, I86V, I92Y | 197, 198, 201, 202, 25, 26, 35, 36, 91, 92, 139, 140, 167, 168 |
| 65 | desA1, C125S, R38A, F42K, L80Y, R81E, L85V, I86V, I92F | 197, 198, 201, 202, 25, 26, 35, 36, 89, 90, 139, 140, 167, 168 |
| 66 | desA1, C125S, R38A, F42K, L80F, R81E, L85F, I86V, I92F | 197, 198, 201, 202, 25, 26, 35, 36, 81, 82, 121, 122, 167, 168 |
| 67 | desA1, C125S, R38A, F42K, L80Y, R81D, L85F, I86V, I92W, E95D | 197, 198, 201, 202, 25, 26, 35, 36, 87, 88, 121, 122, 185, 186, 195, 196 |
| 68 | desA1, C125S, R38A, F42K, L80F, R81E, L85I, I86V, V91E, I92F | 197, 198, 201, 202, 25, 26, 35, 36, 81, 82, 127, 128, 159, 160, 167, 168 |
| 69 | desA1, C125S, R38A, F42K, L80Y, R81E, L85F, I86L, V91E, I92W, E95D | 197, 198, 201, 202, 25, 26, 35, 36, 89, 90, 119, 120, 161, 162, 185, 186, 195, 196 |
| 70 | desA1, C125S, R38A, F42K, L80Y, R81D, L85V, I86V, I92F | 197, 198, 201, 202, 25, 26, 35, 36, 87, 88, 139, 140, 167, 168 |
| 71 | desA1, C125S, R38A, F42K, L80F, R81E, L85V, I86V, I92F | 197, 198, 201, 202, 25, 26, 35, 36, 81, 82, 139, 140, 167, 168 |

(continued)

| Types, modification positions, and altered sequences of interleukin-2 analogs | | |
|---|---|---|
| **Analog** | **Modification position and altered sequence** | **Primer#** |
| 72 | desA1, C125S, R38A, F42K, L80F, R81D, L85V, I86G, I92F | 197, 198, 201, 202, 25, 26, 35, 36, 79, 80, 135, 136, 167, 168 |
| 73 | desA1, C125S, R38A, F42K, L80F, R81D, L85W, I86V, I92F | 197, 198, 201, 202, 25, 26, 35, 36, 79, 80, 141, 142, 167, 168 |
| 74 | desA1, C125S, R38D, F42K, L80Y, R81D, L85V, I86V, I92F | 197, 198, 201, 202, 31, 32, 35, 36, 87, 88, 139, 140, 167, 168 |
| 75 | desA1, C125S, R38A, F42K, Y45A, L80F, R81E, L85V, I86V, I92F | 197, 198, 201, 202, 25, 26, 35, 36, 51, 52, 81, 82, 139, 140, 167, 168 |
| 76 | desA1, C125S, R38A, F42K, K43Q, E61R, L80F, R81D, L85V, I86G, I92F | 197, 198, 201, 202, 25, 26, 39, 40, 61, 62, 79, 80, 135, 136, 167, 168 |
| 77 | desA1, C125S, R38A, F42K, K43E, E61R, L80F, R81D, L85W, I86V, I92F | 197, 198, 201, 202, 25, 26, 37, 38, 61, 62, 79, 80, 143, 144, 167, 168 |
| 78 | desA1, C125S, K35E, R38A, F42K, L80F, R81E, L85V, I86V, I92F | 197, 198, 201, 202, 23, 24, 25, 26, 35, 36, 81, 82, 139, 140, 167, 168 |
| 79 | desA1, C125S, K35E, R38A, F42K, Q74H, L80F, R81E, L85V, I86V, I92F | 197, 198, 201, 202, 23, 24, 25, 26, 35, 36, 71, 72, 81, 82, 139, 140, 167, 168 |
| 80 | desA1, C125S, K35E, R38A, F42K, Q74H, L80F, R81E, P82G, L85V, I86V, I92F | 197, 198, 201, 202, 23, 24, 25, 26, 35, 36, 71, 72, 81, 82, 105, 106, 139, 140, 167, 168 |
| 81 | desA1, C125S, K35E, R38A, F42K, Q74H, L80F, R81E, P82V, L85V, I86V, I92F | 197, 198, 201, 202, 23, 24, 25, 26, 35, 36, 71, 72, 81, 82, 107, 108, 139, 140, 167, 168 |
| 82 | desA1, C125S, L18R, Q22E, L80F, R81D, L85E, I86V, I92F | 197, 198, 201, 202, 25, 26, 29, 30, 35, 36, 43, 44, 79, 80, 117, 118, 167, 168 |
| 83 | desA1, C125S, L18R, L19R, Q22E, L80F, R81D, L85E, I86V, I92F | 197, 198, 201, 202, 5, 6, 25, 26, 29, 30, 35, 36, 43, 44, 79, 80, 117, 118, 167, 168 |
| 84 | desA1, C125S, L18R, Q22E, L80V, R81D, L85E, I86V, I92F | 197, 198, 201, 202, 7, 8, 25, 26, 29, 30, 35, 36, 43, 44, 83, 84, 117, 118, 167, 168 |
| 85 | desA1, C125S, L80F, R81E, L85V, I86V, I92F | 197, 198, 201, 202, 25, 26, 29, 30, 35, 36, 43, 44, 81, 82, 139, 140, 167, 168 |
| 86 | desA1, C125S, L18R, Q22E, L80F, R81E, L85V, I86V, I92F | 197, 198, 201, 202, 7, 8, 25, 26, 29, 30, 35, 36, 43, 44, 81, 82, 139, 140, 167, 168 |
| 87 | desA1, C125S, L18R, L19R, Q22E, L80F, R81E, L85V, I86V, I92F | 197, 198, 201, 202, 5, 6, 25, 26, 29, 30, 35, 36, 43, 44, 81, 82, 139, 140, 167, 168 |
| 88 | desA1, C125S, E61D, L80F, R81E, L85V, I86V, I92F | 197, 198, 201, 202, 25, 26, 29, 30, 35, 36, 43, 44, 57, 58, 81, 82, 139, 140, 167, 168 |
| 89 | desA1, C125S, R38A, E68Q, L80F, R81E, L85V, I86V, I92F | 197, 198, 201, 202, 27, 28, 35, 36, 43, 44, 65, 66, 81, 82, 139, 140, 167, 168 |
| 90 | desA1, C125S, F42W, L80F, R81E, L85V, I86V, I92F | 197, 198, 201, 202, 25, 26, 29, 30, 45, 46, 81, 82, 139, 140, 167, 168 |
| 91 | desA1, C125S, E61Q, L80F, R81E, L85V, I86V, I92F | 197, 198, 201, 202, 25, 26, 29, 30, 35, 36, 43, 44, 59, 60, 81, 82, 139, 140, 167, 168 |
| 92 | desA1, C125S, L80F, R81E, L85V, I86V, V91T, I92F | 197, 198, 201, 202, 25, 26, 29, 30, 35, 36, 43, 44, 81, 82, 139, 140, 147, 148, 167, 168 |
| 93 | desA1, C125S, L80F, R81E, D84E, L85V, I86V, V91T, I92F | 197, 198, 201, 202, 25, 26, 29, 30, 35, 36, 43, 44, 81, 82, 101, 102, 139, 140, 167, 168 |

(continued)

| Types, modification positions, and altered sequences of interleukin-2 analogs | | |
|---|---|---|
| **Analog** | **Modification position and altered sequence** | **Primer#** |
| 94 | desA1, C125S, L80F, R81E, L85V, I86V, I92F, L94F, L96F | 197, 198, 201, 202, 25, 26, 29, 30, 35, 36, 43, 44, 81, 82, 139, 140, 173, 174 |
| 95 | desA1, C125S, V69G, L80F, R81E, L85V, I86V, I92F | 197, 198, 201, 202, 25, 26, 29, 30, 35, 36, 43, 44, 67, 68, 81, 82, 139, 140, 167, 168 |
| 96 | desA1, C125S, V69G, Q74A, L80F, R81E, L85V, I86V, I92F | 197, 198, 201, 202, 25, 26, 29, 30, 35, 36, 43, 44, 69, 70, 81, 82, 139, 140, 167, 168 |
| 97 | desA1, C125S, R38A, L80F, R81D, I92F | 197, 198, 201, 202, 27, 28, 35, 36, 41, 42, 79, 80, 167, 168 |
| 98 | desA1, C125S, R38A, L80F, R81E, L85V, I92F | 197, 198, 201, 202, 27, 28, 35, 36, 41, 42, 81, 82, 137, 138, 167, 168 |
| 99 | desA1, C125S, R38A, L80F, R81E, I86V, I92F | 197, 198, 201, 202, 27, 28, 35, 36, 41, 42, 81, 82, 131, 132, 167, 168 |
| 100 | desA1, C125S, L80F, R81E, L85Y, I86V, I92F | 197, 198, 201, 202, 25, 26, 29, 30, 35, 36, 41, 42, 81, 82, 145, 146, 167, 168 |
| 101 | desA1, C125S, L80F, R81E, I86A, I92F | 197, 198, 201, 202, 25, 26, 29, 30, 35, 36, 41, 42, 81, 82, 129, 130, 167, 168 |
| 102 | desA1, C125S, L80F, R81E, L85V, I86V | 197, 198, 201, 202, 25, 26, 29, 30, 35, 36, 41, 42, 81, 82, 139, 140, 181, 182 |
| 103 | desA1, C125S, L18R, Q22E, R38A, L80F, R81E, L85V, I86V, I92F | 197, 198, 201, 202, 7, 8, 27, 28, 35, 36, 41, 42, 81, 82, 139, 140, 167, 168 |
| 104 | desA1, C125S, L18R, Q22E, E61D, L80F, R81E, L85V, I86V, I92F | 197, 198, 201, 202, 7, 8, 25, 26, 29, 30, 35, 36, 41, 42, 57, 58, 81, 82, 139, 140, 167, 168 |
| 105 | desA1, C125S, L18R, Q22E, E68D, L80F, R81E, L85V, I86V, I92F | 197, 198, 201, 202, 7, 8, 25, 26, 29, 30, 35, 36, 41, 42, 63, 64, 81, 82, 139, 140, 167, 168 |

In the table, "desA1" denotes the deletion of alanine, the amino acid at position 1 in interleukin-2. Table 2 below shows the full-length protein sequences of the interleukin-2 analogs. The bold texts in Table 2 below indicate positions of modification.

TABLE 2

| Amino acid sequences of interleukin-2 analogs | | | |
|---|---|---|---|
| **Analog** | **Amino acid sequence** | **SEQ ID NO of amino acid sequence** | **SEQ ID NO of nucleotide sequence** |
| 1 | PTSSSTKKT QLQLEHLLLD LQMILNGINN YKNPKLTRML TFKFYMPKKA TELKHLQCLE EELKPLEEVL NLAQSKNFHL RPRDLISNIN VIVLELKGSE TTFMCEYADE TATIVEFLNR WITF**S**QSIIS TLT | 2 | 107 |
| 2 | PTSSSTKKT QLQLEHLLLD LQMILNGINN YKNPKLTRML TFKFYMPKKA TELKHLQCLE EELKPLEEVL NLAQSKNFHL RPRDLI**C**NIN VIVLELKGSE TTFMCEYADE TATIVEFLNR WITF**S**QSIIS TLT | 3 | 108 |

(continued)

| Amino acid sequences of interleukin-2 analogs | | | |
|---|---|---|---|
| **Analog** | **Amino acid sequence** | **SEQ ID NO of amino acid sequence** | **SEQ ID NO of nucleotide sequence** |
| 3 | PTSSSTKKT QLQLEHLLLD LQMILNGINN Y**C**NPKLTRML TFKFYMPKKA TELKHLQCLE EELKPLEEVL NLAQSKNFHL RPRDLISNIN VIVLELKGSE TTFMCEYADE TATIVEFLNR WITF**S**QSIIS TLT | 4 | 109 |
| 4 | PTSSSTKKT QLQLEHLLLD LQMILNGINN YKNP**C**LTRML TFKFYMPKKA TELKHLQCLE EELKPLEEVL NLAQSKNFHL RPRDLISNIN VIVLELKGSE TTFMCEYADE TATIVEFLNR WITF**S**QSIIS TLT | 5 | 110 |
| 5 | PTSSSTKKT QLQLEHLLLD LQMILNGINN YKNPKLTRML TF**C**FYMPKKA TELKHLQCLE EELKPLEEVL NLAQSKNFHL RPRDLISNIN VIVLELKGSE TTFMCEYADE TATIVEFLNR WITF**S**QSIIS TLT | 6 | 111 |
| 6 | PTSSSTKKT QLQLEHLLLD LQMILNGINN YKNPKLTRML TFKFYMP**C**KA TELKHLQCLE EELKPLEEVL NLAQSKNFHL RPRDLISNIN VIVLELKGSE TTFMCEYADE TATIVEFLNR WITF**S**QSIIS TLT | 7 | 112 |
| 7 | PTSSSTKKT QLQLEHLLLD LQMILNGINN YKNPKLTRML TFKFYMPK**C**A TELKHLQCLE EELKPLEEVL NLAQSKNFHL RPRDLISNIN VIVLELKGSE TTFMCEYADE TATIVEFLNR WITF**S**QSIIS TLT | 8 | 113 |
| 8 | PTSSSTKKT QLQLEHLLLD LQMILNGINN YKNPKLTRML TFKFYMPKKA TELKHLQCLE EELKPLEEVL NLAQS**C**NFHL RPRDLISNIN VIVLELKGSE TTFMCEYADE TATIVEFLNR WITF**S**QSIIS TLT | 9 | 114 |
| 9 | PTSSSTKKT QLQLEHLLLD LQMILNGINN YKNPKLT**A**ML TFKFYMPKKA TELKHLQCLE EELKPLEEVL NLAQSKNFHL RPRDLISNIN VIVLELKGSE TTFMCEYADE TATIVEFLNR WITF**S**QSIIS TLT | 10 | 115 |
| 10 | PTSSSTKKT QLQLEHLLLD LQMILNGINN YKNPKLTRML T**K**KFYMPKKA TELKHLQCLE EELKPLEEVL NLAQSKNFHL RPRDLISNIN VIVLELKGSE TTFMCEYADE TATIVEFLNR WITF**S**QSIIS TLT | 11 | 116 |
| 11 | PTSSSTKKT QLQLEHLLLD LQMILNGINN YKNPKLTRML T**A**KFYMPKKA TELKHLQCLE EELKPLEEVL NLAQSKNFHL RPRDLISNIN VIVLELKGSE TTFMCEYADE TATIVEFLNR WITF**S**QSIIS TLT | 12 | 117 |
| 12 | PTSSSTKKT QLQLEHLLLD LQMILNGINN YKNPKLT**A**ML T**K**KFYMPKKA TELKHLQCLE EELKPLEEVL NLAQSKNFHL RPRDLISNIN VIVLELKGSE TTFMCEYADE TATIVEFLNR WITF**S**QSIIS TLT | 13 | 118 |
| 13 | PTSSSTKKT QLQLEHLLLD LQMILNGINN YKNPKLT**A**ML T**A**KFYMPKKA TELKHLQCLE EELKPLEEVL NLAQSKNFHL RPRDLISNIN VIVLELKGSE TTFMCEYADE TATIVEFLNR WITF**S**QSIIS TLT | 14 | 119 |

(continued)

| Amino acid sequences of interleukin-2 analogs | | | |
|---|---|---|---|
| **Analog** | **Amino acid sequence** | **SEQ ID NO of amino acid sequence** | **SEQ ID NO of nucleotide sequence** |
| 14 | PTSSSTKKT QLQLEHLL**Y**D LQMILNGINN YKNPKLT**A**ML T**K**KFYMPKKA TELKHLQCLE EELKPLEEVL NLAQSKNFHL RPRDLISNIN VIVLELKGSE TTFMCEYADE TATIVEFLNR WITF**S**QSIIS TLT | 15 | 120 |
| 15 | PTSSSTKKT QLQLEHLLLD LQMILNGINN YKNPKLT**A**ML T**K**KFYMPKKA TELKHLQCLE EELKPLEEVL NLAQSKNFHL RPR**E**LISNIN VIVLELKGSE TTFMCEYADE TATIVEFLNR WITF**S**QSIIS TLT | 16 | 121 |
| 16 | PTSSSTKKT QLQLEHLLLD LQMILNGINN YKNPKLT**A**ML T**K**KFYMPKKA TELKHLQCLE EELKPLEEVL NLAQSKNFHL RPRDLIS**Q**IN VIVLELKGSE TTFMCEYADE TATIVEFLNR WITF**S**QSIIS TLT | 17 | 122 |
| 17 | PTSSSTKKT QLQLEHLLLD LQMILNGINN YKNPKLT**A**ML T**K**KFYMPKKA TELKHLQCLE EELKPLEEVL NLAQSKNFHL RPRDLISNIN **T**IVLELKGSE TTFMCEYADE TATIVEFLNR WITF**S**QSIIS TLT | 18 | 123 |
| 18 | PTSSSTKKT QLQLEHLLLD LQMILNGINN YKNPKLT**A**ML T**K**KFYMPKKA TELKHLQCLE **Q**ELKPLEEVL NLAQSKNFHL RPRDLISNIN VIVLELKGSE TTFMCEYADE TATIVEFLNR WITF**S**QSIIS TLT | 19 | 124 |
| 19 | PTSSSTKKT QLQLEHLLLD LQMILNGINN YKNPKLT**A**ML T**K**KFYMPKKA TELKHLQCLE EELKPLEEVL NLAQSKNFHL **D**PRDLISNIN V**F**VLELKGSE TTFMCEYADE TATIVEFLNR WITF**S**QSIIS TLT | 20 | 125 |
| 20 | PTSSSTKKT QLQLEHLLLD LQMILNGINN YKNPKLT**A**ML T**K**KFYMPKKA TELKHLQCLE EELKPLEEVL NLAQSKNFHL RPRD**VV**SNIN V**F**VLELKGSE TTFMCEYADE TATIVEFLNR WITF**S**QSIIS TLT | 21 | 126 |
| 21 | PTSSSTKKT QLQLEHLLLD LQMILNGINN YKNPKLT**A**ML T**K**KFYMPKKA TELKHLQCLE EELKPLEEVL NLAQSKNFH**F** **D**PRD**VV**SNIN V**F**VLELKGSE TTFMCEYADE TATIVEFLNR WITF**S**QSIIS TLT | 22 | 127 |
| 22 | PTSSSTKKT Q**V**QLEHLLLD LQMILNGINN YKNPKLT**A**ML T**K**KFYMPKKA TELKHLQCLE EELKPLEEVL NLAQSKNFHL RPRDLISNIN VIVLELKGSE TTFMCEYADE TATIVEFLNR WITF**S**QSIIS TLT | 23 | 128 |
| 23 | PTSSSTKKT Q**F**QLEHLLLD LQMILNGINN YKNPKLT**A**ML T**K**KFYMPKKA TELKHLQCLE EELKPLEEVL NLAQSKNFHL RPRDLISNIN VIVLELKGSE TTFMCEYADE TATIVEFLNR WITF**S**QSIIS TLT | 24 | 129 |
| 24 | PTSSSTKKT QLQLEHLL**V**D LQMILNGINN YKNPKLT**A**ML T**K**KFYMPKKA TELKHLQCLE EELKPLEEVL NLAQSKNFHL RPRDLISNIN VIVLELKGSE TTFMCEYADE TATIVEFLNR WITF**S**QSIIS TLT | 25 | 130 |

(continued)

| Amino acid sequences of interleukin-2 analogs | | | |
|---|---|---|---|
| **Analog** | **Amino acid sequence** | **SEQ ID NO of amino acid sequence** | **SEQ ID NO of nucleotide sequence** |
| 25 | PTSSSTKKT QLQLEHLL**F**D LQMILNGINN YKNPKLT**A**ML T**K**KFYMPKKA TELKHLQCLE EELKPLEEVL NLAQSKNFHL RPRDLISNIN VIVLELKGSE TTFMCEYADE TATIVEFLNR WITF**S**QSIIS TLT | 26 | 131 |
| 26 | PTSSSTKKT QLQLEHLLLD LQMILNGINN YKNPKLT**A**ML T**K**KFYMPKKA TELKHLQCLE EELKPLEEVL NLAQSKNFHL RPRDLISN**F**N VIVLELKGSE TTFMCEYADE TATIVEFLNR WITF**S**QSIIS TLT | 27 | 132 |
| 27 | PTSSSTKKT QLQLEHLLLD LQMILNGINN YKNPKLT**A**ML T**K**KFYMPKKA TELKHLQCLE EELKPLEEVL NLAQSKNFHL RPRDLISNIN **F**IVLELKGSE TTFMCEYADE TATIVEFLNR WITF**S**QSIIS TLT | 28 | 133 |
| 28 | PTSSSTKKT QLQLEHLLLD LQMILNGINN YKNPKLT**A**ML T**K**KFYMPKKA TELKHLQCLE EELKPLEEVL NLAQSKNFHL RPRDLISNIN VIV**V**ELKGSE TTFMCEYADE TATIVEFLNR WITF**S**QSIIS TLT | 29 | 134 |
| 29 | PTSSSTKKT QLQLEHLLLD LQMILNGINN YKNPKLT**A**ML T**K**KFYMPKKA TELKHLQCLE EELKPLEEVL NLAQSKNFHL RPRDLISNIN VIVLELKGSE TTFMCEYADE TATIVEFLNR WITF**ST**SIIS TLT | 30 | 135 |
| 30 | PTSSSTKKT QLQLEHLLLD LQMILNGINN YKNPKLT**A**ML TFKFYMPKKA TELKHLQCLE EELKPLEEVL NLAQSKNFHL **D**PRDLISNIN V**F**VLELKGSE TTFMCEYADE TATIVEFLNR | 31 | 136 |
| | WITF**S**QSIIS TLT | | |
| 31 | PTSSSTKKT QLQLEHLLLD LQMILNGINN YKNPKLT**A**ML TFKFYMPKKA TELKHLQCLE EELKPLEEVL NLAQSKNFHL RPR**E**LISNIN VIVLELKGSE TTFMCEYADE TATIVEFLNR WITF**S**QSIIS TLT | 32 | 137 |
| 32 | PTSSSTKKT QLQLEHLLLD LQMILNGINN YKNPKLT**A**ML TFKFYMPKKA TELKHLQCLE EELKPLEEVL NLAQSKNFHL **D**PR**E**LISNIN V**F**VLELKGSE TTFMCEYADE TATIVEFLNR WITF**S**QSIIS TLT | 33 | 138 |
| 33 | PTSSSTKKT QLQLEHLLLD LQMILNGINN YKNPKLT**A**ML TFKFYMPKKA TELKHLQCLE EELKPLEEVL NLAQSKNFH**F** RPRDLISNIN VIVLELKGSE TTFMCEYADE TATIVEFLNR WITF**S**QSIIS TLT | 34 | 139 |
| 34 | PTSSSTKKT QLQLEHLLLD LQMILNGINN YKNPKLT**A**ML TFKFYMPKKA TELKHLQCLE EELKPLEEVL NLAQSKNFH**F** RPR**E**LISNIN VIVLELKGSE TTFMCEYADE TATIVEFLNR WITF**S**QSIIS TLT | 35 | 140 |

(continued)

| Amino acid sequences of interleukin-2 analogs | | | |
|---|---|---|---|
| **Analog** | **Amino acid sequence** | **SEQ ID NO of amino acid sequence** | **SEQ ID NO of nucleotide sequence** |
| 35 | PTSSSTKKT QLQLEHLLLD LQMILNGINN YKNPKLT**A**ML TFKFYMPKKA TELKHLQCLE EELKPLEEVL NLAQSKNFHL RPRDLISNIN VIV**F**E**F**KGSE TTFMCEYADE TATIVEFLNR WITF**S**QSIIS TLT | 36 | 141 |
| 36 | PTSSSTKKT QLQLEHLLLD LQMILNGINN YKNPKLT**A**ML TFKFYMPKKA TELKHLQCLE EELKPLEEVL NLAQSKNFHL RPRDLISNIN VIV**F**E**V**KGSE TTFMCEYADE TATIVEFLNR WITF**S**QSIIS TLT | 37 | 142 |
| 37 | PTSSSTKKT QLQLEHLLLD LQMILNGINN YKNPKLT**A**ML TFKFYMPKKA TELKHLQCLE EELKPLEEVL NLAQSKNFHL RPRDLISNIN VIV**F**E**I**KGSE TTFMCEYADE TATIVEFLNR WITF**S**QSIIS TLT | 38 | 143 |
| 38 | PTSSSTKKT QLQLEHLLLD LQMILNGINN YKNPKLT**A**ML T**K**KFYMPKKA TELKHLQCLE EELKPLEEVL NLAQSKNFHL **D**PRDLISNIN V**F**V**F**E**F**KGSE TTFMCEYADE TATIVEFLNR WITF**S**QSIIS TLT | 39 | 144 |
| 39 | PTSSSTKKT QLQLEHLLLD LQMILNGINN YKNPKLT**A**ML T**K**KFYMPKKA TELKHLQCLE EELKPLEEVL NLAQSKNFHL **D**PRDLISNIN V**F**V**F**E**V**KGSE TTFMCEYADE TATIVEFLNR WITF**S**QSIIS TLT | 40 | 145 |
| 40 | PTSSSTKKT QLQLEHLLLD LQMILNGINN YKNPKLT**A**ML T**K**KFYMPKKA TELKHLQCLE EELKPLEEVL NLAQSKNFHL **D**PRDLISNIN V**F**V**F**E**I**KGSE TTFMCEYADE TATIVEFLNR WITF**S**QSIIS TLT | 41 | 146 |
| 41 | PTSSSTKKT QLQLEHLLLD LQMILNGINN YKNPKLTRML TFKFYMPKKA TELKHLQCLE EELKPLEEVL NLAQSKNFH**F** **D**PRD**VV**SNIN V**F**VLELKGSE TTFMCEYADE TATIVEFLNR WITF**S**QSIIS TLT | 42 | 147 |
| 42 | PTSSSTKKT QLQLEHLLLD LQMILNGINN YKNPKLT**A**ML T**K**KFYMPKKA TELKHLQCLE EELKPLEEVL NLAQSKNFHL **E**PRDLISNIN V**F**VLELKGSE TTFMCEYADE TATIVEFLNR WITF**S**QSIIS TLT | 43 | 148 |
| 43 | PTSSSTKKT QLQLEHLLLD LQMILNGINN YKNPKLT**A**ML T**K**KFYMPKKA TELKHLQCLE EELKPLEEVL NLAQSKNFHL **D**PRDLISNIN V**L**VLELKGSE TTFMCEYADE TATIVEFLNR WITF**S**QSIIS TLT | 44 | 149 |
| 44 | PTSSSTKKT QLQLEHLLLD LQMILNGINN YKNPKLT**A**ML T**K**KFYMPKKA TELKHLQCLE EELKPLEEVL NLAQSKNFHL **D**PR**V**LISNIN V**F**VLELKGSE TTFMCEYADE TATIVEFLNR WITF**S**QSIIS TLT | 45 | 150 |
| 45 | PTSSSTKKT QLQLEHLLLD LQMILNGINN YKNPKLT**A**ML T**K**KFYMPKKA TELKHLQCLE EELKPLEEVL NLAQSKNFHL **D**PR**F**LISNIN V**F**VLELKGSE TTFMCEYADE TATIVEFLNR WITF**S**QSIIS TLT | 46 | 151 |

(continued)

| Amino acid sequences of interleukin-2 analogs | | | |
|---|---|---|---|
| **Analog** | **Amino acid sequence** | **SEQ ID NO of amino acid sequence** | **SEQ ID NO of nucleotide sequence** |
| 46 | PTSSSTKKT QLQLEHLLL**V** LQMILNGINN YKNPKLT**A**ML T**K**KFYMPKKA TELKHLQCLE EELKPLEEVL NLAQSKNFHL **D**PRDLISNIN V**F**VLELKGSE TTFMCEYADE TATIVEFLNR WITF**S**QSIIS TLT | 47 | 152 |
| 47 | PTSSSTKKT QLQLEHLLL**F** LQMILNGINN YKNPKLT**A**ML T**K**KFYMPKKA TELKHLQCLE EELKPLEEVL NLAQSKNFHL **D**PRDLISNIN V**F**VLELKGSE TTFMCEYADE TATIVEFLNR WITF**S**QSIIS TLT | 48 | 153 |
| 48 | PTSSSTKKT QLQLEHLLLD LQMILNGINN YKNPKLT**A**ML T**K**KFYMPKKA TELKHLQCLE EELKPLEEVL NLAQSKNFHL **D**PRDLIS**V**IN V**F**VLELKGSE TTFMCEYADE TATIVEFLNR WITF**S**QSIIS TLT | 49 | 154 |
| 49 | PTSSSTKKT QLQLEHLLLD LQMILNGINN YKNPKLT**A**ML T**K**KFYMPKKA TELKHLQCLE EELKPLEEVL NLAQSKNFHL **D**PRDLIS**F**IN V**F**VLELKGSE TTFMCEYADE TATIVEFLNR WITF**S**QSIIS TLT | 50 | 155 |
| 50 | PTSSSTKKT QLQLEHLLLD LQMILNGINN YKNPKLTRML T**K**KFYMPKKA TELKHLQCLE EELKPLEEVL NLAQSKNFH**F** **D**PRD**VV**SNIN V**F**VLELKGSE TTFMCEYADE TATIVEFLNR WITF**S**QSIIS TLT | 51 | 156 |
| 51 | PTSSSTKKT QLQLEHLLLD LQMILNGINN YKNPKLTRML TFKFYMPKKA TELKHLQCLE **Q**ELKPLEEVL NLAQSKNFHL **D**PRDLISNIN V**F**VLELKGSE TTFMCEYADE TATIVEFLNR WITF**S**QSIIS TLT | 52 | 157 |
| 52 | PTSSSTKKT QLQLEHLLLD LQMILNGINN YKNPKLT**A**ML T**K**KFYMPKKA TELKHLQCLE EELKPLEEVL NLAQSKNFH**F** **D**PRDLISNIN V**F**VLELKGSE TTFMCEYADE TATIVEFLNR WITF**S**QSIIS TLT | 53 | 158 |
| 53 | PTSSSTKKT QLQLEHLLLD LQMILNGINN YKNPKLT**A**ML T**K**KFYMPKKA TELKHLQCLE EELKPLEEVL NLAQSKNFH**F** **D**PR**E**LISNIN V**F**VLELKGSE TTFMCEYADE TATIVEFLNR WITF**S**QSIIS TLT | 54 | 159 |
| 54 | PTSSSTKKT QLQLEHLLLD LQMILNGINN YKNPKLT**A**ML T**K**KFYMPKKA TELKHLQCLE EELKPLEEVL NLA**H**SKNFHL **D**PRDLISNIN V**F**VLELKGSE TTFMCEYADE TATIVEFLNR WITF**S**QSIIS TLT | 55 | 160 |
| 55 | PTSSSTKKT QLQLEHLLLD LQMILNGINN YKNPKLT**A**ML T**K**KFYMPKKA TELKHLQCLE EELKPLEEVL NLA**H**SKNFH**F** **D**PRDLISNIN V**F**VLELKGSE TTFMCEYADE TATIVEFLNR WITF**S**QSIIS TLT | 56 | 161 |
| 56 | PTSSSTKKT QLQLEHLLLD LQMILNGINN YKNPKLT**A**ML T**K**KF**A**MPKKA TELKHLQCLE EELKPLEEVL NLA**H**SKNFHL **D**PRDLISNIN V**F**VLELKGSE TTFMCEYADE TATIVEFLNR WITF**S**QSIIS TLT | 57 | 162 |

(continued)

| Amino acid sequences of interleukin-2 analogs | | | |
|---|---|---|---|
| **Analog** | **Amino acid sequence** | **SEQ ID NO of amino acid sequence** | **SEQ ID NO of nucleotide sequence** |
| 57 | PTSSSTKKT QLQLEHLLLD LQMILNGINN YKNPKLT**A**ML T**K**KF**A**MPKKA TELKHLQCLE EELKPLEEVL NLA**H**SKNFH**F** **D**PRDLISNIN V**F**VLELKGSE TTFMCEYADE TATIVEFLNR WITF**S**QSIIS TLT | 58 | 163 |
| 58 | PTSSSTKKT QLQLEHLLLD LQMILNGINN YKNPKLT**A**ML T**K**KFYMPKKA TELKHLQCLE EELKPLEEVL NLAQSKNFH**F** **D**PRD**AA**SNIN V**F**VLELKGSE TTFMCEYADE TATIVEFLNR WITF**S**QSIIS TLT | 59 | 164 |
| 59 | PTSSSTKKT QLQLEHLLLD LQMILNGINN YKNPKLT**A**ML T**K**KFYMPKKA TELKHLQCLE EELKPLEEVL NLAQSKNFH**F** **D**PRD**AA**SNIN V**Y**VLELKGSE TTFMCEYADE TATIVEFLNR WITF**S**QSIIS TLT | 60 | 165 |
| 60 | PTSSSTKKT QLQLEHLLLD LQMILNGINN YKNPKLT**A**ML T**K**KF**A**MPKKA TELKHLQCLE EELKPLEEVL NLAQSKNFH**Y** RPRD**AA**SNIN V**Y**VLELKGSE TTFMCEYADE TATIVEFLNR WITF**S**QSIIS TLT | 61 | 166 |
| 61 | PTSSSTKKT QLQLEHLLLD LQMILNGINN YKNPKLT**A**ML T**K**KFYMPKKA TELKHLQCLE EELKPLEEVL NLAQSKNFH**Y** **D**PRD**GV**SNIN V**Y**VLELKGSE TTFMCEYADE TATIVEFLNR WITF**S**QSIIS TLT | 62 | 167 |
| 62 | PTSSSTKKT QLQLEHLLLD LQMILNGINN YKNPKLT**A**ML T**K**KFYMPKKA TELKHLQCLE EELKPLEEVL NLAQSKNFH**W** **E**PRD**GA**SNIN V**F**VLELKGSE TTFMCEYADE TATIVEFLNR WITF**S**QSIIS TLT | 63 | 168 |
| 63 | PTSSSTKKT QLQLEHLLLD LQMILNGINN YKNPKLT**A**ML T**K**KFYMPKKA TELKHLQCLE EELKPLEEVL NLAQSKNFH**D** **E**PRD**TG**SNIN V**F**VLELKGSE TTFMCEYADE TATIVEFLNR WITF**S**QSIIS TLT | 64 | 169 |
| 64 | PTSSSTKKT QLQLEHLLLD LQMILNGINN YKNPKLT**A**ML T**K**KFYMPKKA TELKHLQCLE EELKPLEEVL NLAQSKNFH**Y** **N**PRD**VV**SNIN V**Y**VLELKGSE TTFMCEYADE TATIVEFLNR WITF**S**QSIIS TLT | 65 | 170 |
| 65 | PTSSSTKKT QLQLEHLLLD LQMILNGINN YKNPKLT**A**ML T**K**KFYMPKKA TELKHLQCLE EELKPLEEVL NLAQSKNFH**Y** **E**PRD**VV**SNIN V**F**VLELKGSE TTFMCEYADE TATIVEFLNR WITF**S**QSIIS TLT | 66 | 171 |
| 66 | PTSSSTKKT QLQLEHLLLD LQMILNGINN YKNPKLT**A**ML T**K**KFYMPKKA TELKHLQCLE EELKPLEEVL NLAQSKNFH**F** **E**PRD**FV**SNIN V**F**VLELKGSE TTFMCEYADE TATIVEFLNR WITF**S**QSIIS TLT | 67 | 172 |
| 67 | PTSSSTKKT QLQLEHLLLD LQMILNGINN YKNPKLT**A**ML T**K**KFYMPKKA TELKHLQCLE EELKPLEEVL NLAQSKNFH**Y** **D**PRD**FV**SNIN V**W**VL**D**LKGSE TTFMCEYADE TATIVEFLNR WITF**S**QSIIS TLT | 68 | 173 |

(continued)

| Amino acid sequences of interleukin-2 analogs | | | |
|---|---|---|---|
| **Analog** | **Amino acid sequence** | **SEQ ID NO of amino acid sequence** | **SEQ ID NO of nucleotide sequence** |
| 68 | PTSSSTKKT QLQLEHLLLD LQMILNGINN YKNPKLT**A**ML T**K**KFYMPKKA TELKHLQCLE EELKPLEEVL NLAQSKNFH**F** **E**PRD**IV**SNIN **EF**VLELKGSE TTFMCEYADE TATIVEFLNR WITF**S**QSIIS TLT | 69 | 174 |
| 69 | PTSSSTKKT QLQLEHLLLD LQMILNGINN YKNPKLT**A**ML T**K**KFYMPKKA TELKHLQCLE EELKPLEEVL NLAQSKNFH**Y** **E**PRD**FL**SNIN **EW**VL**D**LKGSE TTFMCEYADE TATIVEFLNR WITF**S**QSIIS TLT | 70 | 175 |
| 70 | PTSSSTKKT QLQLEHLLLD LQMILNGINN YKNPKLT**A**ML T**K**KFYMPKKA TELKHLQCLE EELKPLEEVL NLAQSKNFH**Y** **D**PRD**VV**SNIN V**F**VLELKGSE TTFMCEYADE TATIVEFLNR WITF**S**QSIIS TLT | 71 | 176 |
| 71 | PTSSSTKKT QLQLEHLLLD LQMILNGINN YKNPKLT**A**ML T**K**KFYMPKKA TELKHLQCLE EELKPLEEVL NLAQSKNFH**F** **E**PRD**VV**SNIN V**F**VLELKGSE TTFMCEYADE TATIVEFLNR WITF**S**QSIIS TLT | 72 | 177 |
| 72 | PTSSSTKKT QLQLEHLLLD LQMILNGINN YKNPKLT**A**ML T**K**KFYMPKKA TELKHLQCLE EELKPLEEVL NLAQSKNFH**F** **D**PRD**VG**SNIN V**F**VLELKGSE TTFMCEYADE TATIVEFLNR WITF**S**QSIIS TLT | 73 | 178 |
| 73 | PTSSSTKKT QLQLEHLLLD LQMILNGINN YKNPKLT**A**ML T**K**KFYMPKKA TELKHLQCLE EELKPLEEVL NLAQSKNFH**F** **D**PRD**WV**SNIN V**F**VLELKGSE TTFMCEYADE TATIVEFLNR WITF**S**QSIIS TLT | 74 | 179 |
| 74 | PTSSSTKKT QLQLEHLLLD LQMILNGINN YKNPKLT**D**ML T**K**KFYMPKKA TELKHLQCLE EELKPLEEVL NLAQSKNFH**Y** **D**PRD**VV**SNIN V**F**VLELKGSE TTFMCEYADE TATIVEFLNR WITF**S**QSIIS TLT | 75 | 180 |
| 75 | PTSSSTKKT QLQLEHLLLD LQMILNGINN YKNPKLT**A**ML T**K**KF**A**MPKKA TELKHLQCLE EELKPLEEVL NLAQSKNFH**F** EPRD**VV**SNIN V**F**VLELKGSE TTFMCEYADE TATIVEFLNR WITF**S**QSIIS TLT | 76 | 181 |
| 76 | PTSSSTKKT QLQLEHLLLD LQMILNGINN YKNPKLT**A**ML T**KQ**FYMPKKA TELKHLQCLE **R**ELKPLEEVL NLAQSKNFH**F** **D**PRD**VG**SNIN V**F**VLELKGSE TTFMCEYADE TATIVEFLNR WITF**S**QSIIS TLT | 77 | 182 |
| 77 | PTSSSTKKT QLQLEHLLLD LQMILNGINN YKNPKLT**A**ML T**KE**FYMPKKA TELKHLQCLE **R**ELKPLEEVL NLAQSKNFH**F** **D**PRD**WV**SNIN V**F**VLELKGSE TTFMCEYADE TATIVEFLNR WITF**S**QSIIS TLT | 78 | 183 |
| 78 | PTSSSTKKT QLQLEHLLLD LQMILNGINN YKNP**E**LT**A**ML T**K**KFYMPKKA TELKHLQCLE EELKPLEEVL NLAQSKNFH**F** **E**PRD**VV**SNIN V**F**VLELKGSE TTFMCEYADE TATIVEFLNR WITF**S**QSIIS TLT | 79 | 184 |

(continued)

| Amino acid sequences of interleukin-2 analogs | | | |
|---|---|---|---|
| **Analog** | **Amino acid sequence** | **SEQ ID NO of amino acid sequence** | **SEQ ID NO of nucleotide sequence** |
| 79 | PTSSSTKKT QLQLEHLLLD LQMILNGINN YKNP**E**LT**A**ML T**K**KFYMPKKA TELKHLQCLE EELKPLEEVL NLA**H**SKNFH**F** **E**PRD**VV**SNIN V**F**VLELKGSE TTFMCEYADE TATIVEFLNR WITF**S**QSIIS TLT | 80 | 185 |
| 80 | PTSSSTKKT QLQLEHLLLD LQMILNGINN YKNP**E**LT**A**ML T**K**KFYMPKKA TELKHLQCLE EELKPLEEVL NLA**H**SKNFH**F** **EG**RD**VV**SNIN V**F**VLELKGSE TTFMCEYADE TATIVEFLNR WITF**S**QSIIS TLT | 81 | 186 |
| 81 | PTSSSTKKT QLQLEHLLLD LQMILNGINN YKNP**E**LT**A**ML T**K**KFYMPKKA TELKHLQCLE EELKPLEEVL NLA**H**SKNFH**F** **EV**RD**VV**SNIN V**F**VLELKGSE TTFMCEYADE TATIVEFLNR WITF**S**QSIIS TLT | 82 | 187 |
| 82 | PTSSSTKKT QLQLEHL**R**LD L**E**MILNGINN YKNPKLTRML TFKFYMPKKA TELKHLQCLE EELKPLEEVL NLAQSKNFH**F** **D**PRD**EV**SNIN V**F**VLELKGSE TTFMCEYADE TATIVEFLNR WITF**S**QSIIS TLT | 83 | 188 |
| 83 | PTSSSTKKT QLQLEHL**RR**D L**E**MILNGINN YKNPKLTRML TFKFYMPKKA TELKHLQCLE EELKPLEEVL NLAQSKNFH**F** **D**PRD**EV**SNIN V**F**VLELKGSE TTFMCEYADE TATIVEFLNR WITF**S**QSIIS TLT | 84 | 189 |
| 84 | PTSSSTKKT QLQLEHL**R**LD L**E**MILNGINN YKNPKLTRML TFKFYMPKKA TELKHLQCLE EELKPLEEVL NLAQSKNFH**V** **D**PRD**EV**SNIN V**F**VLELKGSE TTFMCEYADE TATIVEFLNR WITF**S**QSIIS TLT | 85 | 190 |
| 85 | PTSSSTKKT QLQLEHLLLD LQMILNGINN YKNPKLTRML TFKFYMPKKA TELKHLQCLE EELKPLEEVL NLAQSKNFH**F** **E**PRD**VV**SNIN V**F**VLELKGSE TTFMCEYADE TATIVEFLNR WITF**S**QSIIS TLT | 86 | 191 |
| 86 | PTSSSTKKT QLQLEHL**R**LD L**E**MILNGINN YKNPKLTRML TFKFYMPKKA TELKHLQCLE EELKPLEEVL NLAQSKNFH**F** **E**PRD**VV**SNIN V**F**VLELKGSE TTFMCEYADE TATIVEFLNR WITF**S**QSIIS TLT | 87 | 192 |
| 87 | PTSSSTKKT QLQLEHL**RR**D L**E**MILNGINN YKNPKLTRML TFKFYMPKKA TELKHLQCLE EELKPLEEVL NLAQSKNFH**F** **E**PRD**VV**SNIN V**F**VLELKGSE TTFMCEYADE TATIVEFLNR WITF**S**QSIIS TLT | 88 | 193 |
| 88 | PTSSSTKKT QLQLEHLLLD LQMILNGINN YKNPKLTRML TFKFYMPKKA TELKHLQCLE **D**ELKPLEEVL NLAQSKNFH**F** **E**PRD**VV**SNIN V**F**VLELKGSE TTFMCEYADE TATIVEFLNR WITF**S**QSIIS TLT | 89 | 194 |
| 89 | PTSSSTKKT QLQLEHLLLD LQMILNGINN YKNPKLT**A**ML TFKFYMPKKA TELKHLQCLE EELKPLE**Q**VL NLAQSKNFH**F** **E**PRD**VV**SNIN V**F**VLELKGSE TTFMCEYADE TATIVEFLNR WITF**S**QSIIS TLT | 90 | 195 |

(continued)

| Amino acid sequences of interleukin-2 analogs | | | |
|---|---|---|---|
| **Analog** | **Amino acid sequence** | **SEQ ID NO of amino acid sequence** | **SEQ ID NO of nucleotide sequence** |
| 90 | PTSSSTKKT QLQLEHLLLD LQMILNGINN YKNPKLTRML T**W**KFYMPKKA TELKHLQCLE EELKPLEEVL NLAQSKNFH**F** **E**PRD**VV**SNIN V**F**VLELKGSE TTFMCEYADE TATIVEFLNR WITF**S**QSIIS TLT | 91 | 196 |
| 91 | PTSSSTKKT QLQLEHLLLD LQMILNGINN YKNPKLTRML TFKFYMPKKA TELKHLQCLE **Q**ELKPLEEVL NLAQSKNFH**F** **E**PRD**VV**SNIN V**F**VLELKGSE TTFMCEYADE TATIVEFLNR WITF**S**QSIIS TLT | 92 | 197 |
| 92 | PTSSSTKKT QLQLEHLLLD LQMILNGINN YKNPKLTRML TFKFYMPKKA TELKHLQCLE EELKPLEEVL NLAQSKNFH**F** **E**PRD**VV**SNIN **TF**VLELKGSE TTFMCEYADE TATIVEFLNR WITF**S**QSIIS TLT | 93 | 198 |
| 93 | PTSSSTKKT QLQLEHLLLD LQMILNGINN YKNPKLTRML TFKFYMPKKA TELKHLQCLE EELKPLEEVL NLAQSKNFH**F** **E**PR**EVV**SNIN **TF**VLELKGSE TTFMCEYADE TATIVEFLNR WITF**S**QSIIS TLT | 94 | 199 |
| 94 | PTSSSTKKT QLQLEHLLLD LQMILNGINN YKNPKLTRML TFKFYMPKKA TELKHLQCLE EELKPLEEVL NLAQSKNFH**F** **E**PRD**VV**SNIN V**F**V**F**E**F**KGSE TTFMCEYADE TATIVEFLNR WITF**S**QSIIS TLT | 95 | 200 |
| 95 | PTSSSTKKT QLQLEHLLLD LQMILNGINN YKNPKLTRML TFKFYMPKKA TELKHLQCLE EELKPLEE**G**L NLAQSKNFH**F** **E**PRD**VV**SNIN V**F**VLELKGSE TTFMCEYADE TATIVEFLNR WITF**S**QSIIS TLT | 96 | 201 |
| 96 | PTSSSTKKT QLQLEHLLLD LQMILNGINN YKNPKLTRML TFKFYMPKKA TELKHLQCLE EELKPLEE**G**L NLA**A**SKNFH**F** **E**PRD**VV**SNIN V**F**VLELKGSE TTFMCEYADE TATIVEFLNR WITF**S**QSIIS TLT | 97 | 202 |
| 97 | PTSSSTKKT QLQLEHLLLD LQMILNGINN YKNPKLT**A**ML TFKFYMPKKA TELKHLQCLE EELKPLEEVL NLAQSKNFH**F** **D**PRDLISNIN V**F**VLELKGSE TTFMCEYADE TATIVEFLNR WITF**S**QSIIS TLT | 98 | 203 |
| 98 | PTSSSTKKT QLQLEHLLLD LQMILNGINN YKNPKLT**A**ML TFKFYMPKKA TELKHLQCLE EELKPLEEVL NLAQSKNFH**F** **E**PRD**V**ISNIN V**F**VLELKGSE TTFMCEYADE TATIVEFLNR WITF**S**QSIIS TLT | 99 | 204 |
| 99 | PTSSSTKKT QLQLEHLLLD LQMILNGINN YKNPKLT**A**ML TFKFYMPKKA TELKHLQCLE EELKPLEEVL NLAQSKNFH**F** **E**PRDL**V**SNIN V**F**VLELKGSE TTFMCEYADE TATIVEFLNR WITF**S**QSIIS TLT | 100 | 205 |
| 100 | PTSSSTKKT QLQLEHLLLD LQMILNGINN YKNPKLTRML TFKFYMPKKA TELKHLQCLE EELKPLEEVL NLAQSKNFH**F** **E**PRD**YV**SNIN V**F**VLELKGSE TTFMCEYADE TATIVEFLNR WITF**S**QSIIS TLT | 101 | 206 |

(continued)

| Amino acid sequences of interleukin-2 analogs | | | |
|---|---|---|---|
| **Analog** | **Amino acid sequence** | **SEQ ID NO of amino acid sequence** | **SEQ ID NO of nucleotide sequence** |
| 101 | PTSSSTKKT QLQLEHLLLD LQMILNGINN YKNPKLTRML TFKFYMPKKA TELKHLQCLE EELKPLEEVL NLAQSKNFH**F** **E**PRDL**A**SNIN V**F**VLELKGSE TTFMCEYADE TATIVEFLNR WITF**S**QSIIS TLT | 102 | 207 |
| 102 | PTSSSTKKT QLQLEHLLLD LQMILNGINN YKNPKLTRML TFKFYMPKKA TELKHLQCLE EELKPLEEVL NLAQSKNFH**F** **E**PRD**VV**SNIN VIVLELKGSE TTFMCEYADE TATIVEFLNR WITF**S**QSIIS TLT | 103 | 208 |
| 103 | PTSSSTKKT QLQLEHL**R**LD L**E**MILNGINN YKNPKLT**A**ML TFKFYMPKKA TELKHLQCLE EELKPLEEVL NLAQSKNFH**F** **E**PRD**VV**SNIN V**F**VLELKGSE TTFMCEYADE TATIVEFLNR WITF**S**QSIIS TLT | 104 | 209 |
| 104 | PTSSSTKKT QLQLEHL**R**LD L**E**MILNGINN YKNPKLTRML TFKFYMPKKA TELKHLQCLE **D**ELKPLEEVL NLAQSKNFH**F** **E**PRD**VV**SNIN V**F**VLELKGSE TTFMCEYADE TATIVEFLNR WITF**S**QSIIS TLT | 105 | 210 |
| 105 | PTSSSTKKT QLQLEHL**R**LD L**E**MILNGINN YKNPKLTRML TFKFYMPKKA TELKHLQCLE EELKPLE**D**VL NLAQSKNFH**F** **E**PRD**VV**SNIN V**F**VLELKGSE TTFMCEYADE TATIVEFLNR WITF**S**QSIIS TLT | 106 | 211 |

**Example 2: Expression of interleukin-2 analogs**

The expression vectors constructed in Example 1 were used to express recombinant interleukin-2 analogs under the control of the T7 promoter. The expression of the E. *coli* strain E. *coli* BL21DE3 (E. *coli B* F$^-$*dcm ompT hsdS*($r_B^-m_B^-$) *gal* λ (DE3); Novagen) was transformed with each of the recombinant interleukin-2 analog expression vectors. A transformation method recommended by Novagen was employed. Each single colony transformed with each of the recombinant expression vectors was taken, inoculated into 2X Luria Broth medium containing ampicillin (50 μg/mL), and cultured at 37°C for 15 hours. The recombinant strain culture and 2X LB medium containing 30% glycerol were mixed at a ratio of 1:1 (v/v), and 1 mL of this mixture was dispensed into each cryo-tube, which was then stored at -150°C. This was used as a cell stock for producing each recombinant protein.

For the expression of the recombinant interleukin-2 analogs, one vial of each cell stock was dissolved and inoculated in 500 mL of 2X LB, and cultured with shaking at 37°C for 14-16 hours. The culture was terminated when the absorbance value at 600 nm reached 4.0 or higher, and the resultant culture was used as a seed culture. The seed culture was inoculated into 1.6 L of a fermentation medium by using a 5-L fermenter (Bioflo-320, NBS, USA), and initial fermentation was started. The culture conditions included a temperature of 37°C, an air volume of 2.0 L/min (1 vvm), and a stirring rate of 650 rpm, and pH 6.7 was maintained using 30% ammonia water. As for the fermentation process, when the nutrients in the culture medium were limited, an additional medium (feeding solution) was added, followed by fed-batch culture. The growth of the strain was monitored through OD values, and when the absorbance value reached 70 or higher, IPTG at a final concentration of 500 μM was introduced. The culture was further continued for about 23-25 hours after IPTG introduction, and after the termination of the culture, the recombinant strain was harvested by using a centrifuge and stored at -80°C until use.

**Example 3: Extraction and refolding of interleukin-2 analogs**

To convert each of the interleukin-2 analogs from the interleukin-2 analog-expressed E. *coli* obtained in Example 2 above into a soluble form, the cells were lysed and refolded. Cell pellets corresponding to a 100 mL aliquot of the culture were suspended in 1-200 mL of a lysis buffer (20 mM Tris-HCl pH 9.0, 1 mM EDTA pH 9.0, 0.2 M NaCl, 0.5% Triton X-100),

and then recombinant *E. coli* was lysed using a microfluidizer at 15,000 psi. After centrifugation at 13,900 g for 30 minutes, the supernatant was discarded, and the pellets were washed with 400 mL of a first wash buffer (50 mM Tris-HCl pH 8.0, 5 mM EDTA pH 9.0). After centrifugation under the same conditions as above, the supernatant was discarded, and the pellets were washed with 400 mL of a second wash buffer (50 mM Tris-HCl pH 8.0, 5 mM EDTA pH 9.0, 2% Triton X-100). After centrifugation under the same conditions as above, the supernatant was discarded, and the pellets were washed with 400 mL of a third wash buffer (50 mM Tris-HCl pH 8.0, 5 mM EDTA pH 9.0, 1% sodium deoxycholate). After centrifugation under the same conditions as above, the supernatant was discarded, and the pellets were washed with 400 mL of a fourth wash buffer (50 mM Tris-HCl pH 8.0, 5 mM EDTA pH 9.0, 1 M NaCl). After centrifugation under the same conditions and washing, the *E. coli* inclusion body pellets were obtained. The washed inclusion body pellets were re-suspended in 400 mL of a solubilizing/reducing buffer (6 M guanidine, 100 mM Tris pH 8.0, 2 mM EDTA pH 9.0, 50 mM DTT), and stirred at 50°C for 30 minutes. After 100 mL of distilled water was added to the solubilized/reduced interleukin-2 analog to dilute 6 M guanidine to 4.8 M guanidine, centrifugation at 13,900 g was conducted for 30 minutes, and then the pellets were discarded, and only the solution was obtained. After 185.7 mL of distilled water was further added to the diluted solution to dilute 4.8 M guanidine to 3.5 M guanidine, pH was adjusted to 5.0 by using 100% acetic acid. The pH-adjusted solution was stirred at room temperature for 1 hour. The solution with impurities precipitated was centrifuged at 13,900 g for 30 minutes, and the supernatant was discarded, and then the pellets were washed with the last wash buffer (3.5 M Guanidine, 20 mM Sodium Acetate pH 5.0, 5 mM DTT). Through centrifugation under the same conditions as above, the pellets were obtained. The washed interleukin-2 analog was dissolved in 400 mL of a refolding buffer (6 mM Guanidine, 100 mM Tris pH 8.0, 0.1 mM $CuCl_2$). The mixture solution was refolded by stirring at 4°C for 15-24 hours.

**Example 4: Size-exclusion column chromatography**

The interleukin-2 analog refolding solution obtained in Example 3 was concentrated to less than 1 mL to be applied to a size-exclusion column for purification. The column was equilibrated with a buffer (2 M guanidine, 100 mM Tris pH 8.0) before the introduction of the refolded solution, and after the introduction of the refolded solution, elution was conducted by flowing the buffer through the column. The eluted sample contained guanidine, and was thus exchanged with a stabilization solution (10 mM sodium acetate pH 4.5, 5% trehalose), and then purity was determined through RP-HPLC and peptide mapping analysis. The sample was used in the experiment when the measured purity reached 80% or higher.

**Example 5: Evaluation of binding affinity of interleukin-2 analogs for receptors**

In order to measure the binding affinities of the interleukin-2 analogs obtained in Example 4 for each of interleukin-2 alpha and beta receptors, surface plasmon resonance (SPR, BIACORE T200, GE Healthcare) was used. The binding affinities of the prepared analogs for the alpha and beta receptors were measured, and the binding affinity of each of the prepared analogs was compared with that of interleukin-2 analog 1 (aldesleukin).

First, an anti-human immunoglobulin antibody (Abcam, #ab97221) was immobilized to a CM5 chip (GE healthcare) to about 5,000 resonance unit (RU) through amine coupling, and then the interleukin-2 alpha receptor (SYMANSIS, #4102H) or interleukin-2 beta receptor (SYMANSIS, #4122H), to each of which a human immunoglobulin Fc region was linked, was bound to the immunoglobulin antibody using an antigen-antibody binding reaction and thus finally immobilized. Thereafter, the recombinant interleukin-2 analog prepared above was diluted with HBS-P+ buffer (Cytiva, BR100671) to various concentrations by serial dilution, and the diluted samples were allowed to flow through CM5 chips, to which the interleukin-2 receptors were finally immobilized, to measure the binding affinity of each interleukin-2 receptor. The binding affinity was measured using the association rate ($K_a$) and the dissociation rate ($K_d$), wherein the association rate was measured by flowing an interleukin-2 analog at a flow rate of 10 $\mu$L/min for 3 minutes while the dissociation rate was measured from each interleukin-2 receptor by flowing only the HBS-P+ buffer for the same period of time and at the same flow rate. Upon the completion of the measurement, the binding affinity of the receptor was evaluated according to the 1:1 binding fitting model in the Biaevaluation program.

Relative binding affinity ($K_D$) (%) = Dissociation constant of analog 1 (aldesleukin) ($K_d$) / Dissociation constant ($K_d$) of analog $\times$ 100

In Table 3 below, "undefinable" indicates that the binding to a corresponding receptor was observed in the surface plasmon resonance measurement, and thus the corresponding physical quantity cannot be defined for the corresponding receptor.

TABLE 3

| Relative binding affinity of interleukin-2 analogs for interleukin-2 alpha or beta receptor compared with interleukin-2 analog 1 (aldesleukin) | | |
|---|---|---|
| **Interleukin-2 receptor** | **Test substance** | **Relative binding affinity (%)** |
| Alpha receptor | Analog 1 | 100.0 |
| | Analog 9 | 74.5 |
| | Analog 12 | Undefinable |
| | Analog 13 | 1.1 |
| | Analog 15 | Undefinable |
| | Analog 16 | 0.2 |
| | Analog 17 | 29.6 |
| | Analog 19 | Undefinable |
| | Analog 20 | Undefinable |
| | Analog 21 | Undefinable |
| | Analog 31 | 5.0 |
| | Analog 34 | 9.4 |
| | Analog 35 | 31.7 |
| | Analog 41 | 121.3 |
| | Analog 52 | Undefinable |
| | Analog 53 | Undefinable |
| | Analog 86 | 71.1 |
| | Analog 88 | 101.5 |
| | Analog 90 | 98.4 |
| | Analog 91 | 7.9 |
| | Analog 92 | 97.3 |
| | Analog 93 | 92.8 |
| | Analog 95 | 10.7 |
| | Analog 96 | 14.9 |
| | Analog 97 | 18.8 |
| | Analog 98 | 7.7 |
| | Analog 99 | 19.9 |
| | Analog 100 | 29.1 |
| | Analog 101 | 24.7 |
| | Analog 102 | 151.4 |
| | Analog 103 | 6.1 |
| | Analog 104 | 122.4 |
| | Analog 105 | 246.8 |

(continued)

| Relative binding affinity of interleukin-2 analogs for interleukin-2 alpha or beta receptor compared with interleukin-2 analog 1 (aldesleukin) | | |
|---|---|---|
| **Interleukin-2 receptor** | **Test substance** | **Relative binding affinity (%)** |
| Beta receptor | Analog 01 | 100.0 |
| | Analog 09 | 337.4 |
| | Analog 12 | 166.2 |
| | Analog 13 | 148.6 |
| | Analog 14 | 129.7 |
| | Analog 15 | 98.1 |
| | Analog 16 | 1261.8 |
| | Analog 17 | 9.4 |
| | Analog 18 | 35.3 |
| | Analog 19 | 455.0 |
| | Analog 20 | 156.5 |
| | Analog 21 | 14084.2 |
| | Analog 24 | 37.9 |
| | Analog 25 | 21.7 |
| | Analog 31 | 235.7 |
| | Analog 34 | 321.8 |
| | Analog 35 | 232.7 |
| | Analog 41 | 22776.2 |
| | Analog 52 | 3821.1 |
| | Analog 53 | 690.7 |
| | Analog 55 | 3025.7 |
| | Analog 57 | 2569.7 |
| | Analog 58 | 7771.2 |
| | Analog 59 | 1533.5 |

(continued)

| Relative binding affinity of interleukin-2 analogs for interleukin-2 alpha or beta receptor compared with interleukin-2 analog 1 (aldesleukin) | | |
|---|---|---|
| **Interleukin-2 receptor** | **Test substance** | **Relative binding affinity (%)** |
| | Analog 61 | 1039.1 |
| | Analog 70 | 10199.2 |
| | Analog 71 | 17083.8 |
| | Analog 73 | 1591.8 |
| | Analog 74 | 8153.4 |
| | Analog 75 | 9571.2 |
| | Analog 76 | 1040.4 |
| | Analog 77 | 644.4 |
| | Analog 84 | 710.7 |
| | Analog 86 | 18745.8 |
| | Analog 88 | 13856.6 |
| | Analog 90 | 12776.2 |
| | Analog 91 | 7361.9 |
| | Analog 92 | 1510.3 |
| | Analog 93 | 696.8 |
| | Analog 94 | 35.5 |
| | Analog 95 | 17.1 |
| | Analog 96 | 229.3 |
| | Analog 97 | 3019.4 |
| | Analog 98 | 11084.5 |
| | Analog 99 | 1509.1 |
| | Analog 100 | 2534.1 |
| | Analog 101 | 113.1 |
| | Analog 102 | 4452.0 |
| | Analog 103 | 13100.0 |
| | Analog 104 | 25439.8 |
| | Analog 105 | 26837.8 |

As explicitly shown in the test results (Table 3), the interleukin-2 analogs of the present invention showed different levels of binding affinity for the interleukin-2 alpha receptor compared with native interleukin-2 or aldesleukin, for example showing no binding affinity or reduced or increased binding affinity compared with interleukin-2 analog 1. However, the interleukin-2 analogs of the present invention showed a stronger binding affinity for the interleukin-2 beta receptor compared with native interleukin-2 or aldesleukin. These results verified that the amino acid sequences of the interleukin-2 analogs had an influence on the binding affinity for the interleukin-2 alpha or beta receptor. These results suggest that the binding affinity for the interleukin-2 receptors can be altered by substitution of amino acids at specific positions.

These experimental results suggest that the interleukin-2 analogs according to the present invention had altered binding affinities for the interleukin-2 alpha and beta receptors and thus can be used in the development of various drugs using the same.

**Example 6: Linking reaction of interleukin-2 analogs and polyethylene glycol (3.4K PEG) linker and purification of interleukin-2 analog linkage products**

In order to prepare long-acting conjugates in which each of the interleukin-2 analogs obtained in Example 4 was linked to an immunoglobulin Fc region, linkage products in which the interleukin-2 analog was linked to one end of a polyethylene glycol (PEG) linker were prepared. For the preparation of the linkage products, interleukin-2 analogs 21, 41, 52, 86, 104 and 105 were used, and polyethylene glycol (ALD(2)3.4K PEG by NOF, Japan) with a molecular weight of 3.4 kDa having hydroxyl hydrogens modified with propyl aldehyde groups at both ends was used as the PEG linker. The PEG linker was attached to the N-terminus of each interleukin-2 analog. The molar ratio of the interleukin-2 analog and the PEG linker was 1:15 to 1:20, and these were reacted at 2°C to 10°C for 1 hour while the concentration of the interleukin-2 analog was 1 mg/mL or less. Particularly, the reaction was conducted under 100 mM potassium phosphate (pH 5.5), and 20 mM sodium cyanoborohydride (SCB) was added as a reducing agent. The reaction solution was exchanged with a 20 mM triethylamine (pH 8.0) buffer by using a desalting column, and then purified by Fractogel® EMD TMAE (S) (Merck Millipore) or Source 15Q (Cytiva) column using the concentration gradients of triethylamine (pH 8.0) and sodium chloride, thereby obtaining each interleukin-2 analog-3.4K PEG linkage product.

**Example 7: Preparation of interleukin-2 analog-3.4K PEG-immunoglobulin Fc region long-acting conjugates**

In order to prepare interleukin-2 analog-3.4K PEG-immunoglobulin Fc region long-acting conjugates, the mole ratio of each of the interleukin-2 analog-3.4K PEG linkage products obtained by the method of Example 6 and an immunoglobulin Fc region (SEQ ID NO: 438) was set to 1:10, and the total protein concentration was set to 30 mg/mL, and the reaction was conducted at 2-10°C for 15 to 16 hours. Particularly, the reaction was conducted under 100 mM potassium phosphate (pH 6.0), and 20 mM sodium cyanoborohydride was added as a reducing agent.

Particularly, in the used immunoglobulin region, two monomers having the amino acid sequence of SEQ ID NO: 438 (consisting of 221 amino acids) form a homodimer through a disulfide bond between cysteine residues, which are the amino acids at position 3 of each monomer, and the monomers of the homodimer each independently have an internal disulfide bond between cysteine at position 35 and cysteine at position 95 and an internal disulfide bond between cysteines at position 141 and cysteine at position 199.

TABLE 4

| Amino acid sequence of immunoglobulin Fc | | |
|---|---|---|
| **Name** | **Amino acid sequence** | **SEQ ID NO** |
| Immunoglobulin Fc | PSCPAPEFLG GPSVFLFPPK PKDTLMISRT PEVTCVVVDV SQEDPEVQFN WYVDGVEVHN AKTKPREEQF NSTYRVVSVL TVLHQDWLNG KEYKCKVSNK GLPSSIEKTI SKAKGQPREP QVYTLPPSQE EMTKNQVSLT CLVKGFYPSD IAVEWESNGQ PENNYKTTPP VLDSDGSFFL YSRLTVDKSR WQEGNVFSCS VMHEALHNHY TQKSLSLSLG K | 438 |

After the reaction was completed, the unreacted immunoglobulin Fc region was removed from the reaction solution by Butyl FF (Cytiva) using Bis-Tris (pH 6.5) and sodium chloride, and the reaction solution was purified using sodium citrate buffer (pH 5.5) and ammonium sulfate by Source 15ISO (Cytiva), thereby obtaining interleukin-2 analog-3.4K PEG-immunoglobulin Fc region conjugates (long-acting conjugates) in which the N-terminus of an interleukin-2 analog is linked to one end of a 3.4 kDa PEG linker and the opposite end of the 3.4 kDa PEG linker is linked to the nitrogen of the N-terminal proline of the Fc region. These long-acting conjugates were analyzed by SDS-PAGE, RP-HPLC, SE-HPLC (FIGS. 1 and 2).

**Example 8: Evaluation of binding affinities of interleukin-2 analog conjugates for interleukin-2 receptors**

In order to measure the binding affinities of each of the interleukin-2 analog long-acting conjugates obtained in Example 7 for the interleukin-2 alpha and beta receptors, surface plasma resonance (SPR, BIACORE T200, GE Healthcare) was used.

Specifically, biotin-labeled human interleukin-2 receptor alpha and beta subunits (ACROBiosystems) each were immobilized to a streptavidin biosensor chip (SA chip, Cytiva) at about 100 RU and 500 RU, and the interleukin-2 analog long-acting conjugates and aldesleukin, diluted in HBS-EP+ buffer (Cytiva, BR100669) by a two-fold dilution method, were

allowed to flow at a flow rate of 20 μL/min. After the binding process for 3 minutes, only HBS-EP+ buffer was allowed to flow for 3 minutes at the same flow rate to induce the dissociation of the interleukin-2 analog long-acting conjugates or aldesleukin from the interleukin-2 receptors. The binding affinity was calculated through the association constant and dissociation constant thus obtained. The binding affinity was evaluated according to the 1:1 binding fitting model in the Biaevaluation program.

The intrinsic interleukin-2 receptor binding affinity of the evaluated interleukin-2 analog conjugates was verified, and particularly, an apparent difference in binding affinity between the respective candidate substances was confirmed in the interleukin-2 receptor alpha subunit. A detailed examination of the results revealed that the interleukin-2 analog conjugates 21 and 52 did not bind to the interleukin-2 receptor alpha subunit, and the interleukin-2 analog conjugates 41, 86, 104, and 105 showed relative binding affinities of 50.8%, 65.2%, 81.0%, and 112.9% compared with aldesleukin, respectively. With respect to the interleukin-2 receptor beta subunit, all the interleukin-2 analog conjugates including the interleukin-2 analog conjugates 21 and 52 showed a higher binding affinity compared with aldesleukin.

Table 5 below summarizes the relative binding affinity (%) of the prepared interleukin-2 analog long-acting conjugates for each of the alpha and beta receptors compared with the binding affinity of aldesleukin. In Table 5, each corresponding long-acting conjugate is expressed by using the interleukin-2 analog number constituting the same (e.g., the long-acting conjugate of interleukin-2 analog 21 is expressed as "interleukin-2 analog conjugate 21").

TABLE 5

| Binding affinities of interleukin-2 analog conjugates for interleukin-2 receptors | | |
|---|---|---|
| **Substance to be evaluated** | **Interleukin-2 receptor alpha subunit** | **Interleukin-2 receptor beta subunit** |
| | **Relative binding affinity (%)** | **Relative binding affinity (%)** |
| Aldesleukin | 100.0 | 100.0 |
| Interleukin-2 analog conjugate 21 | - | 5,426.6 |
| Interleukin-2 analog conjugate 41 | 50.8 | 5,483.0 |
| Interleukin-2 analog conjugate 52 | - | 2,271.8 |
| Interleukin-2 analog conjugate 86 | 65.2 | 15,393.1 |
| Interleukin-2 analog conjugate 104 | 81.0 | 13,423.5 |
| Interleukin-2 analog conjugate 105 | 112.9 | 13,293.8 |

## Example 9: Evaluation of anticancer efficacy by combined administration of interleukin-2 analog long-acting conjugate and immune checkpoint inhibitor in malignant melanoma

In order to investigate the anticancer efficacy when an interleukin-2 analog conjugate and an immune checkpoint inhibitor were administered in combination, malignant melanoma mouse models allografted with B16F10 melanoma cells (B16F10 melanoma tumor syngeneic mouse models) were administered interleukin-2 analog conjugate 86 or aldesleukin (control) alone or in combination with a mouse anti-PD-1 antibody (Bio X cell, Catalog# BP0146), and then evaluated for tumor size and subject survival rate.

Specifically, B16F10 cells (ATCC) were subcutaneously injected into the thighs of C57BL/6 mice, and after a few days, when tumors were observed with the naked eye, eight mice were assigned to each group such that the tumors were similar in size. The interleukin-2 analog conjugate 86 was repeatedly administered at a dose of 0.08 mg/kg or 6.0 mg/kg (based on the weight of an interleukin-2 analog site of the interleukin-2 analog long-acting conjugate) via a subcutaneous route once a week for a total of four weeks. The control group (aldesleukin administration group) was repeatedly administered 3.0 mg/kg of Proleukin (Novartis) via an intraperitoneal route once a day for 5 consecutive days, followed by a 2-day rest period, and this cycle was repeated a total of 4 weeks. The anti-PD-1 antibody alone administration group was repeatedly administered 10.0 mg/kg of mouse-antibody-PD-1 antibody (Bio X cell) via an intraperitoneal route twice a week for a total of 4 weeks. The anti-PD-1 antibody combination groups were administered an anti-PD-1 antibody at the same dose (10.0 mg/kg) based on the interleukin-2 analog conjugate 86 (0.08 mg/kg) administration group, interleukin-2 analog conjugate 86 (6.0 mg/kg) administration group, and the aldesleukin (3.0 mg/kg) administration group. The time where the tumor size exceeded 2,000 $mm^3$ was set as the humane endpoint.

On Day 10 of administration, the tumor size (FIG. 3A) and the subject survival rate for 35 days (FIG. 3B) of each group were observed. As a result, in the single therapies, the interleukin-2 analog conjugate 86 (6.0 mg/kg) showed higher tumor inhibitory ability and subject survival rate compared with the control group despite the administration once a week. Particularly, in the interleukin-2 analogue conjugate 86 (6.0 mg/kg) administration group, complete remission showing

complete tumor removal was observed in two out of eight mice, but complete remission was not observed in the control group.

In combination therapy with anti-PD-1 antibody, the interleukin-2 analog conjugate 86 and anti-PD-1 antibody combined administration group showed higher tumor inhibitory activity and subject survival rate compared with the aldesleukin and anti-PD-1 antibody combined administration group. Particularly, in the interleukin-2 analog conjugate 86 (0.08 mg/kg, corresponding to a very low dose compared with aldesleukin) and anti-PD-1 antibody combined administration group, complete remission showing complete tumor removal was observed in two out of eight mice. In the interleukin-2 analog conjugate 86 (6.0 mg/kg) and anti-PD-1 antibody combined administration group, the subject survival rate was 100%, and complete remission were observed in seven out of eight mice, indicating superior anticancer efficacy. However, the aldesleukin and anti-PD-1 antibody combined administration group showed no complete remission.

**Example 10: Evaluation** of **anticancer efficacy** by **combined administration of interleukin-2 analog long-acting conjugate and immune checkpoint inhibitor in Lewis lung cancer mouse models**

In order to investigate the anticancer efficacy in lung cancer mouse models, Lewis lung cancer mouse models allografted with Lewis lung carcinoma (LL/S) cells (LL/2 tumor syngeneic mouse models) were administered interleukin-2 analog conjugate 86 or aldesleukin alone or in combination with a mouse anti-PD-1 antibody (Bio X cell, Catalog# BP0146), and then evaluated for anti-tumor efficacy.

LL/2 cells (ATCC) were subcutaneously injected into the thighs of C57BL/6 mice, and after a few days, when tumors were observed with the naked eye, ten mice were assigned to each group such that the tumors were similar in size. The groups were divided into single-drug administration groups and drug and immune checkpoint inhibitor combined administration groups, and the single-drug administration groups were assigned to interleukin-2 analog conjugate 86, aldesleukin, and anti-PD-1 antibody administration groups. Specifically, the interleukin-2 analog conjugate 86 administration group was repeatedly administered 6.0 mg/kg (based on IL-2) of the conjugate via a subcutaneous route once a week, a total of two times. The control group was administered 3.0 mg/kg of aldesleukin via an intraperitoneal route once a day for 5 consecutive days, followed by a 2-day rest period, and this cycle was a total of two times. The immune checkpoint inhibitor alone administration group was repeatedly administered an anti-PD1 antibody for two weeks via an intraperitoneal route once every 3 to 4 days, a total of two times a week.

The immune checkpoint inhibitor combined administration groups, separately set from the single-drug administration groups, were prepared by further administration of an anti-PD-1 antibody on the basis of the interleukin-2 analog conjugate 86 group and the control group, wherein the concentration and interval of administration were set to be the same as those of the single-drug administration groups.

On Day 12 of administration, the tumor size was observed for each group, and in order to evaluate the interaction of a drug by combined administration with the immune checkpoint inhibitor, the Bliss independence analysis was performed.

As a result, the interleukin-2 analog conjugate 86 showed excellent anti-tumor efficacy by administration thereof alone in the Lewis lung cancer mouse models (FIG. 4), and showed a significant synergistic effect by use in combination with an immune checkpoint inhibitor, differently from aldesleukin (FIGS. 5A and 5B).

The Lewis lung cancer mouse models, representative immune-infiltrating tumor models (cold tumor), have been reported to show insignificant efficacy by the treatment of an immune checkpoint inhibitor, including anti-PD-1 antibody, alone (Maryland Franklin, 2019, Labcorp). Also in the present example, the administration of the immune checkpoint inhibitor alone did not show significant anticancer efficacy (FIG. 4), but the combined administration of the interleukin-2 analog conjugate 86 according to the present invention and the immune checkpoint inhibitor showed a synergistic effect. These results verified that the interleukin-2 analog conjugate of the present invention induced the conversion of an immune-infiltrating tumor to an infiltrating tumor (hot tumor) in the tumor microenvironment (TME) of the Lewis lung models, indicating that the interleukin-2 analog conjugate exhibited an excellent effect by combined administration with an immune checkpoint inhibitor.

**Example 11: Evaluation of anticancer efficacy by combined administration of interleukin-2 analog long-acting conjugate and anti-PD-1 antibody in pancreatic cancer (pancreatic ductal adenocarcinoma, PDAC) mouse models and verification of memory response in cancer cure models**

In order to evaluate the anticancer efficacy of an interleukin 2 analog long-acting conjugate alone and in combination with an anti-PD-1 antibody against pancreatic cancer (pancreatic ductal adenocarcinoma) as one of the most aggressive malignant tumors, syngeneic tumor mouse models were constructed by subcutaneously injecting mouse-derived pancreatic ductal adenocarcinoma cells (panc02) into the flanks of 6-week-old female C57BL/6 mice. After 14 days, seven animals were randomly assigned to each group on the basis of the tumor volume (~80 $mm^3$).

For testing, the groups were divided into an interleukin-2 analog conjugate 86 alone administration group, an anti-PD-1 antibody alone administration group, and two-drug combined administration group, and administration was conducted for a total of 6 weeks according to the administration regimen of each drug. The anti-tumor efficacy for each of the experimental group was evaluated by tumor growth inhibition (TGI) using the expression below. The detailed drug dose, administration route, number of times of administration, and anti-tumor efficacy for each group are shown in Table 6.

%TGI = (1 - (Increased tumor volume from Day 0 to Day X in the experimental group / Increased tumor volume from Day 0 to Day X in the negative control group)) x 100

TABLE 6

| Experimental group | | Dose | Route and period of administration | Tumor size (tumor growth inhibitory ability)* |
|---|---|---|---|---|
| **Single-drug administration group** | Negative control | | Subcutaneous, once a week x 6 | 397.37 $mm^3$ |
| | Interleukin-2 analog conjugate 86 | 0.3 mg/kg | Subcutaneous, once a week x 6 | 420.02 $mm^3$ (-7.36%) |
| | | 1.7 mg/kg | Subcutaneous, once a week x 6 | 293.15 $mm^3$ (33.05%) |
| | | 8.5 mg/kg | Subcutaneous, once a week x 6 | 30.66 $mm^3$ (116.67%) |
| | | 25 mg/kg | Subcutaneous, once a week x 6 | 10.33 $mm^3$ (123.20%) |
| | Anti-PD-1 antibody | 10 mg/kg | Intraperitoneal, twice a week x 6 | 500.62 $mm^3$ (-33.00%) |
| **Combined administration group** | Interleukin-2 analog conjugate 86 + Anti-PD-1 antibody | 0.3 mg/kg + 10 mg/kg | Subcutaneous, once a week x 6, Intraperitoneal, twice a week x 6 | 243.16 $mm^3$ (49.04%) |
| | | 25 mg/kg + 10 mg/kg | Subcutaneous, once a week x 6, Intraperitoneal, twice a week x 6 | 13.21 $mm^3$ (122.26%) |
| * Analyzed on the basis of Day 32 after drug administration, which is the last measurement day before the occurrence of tumor necrosis, in each group. | | | | |

The anti-tumor efficacy of the interleukin 2 analog conjugate 86 was observed from a dose of 1.7 mg/kg, and this efficacy increased in a dose-dependent manner up to a maximum of 25 mg/kg. The anti-tumor efficacy was not observed in the interleukin-2 analog conjugate 86 (0.3 mg/kg) administration group and the anti-PD-1 antibody alone administration group compared with the negative control group, but a significant level of tumor inhibitory efficacy was observed when the two drugs were administered in combination at the same dose. Particularly, complete remission was observed in the interleukin-2 analog conjugate 86 (8.5 mg/kg or more) alone administration group, and complete remission was observed in all the subjects of the conjugate (25 mg/kg) alone administration group or the conjugate and anti-PD-1 antibody combined administration group.

The subjects confirmed to show complete remission were investigated for a recurrence preventing effect through the memory response by subcutaneously injecting panc02 tumor cells, identical to those of the primary cancer, into the flanks on the 89th day from the first date of drug administration. As a result, the recurrence preventing effect through the memory response was confirmed in 70% or more in the interleukin-2 analog conjugate 86 (25 mg/kg) alone administration group or the conjugate and anti-PD-1 antibody combined administration group (FIG. 6). The results suggest that the interleukin-2 analog conjugate 86 can treat primary pancreatic cancer as well as prevent the recurrence of the same tumor.

The experimental results above suggest that the interleukin-2 analog long-acting conjugates according to the present invention exhibited strong anticancer efficacy and low risk of side effects through altered binding affinities for the interleukin-2 alpha and beta receptors and allowed for high blood exposure through a structure of a long-acting conjugate linked to a human immunoglobulin Fc region, and thus can increase the interval of administration compared with existing

interleukin-2 therapy and enhance the in vivo immune response with high activity at a low dose. Furthermore, the interleukin-2 analog conjugates according to the present invention can exhibit superior anticancer efficacy through a synergistic action with an immune checkpoint inhibitor, so that the interleukin-2 analog conjugates can be used in the safe and effective anticancer treatment when administered in combination with an immune checkpoint inhibitor, and the interleukin-2 analog or long-acting conjugates containing the same according to the present invention can suppress cancer recurrence by inducing an immune memory response in a subject.

While the present invention has been described with reference to the particular illustrative embodiments, a person skilled in the art to which the present invention pertains can understand that the present invention may be embodied in other specific forms without departing from the technical spirit or essential characteristics thereof. Therefore, the exemplary embodiments described above should be construed as being exemplified and not limiting the present invention. The scope of the invention should be construed to mean that the meaning and scope of the appended claims rather than the detailed description and all changes or variations derived from the equivalent concepts fall within the scope of the present invention.

[Description of Government-Sponsored Research]

This study was supported by the National New Drug Development Project Support of the KOREA DRUG DEVELOPMENT FUND funded by the Ministry of Science and ICT, the Ministry of Trade, Industry and Energy, and the Ministry of Health and Welfare (RS-2022-00165557).

## Claims

1. A pharmaceutical composition for prevention or treatment of cancer, the composition comprising an interleukin-2 analog including any one sequence selected from amino acid sequences of SEQ ID NOS: 3 to 106, wherein the composition is administered in combination with an immune checkpoint inhibitor.

2. The composition of claim 1, wherein the interleukin-2 analog is in the form of a long-acting conjugate, and the long-acting conjugate is represented by chemical formula 1 below:

   [Chemical Formula 1] X-L-F

   where: X is an interleukin-2 analog including any one sequence selected from amino acid sequences of SEQ ID NOS: 3 to 106;
   L is a polyethylene glycol linker;
   F is a dimeric immunoglobulin Fc region, and
   the - symbols represent covalent linkages between X and L and between L and F, respectively,
   wherein in the long-acting conjugate, one end of L is covalently linked to only one polypeptide chain of the dimeric Fc region and X is covalently linked to the other end of L.

3. The composition of claim 1 or 2, wherein the interleukin-2 analog comprises any one sequence selected from the group consisting of amino acid sequences of SEQ ID NOS: 10, 13-15, 17, 20-22, 32, 35, 36, 42, 53, 54, 56, 58-60, 62, 71, 72, 74-78, 85, 87, 89, 91-94, and 97-106.

4. The composition of claim 1 or 2, wherein the interleukin-2 analog comprises any one sequence selected from the group consisting of amino acid sequences of SEQ ID NOS: 17, 22, 42, 53, 56, 58-60, 62, 71, 72, 74-77, 87, 89, 91-93, 98-101, and 103-106.

5. The composition of claim 1 or 2, wherein the interleukin-2 analog comprises any one sequence selected from the group consisting of amino acid sequences of SEQ ID NOS: 22, 42, 53, 87, 105, and 106.

6. The composition of claim 1 or 2, wherein the interleukin-2 analog further comprises at least one amino acid at the C-terminus.

7. The composition of claim 1 or 2, wherein the interleukin-2 analog has an increased binding affinity for interleukin-2 beta receptor compared with aldesleukin.

**8.** The composition of claim of 2, wherein the immunoglobulin Fc region is derived from IgG, IgA, IgD, IgE, or IgM, or is a combination or hybrid thereof.

**9.** The composition of claim of 2, wherein the immunoglobulin Fc region is an IgG4 Fc region.

**10.** The composition of claim of 2, wherein the immunoglobulin Fc region is aglycosylated.

**11.** The composition of claim of 2, wherein in the long-acting conjugate, X is covalently linked to one Fc region of the dimeric immunoglobulin Fc region via the polyethylene glycol linker.

**12.** The composition of claim of 2, wherein the polyethylene glycol linker has a molecular weight of 1 to 100 kDa.

**13.** The composition of claim 1 or 2, further comprising a pharmaceutically acceptable excipient.

**14.** The composition of claim 1 or 2, wherein the cancer is any one selected from the group consisting of renal cell cancer, melanoma, colorectal cancer, liver cancer, uterine cancer, ovarian cancer, pancreatic cancer, gallbladder cancer, lung cancer, small cell lung cancer, non-small cell lung cancer, skin cancer, breast cancer, bladder cancer, stomach cancer, head or neck cancer, esophageal cancer, laryngeal cancer, bone cancer, rectal cancer, perianal cancer, colon cancer, fallopian tube carcinoma, endometrial carcinoma, cervical carcinoma, vaginal carcinoma, vulvar carcinoma, Hodgkin's disease, small bowel cancer, endocrine gland cancer, thyroid cancer, parathyroid cancer, adrenal cancer, soft tissue sarcoma, urethral cancer, penile cancer, prostate cancer, chronic or acute leukemia, lymphocyte lymphoma, renal pelvis carcinoma, CNS tumor, primary CNS lymphoma, spinal tumor, brain tumor, glioma (astrocytoma, glioblastoma, oligodendroglioma, ependymoma), blastocytoma, meningioma, brainstem glioma, pituitary adenoma, neuroblastoma, congenital tumor, craniopharyngioma, and brain tumor.

**15.** The composition of claim 1 or 2, wherein the cancer has a low responsiveness to a PD-1 antagonist.

**16.** The composition of claim 1 or 2, wherein the composition is administered via an intraperitoneal, intravenous, intramuscular, subcutaneous, intradermal, oral, topical, intranasal, intrapulmonary, or intrarectal route.

**17.** The composition of claim 1 or 2, wherein the composition is administered at time intervals ranging from 1 week to 1 month.

**18.** The composition of claim 1 or 2, wherein the composition is administered in combination with a composition comprising an immune checkpoint inhibitor simultaneously, separately, sequentially, or in reverse order.

**19.** The composition of claim 1 or 2, wherein the immune checkpoint inhibitor is a PD-1 antagonist.

**20.** The composition of claim of 19, wherein the immune checkpoint inhibitor is at least one selected from an anti-PD-1 antibody or an antigen-binding fragment thereof.

**21.** The composition of claim 20, wherein the anti-PD-1 antibody is selected from the group consisting of nivolumab, pembrolizumab, cemiplimab, spartalizumab, camrelizumab, sintilimab, tislelizumab, toripalimab, dostarlimab, INCMGA00012, AMP-224, and AMP-514.

[FIG. 1]

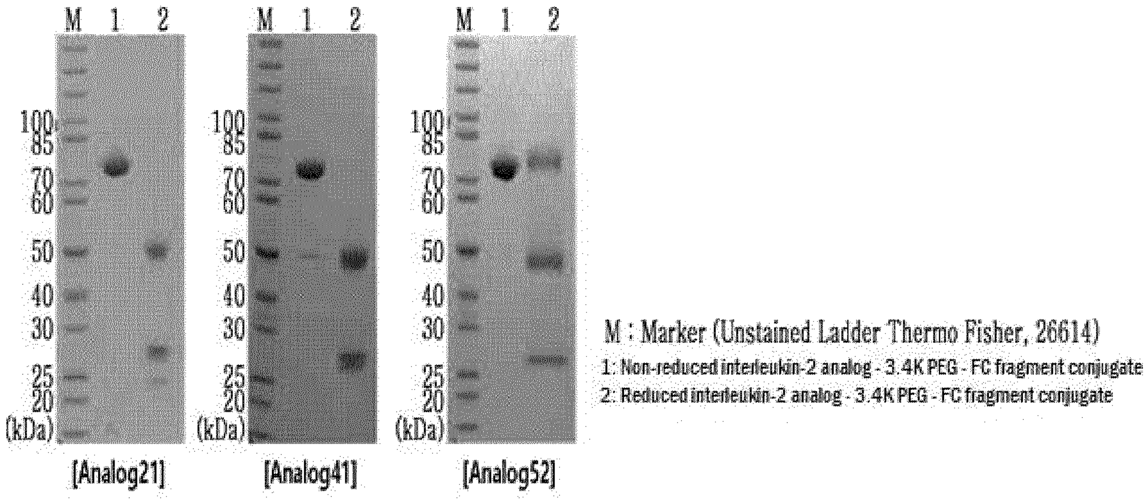
M 1 2
100
85
70
60
50
40
30
25
20
(kDa)
[Analog21]
[Analog41]
[Analog52]
M : Marker (Unstained Ladder Thermo Fisher, 26614)
1: Non-reduced interleukin-2 analog - 3.4K PEG - FC fragment conjugate
2: Reduced interleukin-2 analog - 3.4K PEG - FC fragment conjugate

[FIG. 2A]

**Analog 21**

**SE-HPLC**

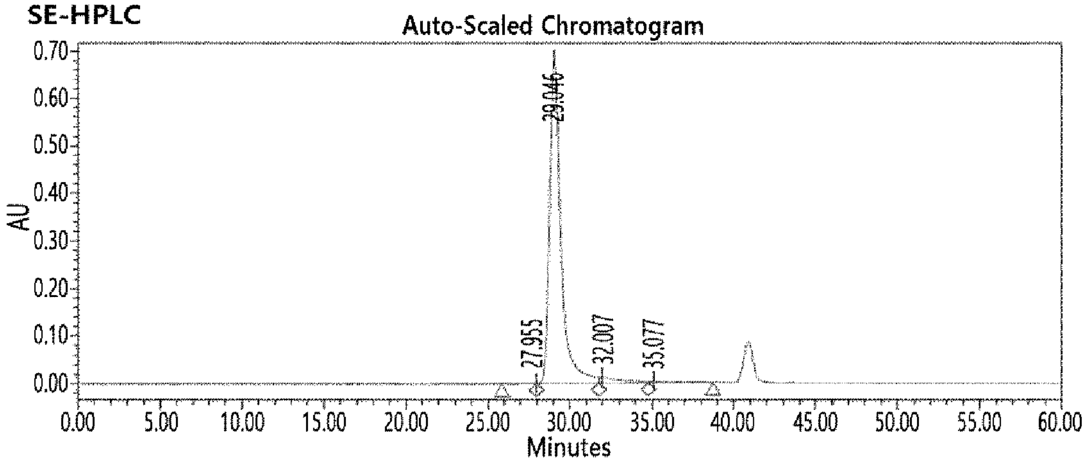


**Peak Results**

| | RT | Int Type | Width (sec) | Area | Height | % Area | Total Area |
|---|---|---|---|---|---|---|---|
| 1 | 27.955 | bv | 127.200 | 50410 | 945 | 0.15 | 34444275 |
| 2 | 29.046 | vv | 228.800 | 32900952 | 682750 | 95.52 | 34444275 |
| 3 | 32.007 | vv | 181.200 | 1164536 | 11799 | 3.38 | 34444275 |
| 4 | 35.077 | vb | 235.600 | 328377 | 3549 | 0.95 | 34444275 |

**RP-HPLC**

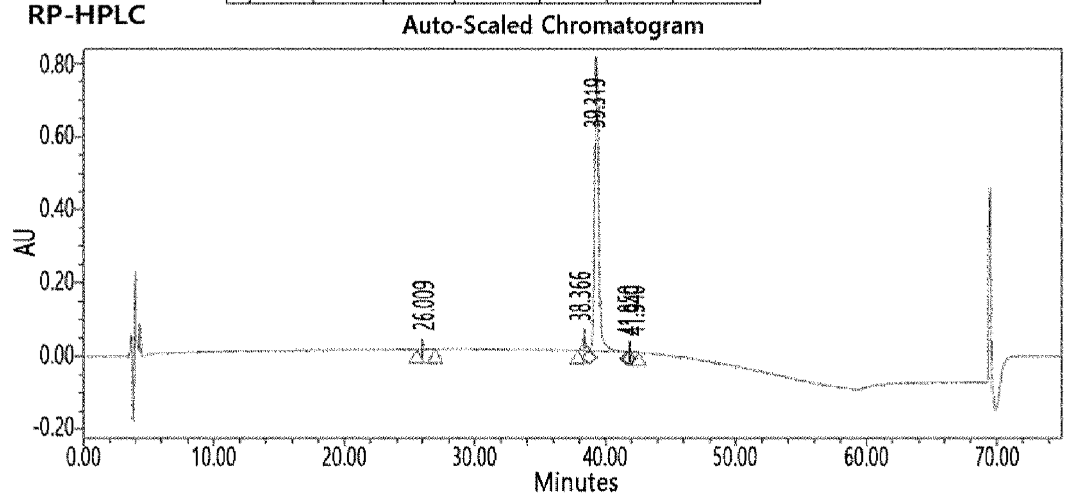


**Peak Results**

| | RT | Int Type | Width (sec) | Area | Height | % Area | Total Area |
|---|---|---|---|---|---|---|---|
| 1 | 26.009 | Bb | 83.600 | 51066 | 1429 | 0.28 | 18317715 |
| 2 | 38.366 | bv | 52.400 | 708275 | 32497 | 3.87 | 18317715 |
| 3 | 39.319 | vv | 173.600 | 17478553 | 777242 | 95.42 | 18317715 |
| 4 | 41.850 | vv | 11.200 | 27638 | 2585 | 0.15 | 18317715 |
| 5 | 41.940 | vb | 42.000 | 52183 | 3089 | 0.28 | 18317715 |

[FIG. 2B]

**Analog 41**

**SE-HPLC**

Auto-Scaled Chromatogram

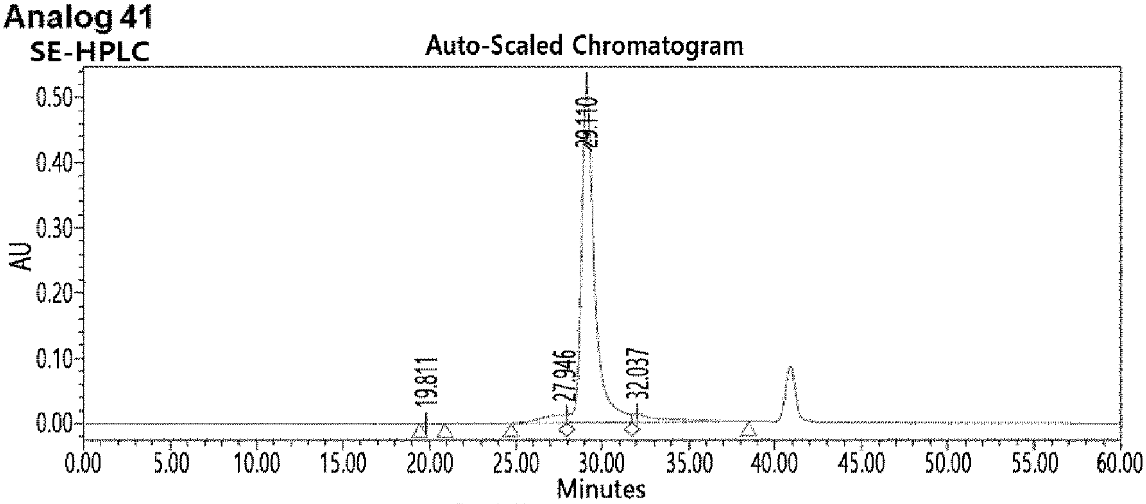


**Peak Results**

| | RT | Int Type | Width (sec) | Area | Height | % Area | Total Area |
|---|---|---|---|---|---|---|---|
| 1 | 19.811 | bb | 87.200 | 25933 | 1206 | 0.09 | 30049290 |
| 2 | 27.946 | bv | 194.000 | 1177831 | 12306 | 3.92 | 30049290 |
| 3 | 29.110 | vv | 227.200 | 27297055 | 520812 | 90.84 | 30049290 |
| 4 | 32.037 | vb | 404.400 | 1548471 | 13016 | 5.15 | 30049290 |

**RP-HPLC**

Auto-Scaled Chromatogram

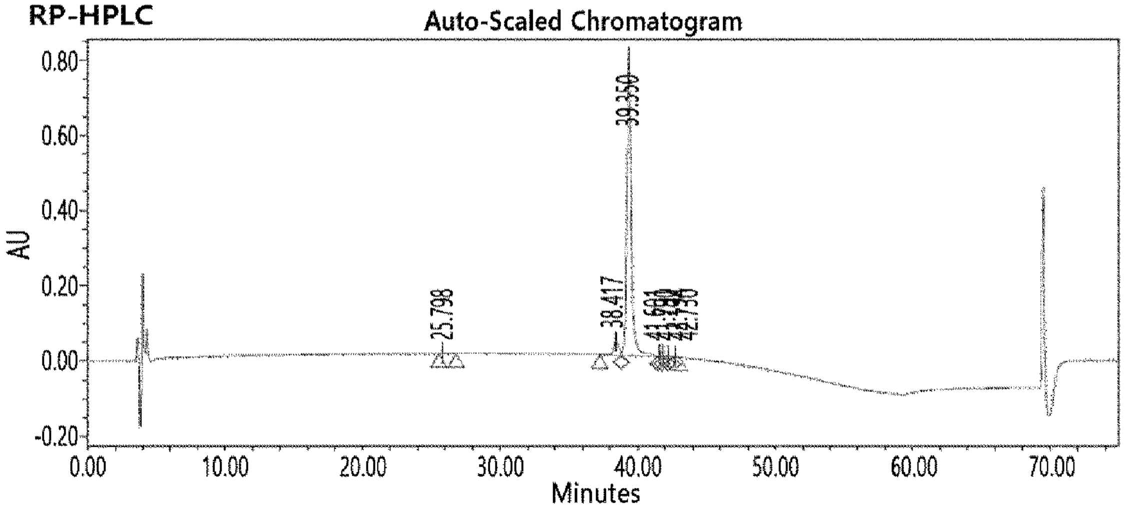


**Peak Results**

| | RT | Int Type | Width (sec) | Area | Height | % Area | Total Area |
|---|---|---|---|---|---|---|---|
| 1 | 25.798 | BB | 75.600 | 47777 | 1788 | 0.24 | 19600965 |
| 2 | 38.417 | bv | 94.000 | 962823 | 36778 | 4.91 | 19600965 |
| 3 | 39.350 | vv | 159.600 | 18335061 | 790978 | 93.54 | 19600965 |
| 4 | 41.601 | vv | 18.000 | 76997 | 4518 | 0.39 | 19600965 |
| 5 | 41.790 | vv | 25.600 | 81262 | 4246 | 0.41 | 19600965 |
| 6 | 42.194 | vv | 22.400 | 41294 | 2181 | 0.21 | 19600965 |
| 7 | 42.730 | vb | 28.800 | 55751 | 3954 | 0.28 | 19600965 |

[FIG. 2C]

**Analog 52**

**SE-HPLC**

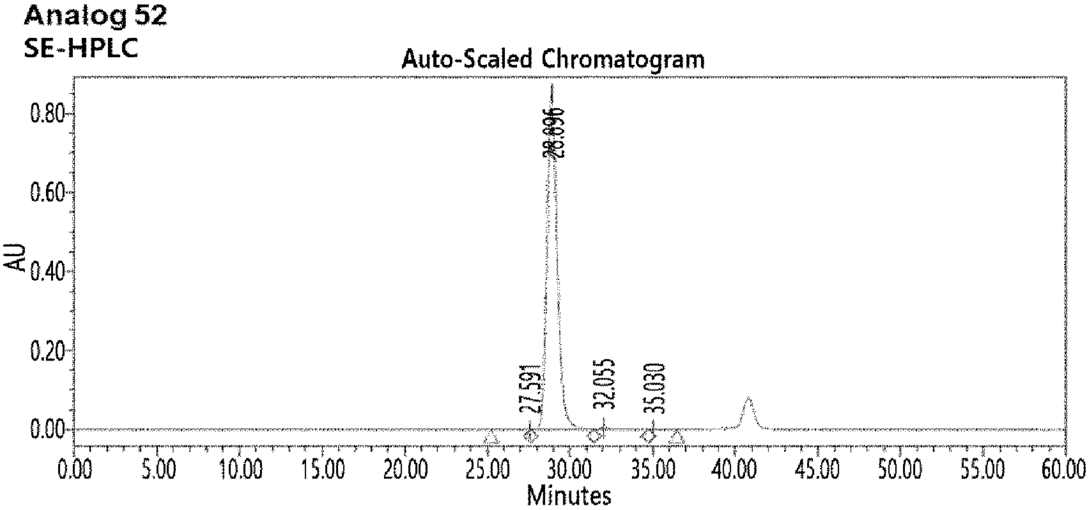


**Peak Results**

| | RT | Int Type | Width (sec) | Area | Height | % Area | Total Area |
|---|---|---|---|---|---|---|---|
| 1 | 27.591 | bv | 144.000 | 24021 | 348 | 0.06 | 37263781 |
| 2 | 28.896 | vv | 230.000 | 37016396 | 851448 | 99.34 | 37263781 |
| 3 | 32.055 | vv | 197.200 | 212592 | 3662 | 0.57 | 37263781 |
| 4 | 35.030 | vb | 104.000 | 10772 | 250 | 0.03 | 37263781 |

**RP-HPLC**

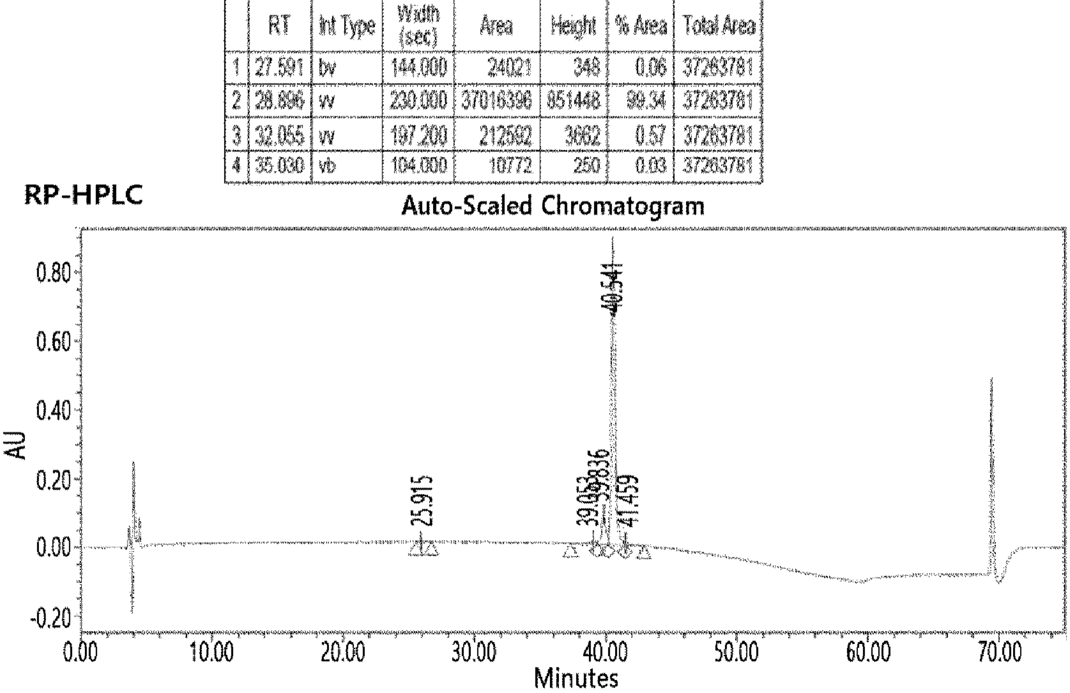


**Peak Results**

| | RT | Int Type | Width (sec) | Area | Height | % Area | Total Area |
|---|---|---|---|---|---|---|---|
| 1 | 25.915 | Bb | 70.400 | 34022 | 1272 | 0.18 | 19021437 |
| 2 | 39.053 | bv | 122.800 | 386132 | 8859 | 2.03 | 19021437 |
| 3 | 39.836 | vv | 47.600 | 1921333 | 88063 | 10.10 | 19021437 |
| 4 | 40.541 | vv | 77.600 | 16466179 | 864087 | 86.57 | 19021437 |
| 5 | 41.459 | vb | 87.200 | 213771 | 6643 | 1.12 | 19021437 |

[FIG. 3]

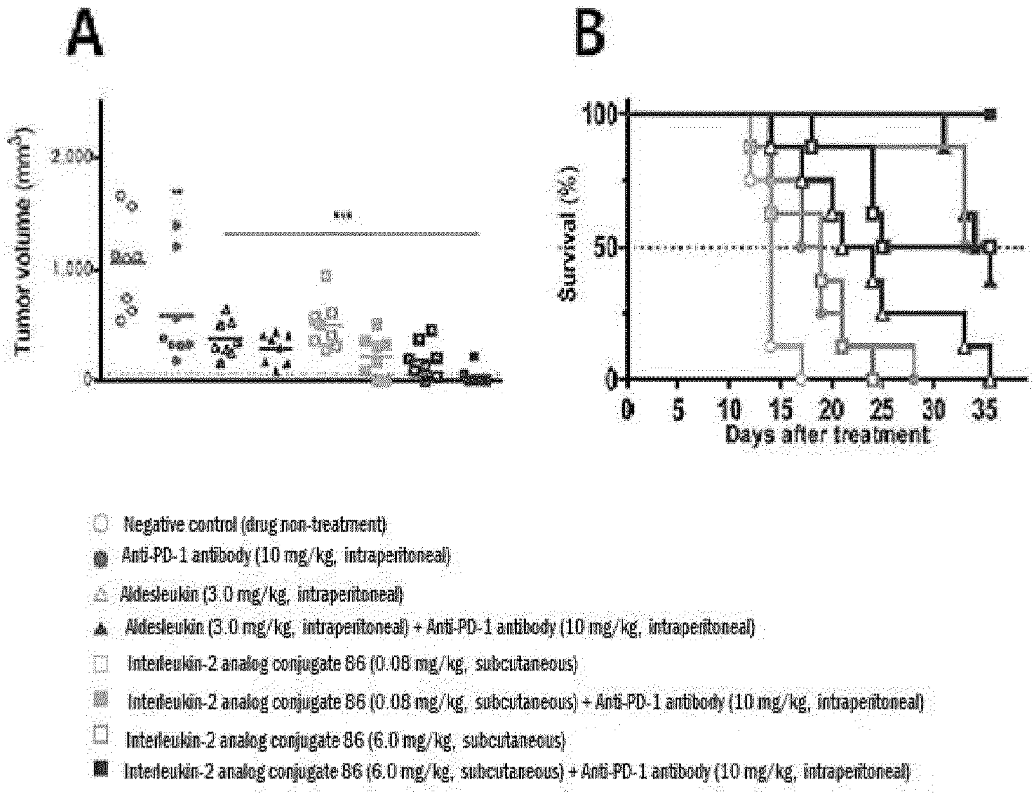
A
B
Tumor volume (mm3)
2,000
1,000
0
***
Survival (%)
100
50
0
0 5 10 15 20 25 30 35
Days after treatment
Negative control (drug non-treatment)
Anti-PD-1 antibody (10 mg/kg, intraperitoneal)
Aldesleukin (3.0 mg/kg, intraperitoneal)
Aldesleukin (3.0 mg/kg, intraperitoneal) + Anti-PD-1 antibody (10 mg/kg, intraperitoneal)
Interleukin-2 analog conjugate 86 (0.08 mg/kg, subcutaneous)
Interleukin-2 analog conjugate 86 (0.08 mg/kg, subcutaneous) + Anti-PD-1 antibody (10 mg/kg, intraperitoneal)
Interleukin-2 analog conjugate 86 (6.0 mg/kg, subcutaneous)
Interleukin-2 analog conjugate 86 (6.0 mg/kg, subcutaneous) + Anti-PD-1 antibody (10 mg/kg, intraperitoneal)

[FIG. 4]

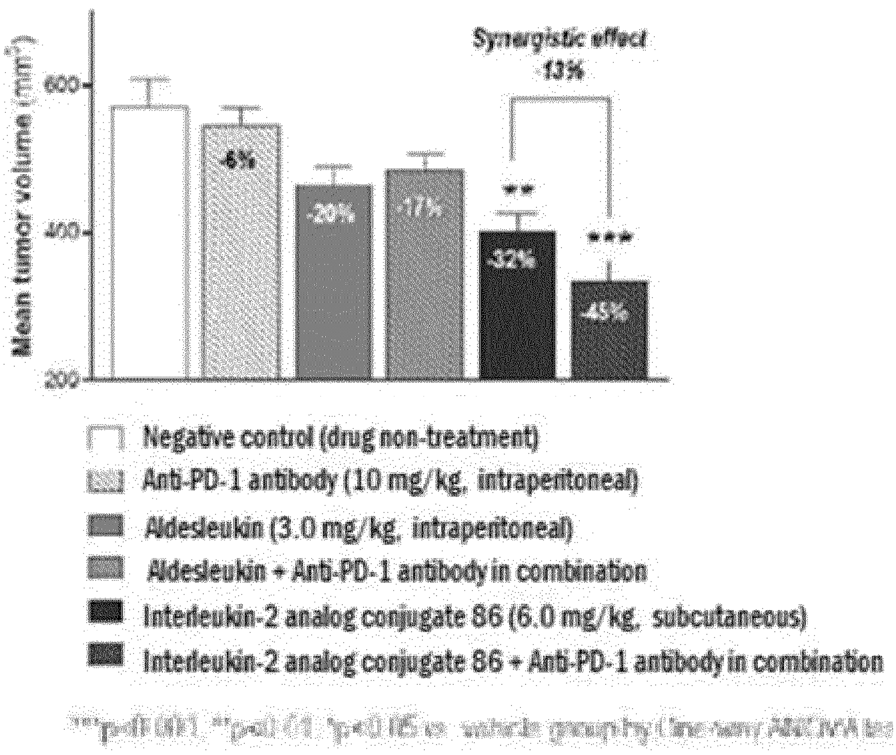
Mean tumor volume (mm3)
600
400
200
Synergistic effect
13%
-6%
-20%
-17%
**
-32%
***
-45%
Negative control (drug non-treatment)
Anti-PD-1 antibody (10 mg/kg, intraperitoneal)
Aldesleukin (3.0 mg/kg, intraperitoneal)
Aldesleukin + Anti-PD-1 antibody in combination
Interleukin-2 analog conjugate 86 (6.0 mg/kg, subcutaneous)
Interleukin-2 analog conjugate 86 + Anti-PD-1 antibody in combination

[FIG. 5]

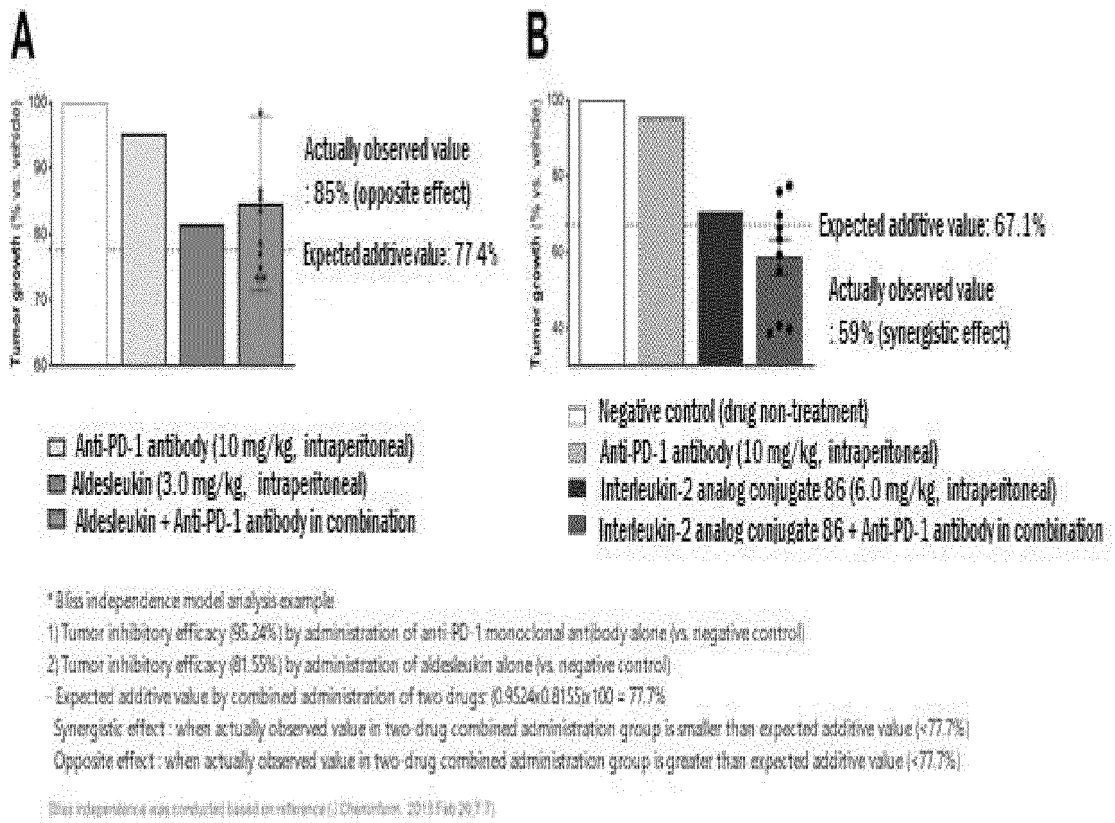
A
B
Tumor growth (% vs. vehicle)
100
90
80
70
60
40
Actually observed value
: 85% (opposite effect)
Expected additive value: 77.4%
Expected additive value: 67.1%
Actually observed value
: 59% (synergistic effect)
Anti-PD-1 antibody (10 mg/kg, intraperitoneal)
Aldesleukin (3.0 mg/kg, intraperitoneal)
Aldesleukin + Anti-PD-1 antibody in combination
Negative control (drug non-treatment)
Anti-PD-1 antibody (10 mg/kg, intraperitoneal)
Interleukin-2 analog conjugate 86 (6.0 mg/kg, intraperitoneal)
Interleukin-2 analog conjugate 86 + Anti-PD-1 antibody in combination
* Bliss independence model analysis example
1) Tumor inhibitory efficacy (95.24%) by administration of anti-PD-1 monoclonal antibody alone (vs. negative control)
2) Tumor inhibitory efficacy (81.55%) by administration of aldesleukin alone (vs. negative control)
- Expected additive value by combined administration of two drugs: (0.9524x0.8155)x100 = 77.7%
Synergistic effect : when actually observed value in two-drug combined administration group is smaller than expected additive value (<77.7%)
Opposite effect : when actually observed value in two-drug combined administration group is greater than expected additive value (<77.7%)

[FIG. 6]

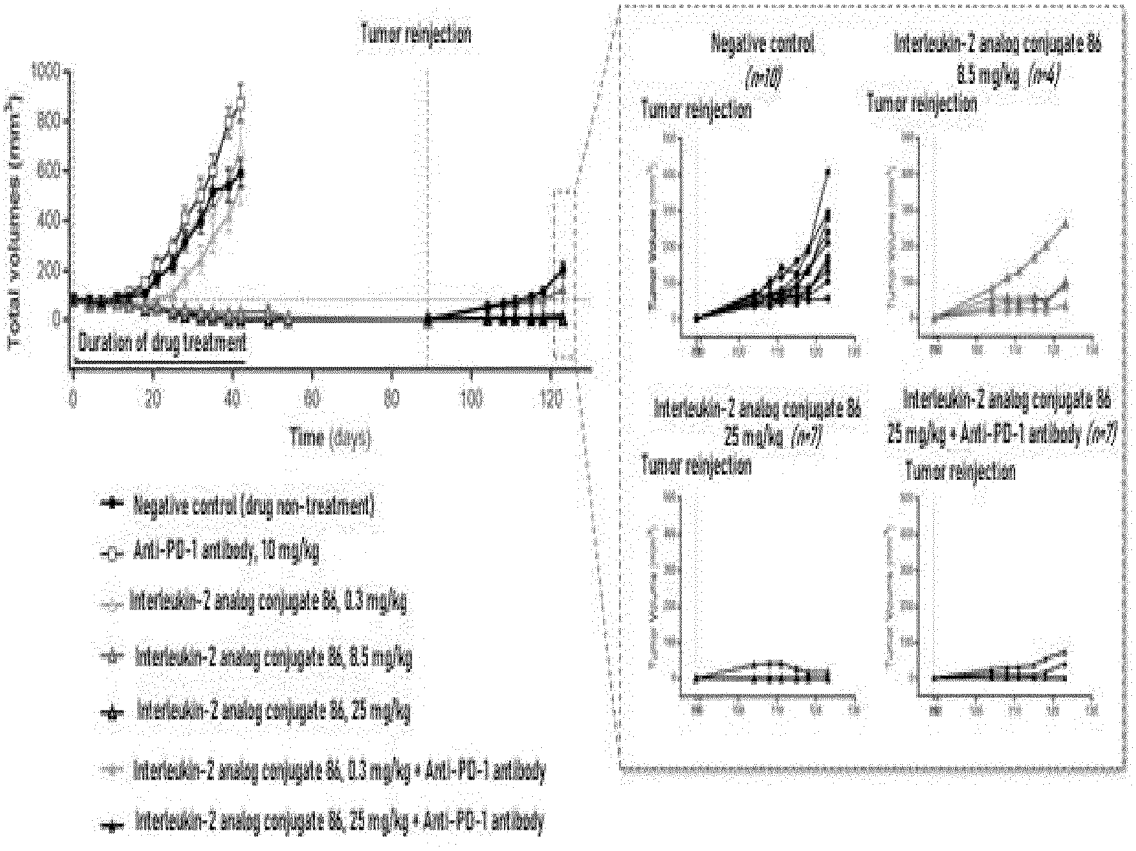
Tumor reinjection
Total volumes (mm³)
1000
800
600
400
200
0
Duration of drug treatment
0
20
40
60
80
100
120
Time (days)
Negative control (drug non-treatment)
Anti-PD-1 antibody, 10 mg/kg
Interleukin-2 analog conjugate 86, 0.3 mg/kg
Interleukin-2 analog conjugate 86, 8.5 mg/kg
Interleukin-2 analog conjugate 86, 25 mg/kg
Interleukin-2 analog conjugate 86, 0.3 mg/kg + Anti-PD-1 antibody
Interleukin-2 analog conjugate 86, 25 mg/kg + Anti-PD-1 antibody
Negative control (n=10)
Tumor reinjection
Interleukin-2 analog conjugate 86 8.5 mg/kg (n=4)
Tumor reinjection
Interleukin-2 analog conjugate 86 25 mg/kg (n=7)
Tumor reinjection
Interleukin-2 analog conjugate 86 25 mg/kg + Anti-PD-1 antibody (n=7)
Tumor reinjection

**INTERNATIONAL SEARCH REPORT**

International application No.
**PCT/KR2024/002946**

**A. CLASSIFICATION OF SUBJECT MATTER**

**A61K 38/20**(2006.01)i; **A61K 47/68**(2017.01)i; **A61K 45/06**(2006.01)i; **A61P 35/00**(2006.01)i

According to International Patent Classification (IPC) or to both national classification and IPC

**B. FIELDS SEARCHED**

Minimum documentation searched (classification system followed by classification symbols)

A61K 38/20(2006.01); A61K 38/17(2006.01); A61K 39/00(2006.01); A61K 47/68(2017.01); A61K 9/16(2006.01); C07K 16/24(2006.01); C07K 19/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 인터루킨-2 아날로그(interleukin-2 analog), 면역관문 억제제(immune checkpoint inhibitor), 병용(coadministration), 암(cancer)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2022-0136285 A (HANMI PHARM. CO., LTD.) 07 October 2022 (2022-10-07) See claims 1, 12-14, 16-21, 27-29 and 31-32; and table 2. | 1-21 |
| Y | KR 10-2018-0129684 A (INSTITUTE FOR BASIC SCIENCE et al.) 05 December 2018 (2018-12-05) See claims 1 and 16-17; and paragraphs [0094]-[0095] and [0113]. | 1-21 |
| A | KR 10-2022-0148304 A (CYTUNE PHARMA et al.) 04 November 2022 (2022-11-04) See entire document. | 1-21 |
| A | KR 10-2021-0053891 A (ONCOUR PHARMA, INC.) 12 May 2021 (2021-05-12) See entire document. | 1-21 |
| A | KR 10-2022-0051153 A (PINETREE THERAPEUTICS, INC.) 26 April 2022 (2022-04-26) See entire document. | 1-21 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 June 2024** | **26 June 2024** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/002946**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/KR2024/002946**

| Patent document cited in search report | Publication date (day/month/year) | Patent family member(s) | Publication date (day/month/year) |
|---|---|---|---|
| KR 10-2022-0136285 A | 07 October 2022 | BR 112023018530 A2 | 10 October 2023 |
| | | CA 3205058 A1 | 26 October 2022 |
| | | CL 2023002504 A1 | 26 January 2024 |
| | | CN 116916969 A | 20 October 2023 |
| | | EP 4316528 A1 | 07 February 2024 |
| | | IL 304365 A | 01 September 2023 |
| | | JP 2024-512418 A | 19 March 2024 |
| | | TW 202304521 A | 01 February 2023 |
| | | US 2024-0009317 A1 | 11 January 2024 |
| | | WO 2022-211537 A1 | 06 October 2022 |
| KR 10-2018-0129684 A | 05 December 2018 | BR 112019024745 A2 | 16 June 2020 |
| | | BR 112019024745 B1 | 16 January 2024 |
| | | CA 3064534 A1 | 29 November 2018 |
| | | CN 110914297 A | 24 March 2020 |
| | | CN 110914297 B | 06 June 2023 |
| | | EP 3630825 A1 | 08 April 2020 |
| | | EP 3630825 B1 | 14 February 2024 |
| | | JP 2020-520672 A | 16 July 2020 |
| | | JP 7057980 B2 | 21 April 2022 |
| | | KR 10-2099593 B1 | 13 April 2020 |
| | | US 11117958 B2 | 14 September 2021 |
| | | US 2020-0140538 A1 | 07 May 2020 |
| | | WO 2018-217058 A1 | 29 November 2018 |
| KR 10-2022-0148304 A | 04 November 2022 | CA 2920539 A1 | 12 February 2015 |
| | | CN 105579062 A | 11 May 2016 |
| | | EP 3030262 A1 | 15 June 2016 |
| | | EP 3030262 B1 | 09 October 2019 |
| | | EP 3659622 A1 | 03 June 2020 |
| | | JP 2016-527286 A | 08 September 2016 |
| | | JP 2019-189638 A | 31 October 2019 |
| | | JP 2022-091985 A | 21 June 2022 |
| | | JP 6794255 B2 | 02 December 2020 |
| | | KR 10-2457731 B1 | 21 October 2022 |
| | | US 2016-0184399 A1 | 30 June 2016 |
| | | US 2022-0168395 A1 | 02 June 2022 |
| | | US 2022-0202904 A1 | 30 June 2022 |
| | | WO 2015-018529 A1 | 12 February 2015 |
| KR 10-2021-0053891 A | 12 May 2021 | AU 2019-312589 A1 | 04 February 2021 |
| | | AU 2019-312589 A1 | 06 February 2020 |
| | | BR 112021001858 A2 | 04 May 2021 |
| | | CA 3107134 A1 | 06 February 2020 |
| | | CN 112823001 A | 18 May 2021 |
| | | EP 3829548 A1 | 09 June 2021 |
| | | IL 280468 A | 01 March 2021 |
| | | IL 280468 D0 | 01 March 2021 |
| | | JP 2021-532136 A | 25 November 2021 |
| | | MX 2021001252 A | 27 May 2021 |
| | | SG 11202100700 A | 25 February 2021 |
| | | US 2021-290661 A1 | 23 September 2021 |
| | | WO 2020-028544 A1 | 06 February 2020 |

Form PCT/ISA/210 (patent family annex) (July 2022)

**INTERNATIONAL SEARCH REPORT**
**Information on patent family members**

International application No.
**PCT/KR2024/002946**

| Patent document cited in search report | Publication date (day/month/year) | Patent family member(s) | Publication date (day/month/year) |
|---|---|---|---|
| KR 10-2022-0051153 A | 26 April 2022 | CA 3075403 A1 | 14 March 2019 |
| | | EP 3680250 A1 | 15 July 2020 |
| | | KR 10-2019-0028343 A | 18 March 2019 |
| | | KR 10-2020-0099118 A | 21 August 2020 |
| | | KR 10-2021-0049746 A | 06 May 2021 |
| | | US 2020-0276328 A1 | 03 September 2020 |
| | | WO 2019-050326 A1 | 14 March 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020170070091
- WO 9734631 A
- WO 9632478 A


### Non-patent literature cited in the description


- **PEARSON et al.** *Proc. Natl. Acad. Sci. USA*, 1988, vol. 85, 2444
- **NEEDLEMAN** ; **WUNSCH**. *J. Mol. Biol.*, 1970, vol. 48, 443-453
- **RICE et al.** Needleman program of the European Molecular Biology Open Software Suite (EMBOSS) package. *Trends Genet.*, 2000, vol. 16, 276-277
- **DEVEREUX, J. et al.** *Nucleic Acids Research*, 1984, vol. 12, 387
- **ATSCHUL, [S.] [F.** *J MOLEC BIOL*, 1990, vol. 215, 403
- Guide to Huge Computers. Academic Press, 1994
- **CARILLO**. *SIAM J Applied Math*, 1988, vol. 48, 1073
- **NEEDLEMAN et al.** *J Mol Biol.*, 1970, vol. 48, 443
- **SMITH** ; **WATERMAN**. *Adv. Appl. Math*, 1981, vol. 2, 482
- **GRIBSKOV et al.** *Nucl. Acids Res.*, 1986, vol. 14, 6745
- Atlas of Protein Sequence and Structure. National Biomedical Research Foundation, 1979, 353-358
- **J. SAMBROOK et al.** Molecular Cloning, A Laboratory Manual. Cold Spring Harbor Laboratory press, 1989
- **F. M. AUSUBEL et al.** Current Protocols in Molecular Biology. John Wiley & Sons, Inc.